(19)

Europäisches
Patentamt
European
Patent Office
Office européen
des brevets

(11) **EP 4 273 162 A1**

(12) **EUROPEAN PATENT APPLICATION**

(43) Date of publication:
**08.11.2023 Bulletin 2023/45**

(21) Application number: **22172186.3**

(22) Date of filing: **06.05.2022**

(51) International Patent Classification (IPC):
**C07K 16/00** (2006.01)   **C07K 16/28** (2006.01)
**C07K 16/30** (2006.01)

(52) Cooperative Patent Classification (CPC):
**C07K 16/00; C07K 16/28; C07K 16/2827;
C07K 16/30;** C07K 2317/31; C07K 2317/35;
C07K 2317/567; C07K 2317/622; C07K 2317/92;
C07K 2317/94

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Numab Therapeutics AG
8820 Wädenswil (CH)**

(72) Inventors:
• **Warmuth, Stefan
8804 Au (CH)**

• **Hess, Christian
8055 Zurich (CH)**
• **Johansson, Maria
1026 Denges (CH)**
• **Weinert, Christopher
8400 Winterthur (CH)**

(74) Representative: **Virnekäs, Bernhard
Wallinger Ricker Schlotter Tostmann
Patent- und Rechtsanwälte Partnerschaft mbB
Zweibrückenstrasse 5-7
80331 München (DE)**

(54) **ANTIBODY VARIABLE DOMAINS AND ANTIBODIES HAVING DECREASED IMMUNOGENICITY**

(57)    The present invention relates to antibody variable domains, which exhibit a reduced binding to pre-existing anti-drug antibodies (ADA), and to antibodies comprising one or more of said antibody variable domains. The present invention further relates to nucleic acids encoding said antibody variable domains or said antibodies, vector(s) comprising said nucleic acids, host cell(s) comprising said nucleic acids or said vector(s), and a method of producing said antibody variable domains or said multispecific antibodies. Additionally, the present invention relates to pharmaceutical compositions comprising said antibodies and to methods of use thereof.

Figure 2:

EP 4 273 162 A1

## Description

### FIELD OF THE INVENTION

The present invention relates to antibody variable domains, which exhibit a reduced binding to pre-existing anti-drug antibodies (ADA), and to antibodies comprising one or more of said antibody variable domains. The present invention further relates to nucleic acids encoding said antibody variable domains or said antibodies, vector(s) comprising said nucleic acids, host cell(s) comprising said nucleic acids or said vector(s), and a method of producing said antibody variable domains or said multispecific antibodies. Additionally, the present invention relates to pharmaceutical compositions comprising said antibodies and to methods of use thereof.

### BACKGROUND OF THE INVENTION

In the past forty years since the development of the first monoclonal antibodies ("mAbs"; Köhler & Milstein, Nature, 1975, Vol.256, pp. 495-497), antibodies have become an increasingly important class of biomolecules for research, diagnostic and therapeutic purposes. Initially, antibodies were exclusively obtained by immunizing animals with the corresponding antigen of interest. While antibodies of non-human origin can be used in research and diagnostics, in therapeutic approaches the human body typically recognize non-human antibodies as foreign and raise an immune response against the non-human antibody drug substance, rendering it less or not effective. Even if the administered antibody therapeutics have been humanized, e.g. by grafting of CDRs of non-human origin into human immunoglobulin frameworks to minimize the non-human component, they can still elicit an immune response, which compromises the efficacy and/or safety of these therapeutics.

Such an immune response typically involves the binding of anti-drug antibodies (ADAs) to the therapeutic agent. These ADAs can be antibodies, which are already present in human serum (so called pre-existing ADAs) and/or antibodies, which are formed during the course of the therapy.

The risk of ADA-binding can be significantly enhanced for therapeutic antibodies that comprise or are built of portions of a naturally occurring human antibody, e.g. Fab or Fv antibody fragments. It is believed that one of the main reasons for this increase in ADA-binding is that in antibody fragments, typically a significant number of amino acids that are formerly shielded by the contact to other antibody portions or domains, become exposed to the solvent and are present to the immune system as potential epitopes.

According to literature, antibody responses in patients are dependent on the presence of both B-cell epitopes and T-cell epitopes. When a B-cell receptor recognizes and binds an antigen such as an administered therapeutic antibody, the antigen is internalized into the B cell by receptor-mediated endocytosis and undergoes proteolytic processing. The resulting peptides are subsequently presented by MHC class II molecules. Upon recognition of the T cell epitope by a T helper cell, the latter stimulates the corresponding B cells to proliferate and differentiate into antibody producing plasma cells.

Several strategies have been provided in the prior art to further lower the response of the immune system of a patient to the administered antibodies.

For example, Zhao, L. and Li, J. (2010), BMC Struct. Biol., 10, S6, disclose a method for the prediction of potential B-cell epitopes on a protein surface, based on the structural information of antibody-antigen complexes. The authors identified common structural elements that are often present in B-cell epitopes. In particular they found that in antigen epitopes recognized by antibodies, polar amino acids with flexible side chains such as arginine (R), lysin (K), asparagine (N), glutamine (Q), and histidine (H) are significantly overrepresented. Knowledge of these critical structural elements forms the basis for strategies to avoid them.

Nataga, S. and Pastan, I. (2009), Adv Drug Deliv Rev, p. 977-985, Onda, M. et al (2008), PNAS Vol 105(32): 1 1311-11316 and Mazor, R. et al (2016), Immunol Rev. Vol. 270(1): 152-64, disclose a method for reducing the immunogenicity of foreign proteins by identifying B-cell epitopes on the protein and eliminating them by mutagenesis. The authors substituted bulky hydrophilic residues such as arginine (R), glutamine (Q), glutamic acid (E) or lysin (K) within solvent exposed areas by small amino acids (such as alanine, glycine and serine).

WO2011/075861 discloses a method for decreasing the immunogenicity of antibody variable domains, in particular scFvs, by mutating one or more amino acid residues located in the interface between the variable chain and the constant chain of a corresponding full-length antibody. It is further disclosed that (i) residues that are present in turn regions of secondary structures, (ii) residues that have large, flexible side chains or a bulky side chain, or (iii) residues that are hydrophobic, are prone to be B-cell epitopes and thus elicit an immunogenic reaction, and that removal of such amino acid residues interrupts B-cell epitopes. It is further specified that the one or more amino acid residues to be substituted are leucine (L), valine (V), aspartic acid (D), phenylalanine (F), arginine (R) and/or glutamic acid (E). WO2011/075861 discloses one example of an scFv having the heavy chain point mutations L12S, L103T and L144T (AHo numbering) that, compared to the unmutated version, exhibits reduced binding to pre-existing ADAs present in human sera. Thus, WO2011/075861 teaches a method for decreasing the immunogenicity of antibody variable domains towards pre-existing ADAs by replacing small hydrophobic residues such as L and V located in the interface between the variable chain and the constant chain of a corresponding full-length antibody with small and weakly hydrophilic amino acids (such as S and T) and by avoiding large and bulky hydrophilic residues located in said interface.

Although the currently available methods provide useful indications, how the immunogenicity of antibody variable domains can be reduced, the chance of success is case-dependent and the antibody variable domains obtained by these methods often exhibit a significant residual immunogenicity. Thus, there is still a large unmet need for antibody variable domains, which exhibit low immunogenicity, and which can generally be applied in the construction of antibody fragments. More specifically, it would be desirable to have antibody variable domains at hand, which exhibiting low immunogenicity, in particular with regard to reduced binding to pre-existing ADAs, and which can generally be applied in the construction of antibody fragments. It is furthermore desirable that these antibody variable domains provide a high stability, when integrated in the final antibody format, which would allow their application in the construction of stable antibody fragments and fragment-based multispecific antibodies suitable for therapeutic development.

## SUMMARY OF THE INVENTION

It is an object of the present invention to provide antibody variable domain variants, which exhibit reduced immunogenicity, more specifically, antibody variable domain variants that are significantly less recognized by pre-existing ADAs when compared to their unmodified variants. Furthermore, these antibody variable domain variants should be highly stable to allow their application in the construction of antibody fragments and multispecific antibodies suitable for pharmaceutical development.

The inventors have now surprisingly found that antibody variable domains, which are in particular substituted at one or more of the heavy chain framework positions 101, 146 and/or 148 (according to AHo numbering) by small moderately hydrophilic amino acids, i. e. alanine (A) or serine (S), or by hydrophilic amino acids with flexible and bulky side chains, i.e. lysine (K), arginine (R), aspartate (D), glutamate (E), asparagine (N) or glutamine (Q), when being in scFv format, exhibit a significantly reduced binding to pre-existing anti-drug antibodies (ADA) present in human sera, when compared to their unsubstituted versions. It was further found that additional substitution at one or at both of the heavy chain framework positions 12 and 144 by alanine (A), lysine (K) or arginine (R) may further reduce the binding of said antibody variable domains to pre-existing anti-drug antibodies (ADA) present in human sera, when being in scFv format.

Accordingly, in a first aspect, the present invention relates to an antibody variable domain, which binds to a target antigen, comprising:

(i) a variable heavy chain (VH) comprising from N-terminus to C-terminus, the regions HFW1-HCDR1-HFW2-HCDR2-HFW3-HCDR3-HFW4, wherein each HFW designates a heavy chain framework region, and each HCDR designates a heavy chain complementarity-determining region,
wherein said variable heavy chain framework regions HFW1, HFW2, HFW3 and HFW4 are selected from a human VH framework, wherein said HFW1, HFW2, HFW3 and HFW4 have one or more substitutions selected from the group consisting of (AHo numbering):

- 　an alanine (A), serine (S), lysine (K), arginine (R), aspartate (D), glutamate (E), asparagine (N) or glutamine (Q) at amino acid position 101;
- 　an alanine (A), serine (S), lysine (K), arginine (R), aspartate (D), glutamate (E), asparagine (N) or glutamine (Q) at amino acid position 146; and
- 　a leucine (L), lysine (K), or asparagine (N) at amino acid position 148;
and

(ii) a variable light chain (VL), wherein the variable light chain comprises, from N-terminus to C-terminus, the regions LFW1-LCDR1-LFW2-LCDR2-LFW3-LCDR3-LFW4, wherein each LFW designates a light chain framework region, and each LCDR designates a light chain complementarity-determining region.

In a second aspect, the present invention relates to an antibody comprising one or more antibody variable domains of the present invention.

In a third aspect, the present invention relates to a nucleic acid or two nucleic acids encoding the antibody variable domain or the antibody of the present invention.

In an fourth aspect, the present invention relates to a vector or two vectors comprising the nucleic acid or the two nucleic acids of the present invention.

In a fifth aspect, the present invention relates to a host cell or host cells comprising the vector or the two vectors of the present invention.

In a sixth aspect, the present invention relates to a method for producing the antibody variable domain of the present invention or the antibody of the present invention, comprising (i) providing the nucleic acid or the two nucleic acids of the present invention, or the vector or the two vectors of the present invention, expressing said nucleic acid or said two nucleic acids, or said vector or vectors, and collecting said antibody variable domain or said antibody from the expression system, or (ii) providing a host cell or host cells of the present invention, culturing said host cell or said host cells; and collecting said antibody variable domain or said antibody from the cell culture.

In a seventh aspect, the present invention relates to a pharmaceutical composition comprising the antibody of the present invention and a pharmaceutically acceptable carrier.

In an eighth aspect, the present invention relates to the pharmaceutical composition of the present invention for use as a medicament.

In a ninth aspect, the present invention relates to a method for generating a modified antibody exhibiting a decreased binding to pre-existing anti-drug-antibodies (ADAs) present in human sera from healthy donors when compared to its unmodified version, and wherein the decrease in binding is determined by an ELISA-based pre-existing anti-drug-antibody binding assay, where the antibody is fragment-based or is an antibody comprising one or more scFv fragments, the method comprises the step of introducing one or more of the following substitutions (AHo numbering) in the VH sequence(s) of a fragment-based antibody or in the VH sequence(s) of the scFv fragment(s) of an antibody:

- an alanine (A), serine (S), lysine (K), arginine (R), aspartate (D), glutamate (E), asparagine (N) or glutamine (Q) at amino acid position 101;
- an alanine (A), serine (S), lysine (K), arginine (R), aspartate (D), glutamate (E), asparagine (N) or glutamine (Q) at amino acid position 146;
- a leucine (L), lysine (K), or asparagine (N) at amino acid position 148 to obtain said modified antibody.

The aspects, advantageous features and preferred embodiments of the present invention summarized in the following items, respectively alone or in combination, further contribute to solving the object of the invention:

1. An antibody variable domain, which binds to a target antigen, comprising:

(i) a variable heavy chain (VH) comprising from N-terminus to C-terminus, the regions HFW1-HCDR1-HFW2-HCDR2-HFW3-HCDR3-HFW4, wherein each HFW designates a heavy chain framework region, and each HCDR designates a heavy chain complementarity-determining region,
wherein said variable heavy chain framework regions HFW1, HFW2, HFW3 and HFW4 are selected from a human VH framework, wherein said HFW1, HFW2, HFW3 and HFW4 have one or more substitutions selected from the group consisting of (AHo numbering):

- an alanine (A), serine (S), lysine (K), arginine (R), aspartate (D), glutamate (E), asparagine (N) or glutamine (Q) at amino acid position 101;
- an alanine (A), serine (S), lysine (K), arginine (R), aspartate (D), glutamate (E), asparagine (N) or glutamine (Q) at amino acid position 146; and
- a leucine (L), lysine (K), or asparagine (N) at amino acid position 148;
and

(ii) a variable light chain (VL), wherein the variable light chain comprises, from N-terminus to C-terminus, the regions LFW1-LCDR1-LFW2-LCDR2-LFW3-LCDR3-LFW4, wherein each LFW designates a light chain framework region, and each LCDR designates a light chain complementarity-determining region.

2. The antibody variable domain of item 1, wherein said HFW1, HFW2, HFW3 and HFW4 have one or more substitutions selected from the group consisting of (AHo numbering):

- an alanine (A), serine (S), lysine (K), arginine (R), aspartate (D), glutamate (E), asparagine (N) or glutamine (Q) at amino acid position 101;
- an alanine (A), serine (S), lysine (K), arginine (R), aspartate (D), glutamate (E), asparagine (N) or glutamine (Q) at amino acid position 146; and
- a leucine (L), lysine (K), or asparagine (N) at amino acid position 148.

3. The antibody variable domain of item 1, wherein said HFW1, HFW2, HFW3 and HFW4 have one or more substitutions selected from the group consisting of (AHo numbering):

- an alanine (A), serine (S), lysine (K), arginine (R), asparagine (N) or glutamine (Q) at amino acid position 101;
- an alanine (A), serine (S), lysine (K), arginine (R), aspartate (D), glutamate (E), asparagine (N) or glutamine (Q) at amino acid position 146; and
- a leucine (L), lysine (K), or asparagine (N) at amino acid position 148.

4. The antibody variable domain of item 1, wherein said HFW1, HFW2, HFW3 and HFW4 have one or more substitutions selected from the group consisting of (AHo numbering):

- an alanine (A), serine (S), lysine (K), arginine (R), asparagine (N) or glutamine (Q) at amino acid position 101;
- a lysine (K), aspartate (D), glutamate (E) arginine (R), or glutamine (Q) at amino acid position 146; and
- a lysine (K) at amino acid position 148.

5. The antibody variable domain of any one of items 1 to 4, wherein said HFW1, HFW2, HFW3 and HFW4 additionally have one or two substitutions selected from the group consisting of (AHo numbering):

- an alanine (A), lysine (K) or arginine (R) at amino acid position 12, particularly an alanine (A) or arginine (R) at amino acid position 12; and
- an alanine (A), lysine (K) or arginine (R) at amino acid position 144.

6. The antibody variable domain of any one of the preceding items, wherein

a. said variable light chain framework regions LFW1, LFW2, LFW3 and LFW4 are selected from a human antibody Vκ framework or a human antibody Vλ framework, in particular a human antibody Vκ framework; or
b. said variable light chain framework regions LFW1, LFW2 and LFW3 are selected from a human antibody Vκ framework, and said variable light chain framework region LFW4 is selected from a Vλ framework.

7. The antibody variable domain of any one of the preceding items, wherein

a. said variable light chain framework regions LFW1, LFW2, LFW3 and LFW4 are selected from a human antibody Vκ framework; or
b. said variable light chain framework regions LFW1, LFW2 and LFW3 are selected from a human antibody Vκ framework, and said variable light chain framework region LFW4 is selected from a Vλ framework,

and wherein said LFW3 has the following substitution (AHo numbering):

- a glutamate (E), arginine (R) or a glutamine (Q) at amino acid position 101.

8. The antibody variable domain of item 1, wherein said HFW1, HFW2, HFW3 and HFW4 have one of the following substitutions (AHo numbering):

a. an alanine (A), serine (S), lysine (K), arginine (R), asparagine (N) or glutamine (Q) at amino acid position 101;
b. an alanine (A), serine (S), lysine (K), arginine (R), aspartate (D), glutamate (E), asparagine (N) or glutamine (Q) at amino acid position 146;
c. an alanine (A), serine (S), lysine (K), arginine (R), asparagine (N) or glutamine (Q) at amino acid position 101; and
an alanine (A), serine (S), lysine (K), arginine (R), aspartate (D), glutamate (E), asparagine (N) or glutamine (Q) at amino acid position 146;
d. an alanine (A), lysine (K) or arginine (R) at amino acid position 144; and
a leucine (L), lysine (K) or asparagine (N) at amino acid position 148;
e. an alanine (A), lysine (K) or arginine (R) at amino acid position 144;

an alanine (A), serine (S), lysine (K), arginine (R), aspartate (D), glutamate (E), asparagine (N) or glutamine (Q) at amino acid position 146; and
a leucine (L), lysine (K) or asparagine (N) at amino acid position 148;

f. an alanine (A), serine (S), lysine (K), arginine (R), asparagine (N) or glutamine (Q) at amino acid position 101;

an alanine (A), lysine (K) or arginine (R) at amino acid position 144; and

an alanine (A), serine (S), lysine (K), arginine (R), aspartate (D), glutamate (E), asparagine (N) or glutamine (Q) at amino acid position 146.

g. an alanine (A) or arginine (R) at amino acid position 12;

an alanine (A), serine (S), lysine (K), arginine (R), asparagine (N) or glutamine (Q) at amino acid position 101;
an alanine (A), lysine (K) or arginine (R) at amino acid position 144; and
an alanine (A), serine (S), lysine (K), arginine (R), aspartate (D), glutamate (E), asparagine (N) or glutamine (Q) at amino acid position 146.

9. The antibody variable domain of item 1, wherein said HFW1, HFW2, HFW3 and HFW4 have one of the following substitutions (AHo numbering):

a. an alanine (A), lysine (K), arginine (R) or asparagine (N) at amino acid position 101;
b. a lysine (K), arginine (R), aspartate (D), glutamate (E) or glutamine (Q) at amino acid position 146;
c. a serine (S), lysine (K), arginine (R), asparagine (N) or glutamine (Q) at amino acid position 101; and
a lysine (K), aspartate (D), glutamate (E) or glutamine (Q) at amino acid position 146;
d. an alanine (A) or lysine (K) at amino acid position 144; and
a leucine (L) or asparagine (N) at amino acid position 148;
e. a lysine (K) at amino acid position 144;

a glutamate (E) at amino acid position 146; and
a lysine (K) at amino acid position 148;

f. a serine (S), arginine (R) or glutamine (Q) at amino acid position 101;

an alanine (A) or lysine (K) at amino acid position 144; and
an arginine (R), or glutamine (Q) at amino acid position 146

g. an alanine (A) at amino acid position 12;

a serine (S), arginine (R) or glutamine (Q) at amino acid position 101;
an alanine (A) or lysine (K) at amino acid position 144; and
an arginine (R), or glutamine (Q) at amino acid position 146.

10. The antibody variable domain of any one of items 1 to 9, wherein said variable heavy chain framework regions HFW1, HFW2, HFW3 and HFW4 are selected from the human VH framework subtypes VH1a, VH1b, VH3, VH4, VH5 and VH6.

11. The antibody variable domain of any one of items 1 to 9, wherein said variable heavy chain framework regions HFW1, HFW2, HFW3 and HFW4 are selected from the human VH framework subtypes VH1a, VH1b, VH3 and VH4.

12. The antibody variable domain of any one of items 1 to 9, wherein said variable heavy chain framework regions HFW1, HFW2, HFW3 and HFW4 are of the human VH framework subtype VH3.

13. The antibody variable domain of any one of items 1 to 9, wherein said variable heavy chain framework regions HFW1, HFW2, HFW3 and HFW4 are selected from

a. the combination of framework regions HFW1, HFW2, HFW3 and HFW4 (*i. e.* the non-italicized residues in Table 5) of any one of the SEQ ID NOs: 157, 158, 159, 160, 161, 162, 163, 164, 165, 166, 167, 168, 169, 170, 171, 172, 173, 174 and 175; and
b. the combination of framework regions HFW1, HFW2, HFW3 and HFW4 (*i. e.* the non-italicized residues in Table 5) of any one of the SEQ ID NOs: 157, 158, 159, 160, 161, 162, 163, 164, 165, 166, 167, 168, 169, 170, 171, 172, 173, 174 and 175 having 1, 2 or 3 mutations within the framework regions at positions different from 12, 101, 144, 146 and 148 (AHo numbering);

and wherein said HFW1, HFW2, HFW3 and HFW4 have one or more of the substitutions as defined in any one of items 1 to 9.

14. The antibody variable domain of any one of items 1 to 9, wherein said variable heavy chain framework regions HFW1, HFW2, HFW3 and HFW4 are selected from

   a. the combination of framework regions HFW1, HFW2, HFW3 and HFW4 (*i. e.* the non-italicized residues in Table 5) of any one of the SEQ ID NOs: 157, 158, 159, 160, 161, 162, 163, 164, 165, 166, 167, 168, 169 and 170; and
   b. the combination of framework regions HFW1, HFW2, HFW3 and HFW4 (*i. e.* the non-italicized residues in Table 5) of any one of the SEQ ID NOs: 157, 158, 159, 160, 161, 162, 163, 164, 165, 166, 167, 168, 169 and 170 having 1, 2 or 3 mutations within the framework regions at positions different from 12, 101, 144, 146 and 148 (AHo numbering);

and wherein said HFW1, HFW2, HFW3 and HFW4 have one or more of the substitutions as defined in any one of items 1 to 9.

15. The antibody variable domain of any one of the preceding items, wherein said variable heavy chain framework regions HFW1, HFW2, HFW3 and HFW4 are selected from

   a. the combination of framework regions HFW1, HFW2, HFW3 and HFW4 (*i. e.* the non-italicized residues in Tables 1 and 3) of any one of the SEQ ID NOs: 5 - 36 and 83 - 114, particularly of any one of the SEQ ID NOs: 8 - 36 and 86 - 114; and
   b. the combination of framework regions HFW1, HFW2, HFW3 and HFW4 (*i. e.* the non-italicized residues in Tables 1 and 3) of any one of the SEQ ID NOs: 5 - 36 and 83 - 114, particularly of any one of the SEQ ID NOs: 8 - 36 and 86 - 114, having 1, 2 or 3 mutations within the framework regions at positions different from 12, 101, 144, 146 and 148 (AHo numbering).

16. The antibody variable domain of any one of the preceding items, wherein said variable heavy chain framework regions HFW1, HFW2, HFW3 and HFW4 are selected from

   a. the combination of framework regions HFW1, HFW2, HFW3 and HFW4 (*i. e.* the non-italicized residues in Tables 1 and 3) of any one of the SEQ ID NOs: 8 - 25, 28, 30 - 36, 86 - 103, 106 and 108 - 114, particularly of any one of the SEQ ID NOs: 8 - 25 and 86 - 103; and
   b. the combination of framework regions HFW1, HFW2, HFW3 and HFW4 (*i. e.* the non-italicized residues in Tables 1 and 3) of any one of the SEQ ID NOs: 8 - 25, 28, 30 - 36, 86 - 103, 106 and 108 - 114, particularly of any one of the SEQ ID NOs: 8 - 25 and 86 - 103, having 1, 2 or 3 mutations within the framework regions at positions different from 12, 101, 144, 146 and 148 (AHo numbering).

17. The antibody variable domain of any one of the preceding items, wherein the human antibody Vκ framework is selected from the Vκ1 framework subtype.

18. The antibody variable domain of any one of the preceding items, wherein LFW1, LFW2 and LFW3, and optionally also LFW4, if LFW4 is selected from a human antibody Vκ framework subtype, are selected from

   a. the combination of framework regions LFW1, LFW2, LFW3 and optionally LFW4 (*i. e.* the non-italicized residues in Table 5) of any one of the SEQ ID NOs: 176, 177, 178 and 179; and
   b. the combination of framework regions LFW1, LFW2, LFW3 and optionally LFW4 (*i. e.* the non-italicized residues in Table 5) of any one of the SEQ ID NOs: 176, 177, 178 and 179 having 1, 2 or 3 mutations within the framework region at positions different from 101 (AHo numbering);

and wherein said LFW3 optionally has the following substitution (AHo numbering):

- a glutamate (E), arginine (R) or a glutamine (Q) at amino acid position 101.

19. The antibody variable domain of any one of the preceding items, wherein said variable light chain framework region LFW4 is selected from a Vλ framework subtype, particularly has a sequence selected from the group consisting of SEQ ID NOs: 188, 189, 190, 191, 192, 193, 194, 195 and 196.

20. The antibody variable domain of any one of the preceding items, wherein said variable light chain framework regions LFW1, LFW2, LFW3 and LFW4 are selected from

a. the combination of framework regions LFW1, LFW2, LFW3 and LFW4 (*i. e.* the non-italicized residues in Tables 1, 3 and 5) of any one of the SEQ ID NOs: 37, 38, 39, 115, 116, 117, 180, 181, 182, 183, 184, 185, 186 and 187; and

b. the combination of framework regions LFW1, LFW2, LFW3 and LFW4 (*i. e.* the non-italicized residues in Tables 1, 3 and 5) of any one of the SEQ ID NOs: 37, 38, 39, 115, 116, 117, 180, 181, 182, 183, 184, 185, 186 and 187 having 1, 2 or 3 mutations within the framework region at positions different from 101 (AHo numbering).

21. The antibody variable domain of any one of the preceding items, wherein the format of said antibody variable domain is selected from an Fv, a dsFv and an scFv, particularly from an Fv and scFv.

22. The antibody variable domain of any one of the preceding items, wherein said antibody variable domain, when being in scFv format, exhibits a reduced binding to pre-existing anti-drug antibodies (ADA) present in human sera when compared to a version of said antibody variable domain that does not comprise the substitutions defined in item 1, as determined in a pre-existing ADA-binding assay.

23. The antibody variable domain of any one of items 13 to 16 and 20, wherein said antibody variable domain, when being in scFv format, is further characterized by one or more of the following features:

a. has a melting temperature (Tm), determined by differential scanning fluorimetry (DSF), of at least 63°C, particularly of at least 64°C, particularly of at least 65°C, when formulated in 20 mM Histidine, pH 6.0;

b. has a loss in monomer content, after storage for 28 days at 4°C, of less than 5 %, particularly of less than 3 %, particularly of less than 2 %, when formulated at a concentration of 10 mg/ml in 20 mM Histidine at pH 6.0;

c. has a loss in monomer content, after storage for 28 days at 40°C, of less than 15 %, particularly of less than 10 %, particularly of less than 8 %, when formulated at a concentration of 10 mg/ml in 20 mM Histidine at pH 6.0;

d. has a loss in protein content, after storage for 28 days at 4°C, of less than 15 %, particularly of less than 10 %, when formulated at a concentration of 10 mg/ml in 20 mM Histidine, pH 6.0;

e. has a loss in protein content, after storage for 28 days at 40°C, of less than 10 %, particularly of less than 5 %, when formulated at a concentration of 10 mg/ml in 20 mM Histidine, pH 6.0;

f. binds its target antigen with a dissociation constant (KD) that is lower or not greater than 3-folds, when compared with the KD of a version of said antibody variable domain that does not comprise the substitutions defined in item 1, as measured by surface plasmon resonance (SPR).

24. An antibody comprising one or more antibody variable domains as defined in any one of items 1 to 23.

25. The antibody of item 24, wherein the antibody is

(i) monospecific and monovalent, bivalent or trivalent for its target antigen;
(ii) bispecific and, independently of each other, monovalent or bivalent for each target antigen;
(iii) trispecific and monovalent for each target antigen;
(iv) trispecific and bivalent for one of the target antigens and monovalent for the other target antigens;
(v) trispecific and bivalent for two of the target antigens and monovalent for the third target antigen;
(vi) tetraspecific and monovalent for each target antigen;
(vii) tetraspecific and bivalent for one of the target antigens and monovalent for the other target antigens; or
(viii) tetraspecific and bivalent for two of the target antigens and monovalent for the other target antigens.

26. The antibody of item 24, wherein the antibody comprises

(i) a first and a second antibody variable domain as defined in any of items 1 to 22, wherein said first and said second antibody variable domains have specificity for two different target antigens; and
(ii) one, two, three or four further antibody variable domains as defined in any of items 1 to 19, having, independently of each other, either specificity for one of the target antigens of the first and second variable domains, or specificity for a target antigen different from the target antigens of the first and second variable domains.

27. The antibody of any one of items 24 to 26, wherein the format of said antibody is selected from Fc-bearing bivalent bispecific formats, from Fc-bearing trivalent bispecific and tetravalent bispecific formats, and from trivalent bispecific IgG formats and tetravalent bispecific IgG formats, wherein said trivalent or tetravalent formats comprise one or more Fv, scFv or disulfide stabilized scFv;

more particularly wherein the format of said antibody is selected from an Fc-bearing KiH-based format; DVD-Ig;

CODV-IgG and Morrison (IgG CH$_3$-scFv fusion (Morrison-H) or IgG CL-scFv fusion (Morrison-L)), even more particularly from DVD-Ig and Morrison (IgG CH$_3$-scFv fusion (Morrison-H) or IgG CL-scFv fusion (Morrison-L)).

28. The antibody of item 27, wherein the IgG is selected from the IgG subclasses IgG1 and IgG4, in particular IgG4.

29. The antibody of any one of items 24 to 26, wherein said antibody does not comprise an immunoglobulin Fc region.

30. The antibody of item 29, wherein said antibody is in a format selected from the group consisting of: a tandem scDb (Tandab), a linear dimeric scDb (LD-scDb), a circular dimeric scDb (CD-scDb), a tandem tri-scFv, a tribody (Fab-(scFv)2), a Fab-Fv2, a triabody, an scDb-scFv, a tetrabody, a didiabody, a tandem-di-scFv and a MATCH.

31. The antibody of item 29, wherein said antibody does not comprise CH1 and/or CL regions.

32. The antibody of item 31, wherein said antibody is in a scDb-scFv, a triabody, a tetrabody or a MATCH format, in particular wherein said antibody is in a MATCH or scDb-scFv format, more particularly wherein said antibody is in a MATCH format, more particularly a MATCH3 or a MATCH4 format.

33. The antibody of any one of the items 24 to 31, wherein said antibody exhibits a reduced binding to pre-existing anti-drug antibodies (ADA) present in human sera, in particular reduced binding to pre-existing ADAs when compared to a reference antibody which does not comprise the substitutions defined in item 1, as determined in a pre-existing ADA-binding assay.

34. A nucleic acid or two nucleic acids encoding the antibody variable domain of any one of items 1 to 23, or the antibody of any one of items 24 to 33.

35. A vector or two vectors comprising the nucleic acid or the two nucleic acids of item 34.

36. A host cell or host cells comprising the vector or the two vectors of item 35.

37. A method for producing the antibody variable domain of any one of items 1 to 23, or the antibody of any one of items 24 to 33, comprising (i) providing the nucleic acid or the two nucleic acids of item 34, or the vector or the two vectors of item 35, expressing said nucleic acid sequence or nucleic acids, or said vector or vectors, and collecting said antibody variable domain or said antibody from the expression system, or (ii) providing a host cell or host cells of item 35, culturing said host cell or said host cells; and collecting said antibody variable domain or said antibody from the cell culture.

38. A pharmaceutical composition comprising the antibody of any one of items 24 to 33 and a pharmaceutically acceptable carrier.

39. The antibody of any one of items 24 to 33, or the pharmaceutical composition of item 38, for use as a medicament.

40. A method for generating a modified antibody exhibiting a decreased binding to pre-existing anti-drug-antibodies (ADAs) present in human sera from healthy donors when compared to its unmodified version, and wherein the decrease in binding is determined by an ELISA-based pre-existing anti-drug-antibody binding assay, where the antibody is fragment-based or is an antibody comprising one or more scFv fragments, the method comprises the step of introducing one or more of the following substitutions (AHo numbering) in the VH sequence(s) of a fragment-based antibody or in the VH sequence(s) of the scFv fragment(s) of an antibody:

- an alanine (A), serine (S), lysine (K), arginine (R), aspartate (D), glutamate (E), asparagine (N) or glutamine (Q) at amino acid position 101;
- an alanine (A), serine (S), lysine (K), arginine (R), aspartate (D), glutamate (E), asparagine (N) or glutamine (Q) at amino acid position 146;
- a leucine (L), lysine (K), or asparagine (N) at amino acid position 148 to obtain said modified antibody.

41. The method of item 40, wherein said modified antibody comprises an antibody variable domain as defined in any one of the items 1 to 23.

**BRIEF DESCRIPTION OF THE DRAWINGS**

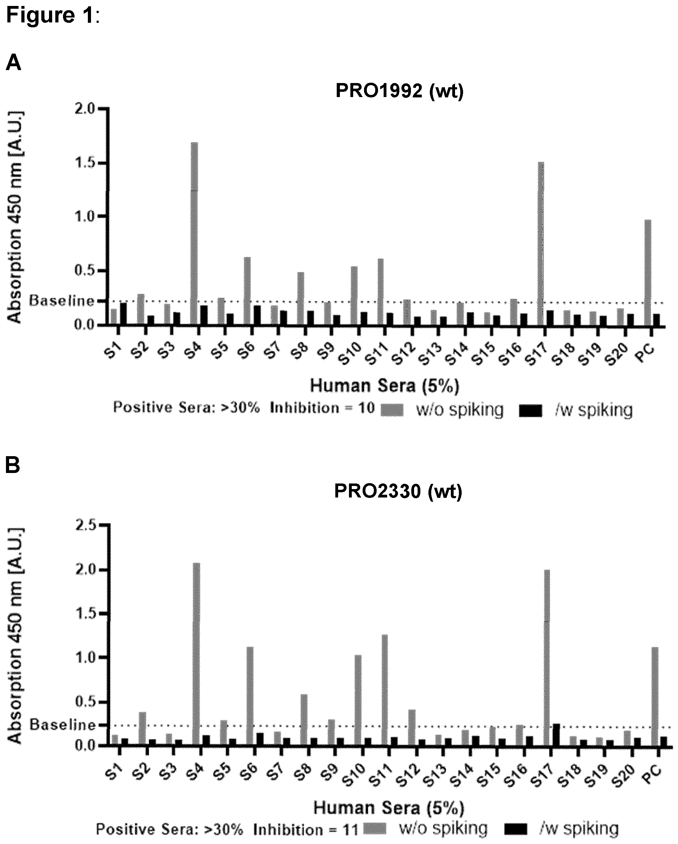

**FIG. 1** shows the absorption levels of pre-existing ADAs in 20 human serum samples for PRO1922 (A) and PRO2230 (B), determined by the ELISA-based pre-existing ADA-binding assay described in Example 2. The measurements were performed with spiked and unspiked serum samples (confirmation assay setup). The difference between spiked and non-spiked sera has to be greater than 30%. Each spiked serum has to be above the baseline to be counted as significant. The baseline consists of the mean of all spiked sera for a sample with three standard deviations added. Absorption levels measured via 280 nm in A.U.

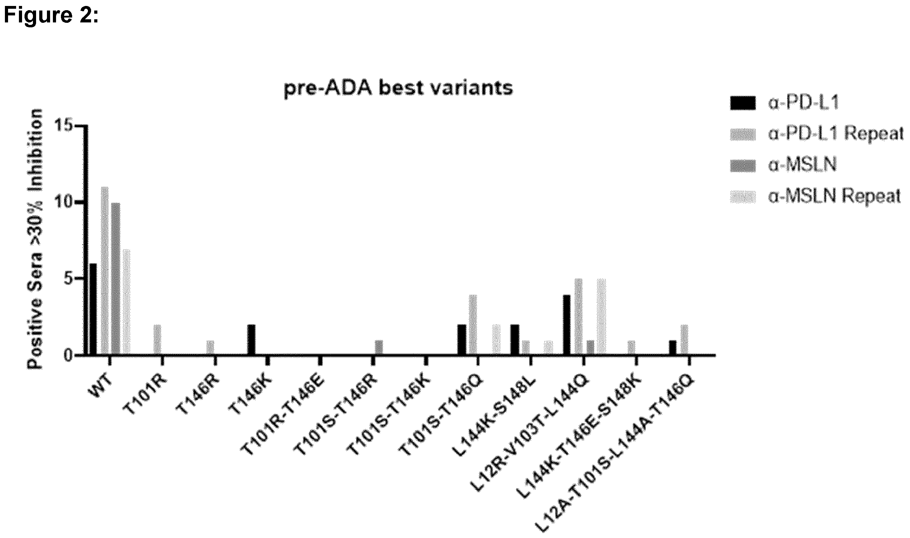

**FIG. 2** shows the number of positive sera out of 20 human serum samples for ten PRO2330 ($\alpha$-PD-L1 and $\alpha$-PD-L1 Repeat) scFv variants and ten PRO1992 (a-MSLN and $\alpha$-MSLN Repeat) scFv variants compared to their respective wt (PRO2330 and PRO1992) and L12R-V103T-L11Q references, as determined by two rounds of the ELISA-based pre-existing ADA-binding assay described in example 2. The measurements were performed with spiked and unspiked serum samples. The difference between spiked and non-spiked sera has to be greater than 30%. Each spiked serum has to be above the baseline to be counted as significant. The baseline consists of the mean of all spiked sera for a sample with three standard deviations added. No bar for a variant means zero positive sera for pre-ADAs.

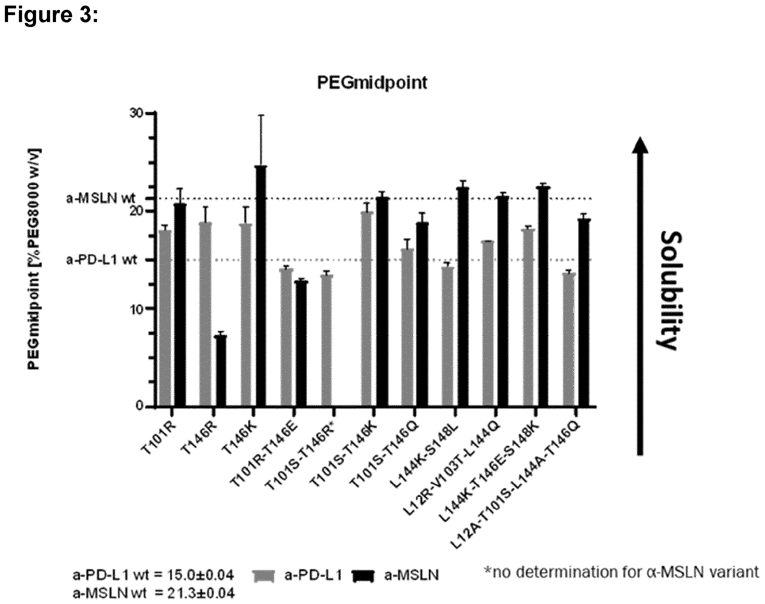

**FIG. 3** shows the calculated Midpoint [%] of the PEG precipitation curve as a function for ten PRO2330 (a-PD-L1) scFv variants and ten PRO1992 (a-MSLN) scFv variants compared to their respective PRO2330 and PRO1992 wild-types (a-PD-L1 wt and a-MSLN wt) and L12R-V103T-L11Q references. The midpoint is calculated as half-maximal precipitation PEG concentration with a confidence interval of 95% for the mean (CI 95%). Error bars represent the standard error correlated to fitting a sigmoidal function to the experimental data.

**DETAILED DESCRIPTION OF THE INVENTION**

The inventors have surprisingly found that antibody variable domains, which are substituted at one or more of the heavy chain framework positions 101, 146 and 148 (according to AHo numbering) by small moderately hydrophilic amino acids, i. e. alanine (A) or serine (S), or by hydrophilic amino acids with flexible and bulky side chains, i.e. lysine (K), arginine (R), aspartate (D), glutamate (E), asparagine (N) or glutamine (Q), when being in scFv format, exhibit a significantly reduced binding to pre-existing anti-drug antibodies (ADA) present in human sera, when compared to their unsubstituted versions. It was further found that additional substitution at one or at both of the heavy chain framework positions 12 and 144 by alanine (A), lysine (K) or arginine (R) may further reduce the binding of said antibody variable domains, when being in scFv format, to pre-existing anti-drug antibodies (ADA) present in human sera.

Despite that fact that many methods and strategies have been developed to reduce the immunogenicity of antibody variable domains, the immunogenicity reductions achievable with these methods are often not as strong as would be desirable from a pharmaceutical development perspective. Thus, there is still a large unmet need for antibody variable domains, which exhibit low immunogenicity. More specifically, it would be desirable to have antibody variable domains at hand, which exhibiting low immunogenicity, in particular with regard to reduced binding to pre-existing ADAs, and which can generally be applied in the construction of antibody fragments. It is furthermore desirable that these antibody variable domains provide a high stability, when integrated in the final antibody format, which would allow their application in the construction of stable antibody fragments and fragment-based multispecific antibodies suitable for therapeutic development.

The antibody variable domain of the present invention could be successfully used for the construction of several highly stable and functional scFvs that can readily be used in the construction of antibody fragment-based multispecific antibody formats.

Unless defined otherwise, all technical and scientific terms used herein have the same meaning as commonly understood by those of ordinary skill in the art to which this invention pertains.

The terms "comprising" and "including" are used herein in their open-ended and non-limiting sense unless otherwise noted. With respect to such latter embodiments, the term "comprising" thus includes the narrower term "consisting of".

The terms "a" and "an" and "the" and similar references in the context of describing the invention (especially in the context of the following claims) are to be construed to cover both the singular and the plural, unless otherwise indicated herein or clearly contradicted by context. For example, the term "a cell" includes a plurality of cells, including mixtures thereof. Where the plural form is used for compounds, salts, and the like, this is taken to mean also a single compound, salt, or the like.

In a first aspect, the present invention relates to an antibody variable domain, which binds to a target antigen, comprising:

(i) a variable heavy chain (VH) comprising from N-terminus to C-terminus, the regions HFW1-HCDR1-HFW2-HCDR2-HFW3-HCDR3-HFW4, wherein each HFW designates a heavy chain framework region, and each HCDR designates a heavy chain complementarity-determining region,
wherein said variable heavy chain framework regions HFW1, HFW2, HFW3 and HFW4 are selected from a human VH framework, wherein said HFW1, HFW2, HFW3 and HFW4 have one or more substitutions selected from the group consisting of (AHo numbering):

- an alanine (A), serine (S), lysine (K), arginine (R), aspartate (D), glutamate (E), asparagine (N) or glutamine (Q) at amino acid position 101;
- an alanine (A), serine (S), lysine (K), arginine (R), aspartate (D), glutamate (E), asparagine (N) or glutamine (Q) at amino acid position 146; and
- a leucine (L), lysine (K), or asparagine (N) at amino acid position 148;
and

(ii) a variable light chain (VL), wherein the variable light chain comprises, from N-terminus to C-terminus, the regions LFW1-LCDR1-LFW2-LCDR2-LFW3-LCDR3-LFW4, wherein each LFW designates a light chain framework region, and each LCDR designates a light chain complementarity-determining region.

The term "antibody" and the like, as used herein, includes whole antibodies or single chains thereof; and any antigen-binding variable domain (i. e., "antigen-binding portion") or single chains thereof; and molecules comprising antibody CDRs, VH regions or VL regions (including without limitation multispecific antibodies). A naturally occurring "whole antibody" is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDRs), flanked by regions that are more conserved, termed framework regions (FRs). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e. g., effector cells) and the first component (Clq) of the classical complement system.

The term "antibody variable domain", as used herein, refers to one or more parts of an intact antibody that have the ability to specifically bind to a given antigen (e. g., PDL1, CD137, ROR1, MSLN, CD3, IL-4R, IL-31 or hSA). This can be any antigen-binding fragment (i. e., "antigen-binding portion") of an intact antibody or single chains thereof; and molecules comprising antibody CDRs, VH regions or VL regions. Specifically, in case of the multispecific antibodies of the present invention, the term "antibody variable domain", as used herein, refers in particular to an Fv fragment consisting of the VL and VH domains of a single arm of an antibody (Fv); a disulfide stabilized Fv fragment (dsFv); a single chain Fv fragment (scFv); and a single chain Fv fragment having an additional light chain constant domain ($C_L$) fused to it (scAB). Preferably, the antibody variable domain of the present invention is selected from an Fv fragment, a disulfide stabilized Fv fragment (dsFv) and an scFv fragment. In particular embodiments, the antibody variable domain of the present invention is a single-chain Fv fragment (scFv). In other particular embodiments, the VL and VH domains of the scFv fragment are stabilized by an interdomain disulfide bond, in particular said VH domain comprises a single cysteine residue in position 51 (AHo numbering) and said VL domain comprises a single cysteine residue in position 141 (AHo numbering).

The term "Complementarity Determining Regions" ("CDRs") refers to amino acid sequences with boundaries determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273, 927-948 ("Chothia" numbering scheme); ImMunoGenTics (IMGT) numbering (Lefranc, M.-P., The Immunologist, 7, 132-136 (1999); Lefranc, M.-P. et al., Dev. Comp. Immunol., 27, 55-77 (2003)) ("IMGT" numbering scheme); and the numbering scheme described in Honegger & Plückthun, J. Mol. Biol. 309 (2001) 657-670 ("AHo" numbering). For example, for classic formats, under Kabat, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under Chothia the CDR amino acids in the VH are numbered 26-32 (HCDR1), 52-56 (HCDR2), and 95-102 (HCDR3); and the amino acid residues in VL are numbered 24-34 (LCDR1), 50-56 (LCDR2), and 89-97 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. Under IMGT the CDR amino acid residues in the VH are numbered approximately 26-35 (HCDR1), 51-57 (HCDR2) and 93-102 (HCDR3), and the CDR amino acid residues in the VL are numbered approximately 27-32 (LCDR1), 50-52 (LCDR2), and 89-97 (LCDR3) (numbering according to "Kabat"). Under IMGT, the CDRs of an antibody can be determined using the program IMGT/DomainGap Align.

In the context of the present invention, the numbering system suggested by Honegger & Plückthun ("AHo") is used (Honegger & Plückthun, J. Mol. Biol. 309 (2001) 657-670), unless specifically mentioned otherwise. In particular, the following residues are defined as CDRs according to AHo numbering scheme: LCDR1 (also referred to as CDR-L1): L24-L42; LCDR2 (also referred to as CDR-L2): L58-L72; LCDR3 (also referred to as CDR-L3): L107-L138; HCDR1 (also referred to as CDR-H1): H27-H42; HCDR2 (also referred to as CDR-H2): H57-H76; HCDR3 (also referred to as CDR-H3): H108-H138. Correspondingly, in the context of the present invention, the following residues are defined as light chain frameworks 1 to 4 (LFW1-LFW4) and heavy chain frameworks 1 to 4 (HFW1-HFW4), respectively, according to AHo numbering scheme: LFW1: L1-L23; LFW2: L43-L57; LFW3: L73-L106; LFW4: L139-L149; HFW1: H1-H26; HFW2: H43-H56; HFW3: H77-H107; HFW4: H139-H149. For the sake of clarity, the numbering system according to Honegger & Plückthun takes the length diversity into account that is found in naturally occurring antibodies, both in the different VH and VL subfamilies and, in particular, in the CDRs, and provides for gaps in the sequences. Thus, in a given antibody variable domain usually not all positions 1 to 149 will be occupied by an amino acid residue.

The term "binds to" as used herein refers to the ability of an individual antibody to react with an antigenic determinant. However, this does not exclude that said individual antibody can for example also react with homologues of said antigenic determinant (e. g. with antigen determinants from other species) or with other antigen determinants belonging to the same protein family.

The term "binding specificity" as used herein refers to the ability of an individual antibody to react with one antigenic determinant and not with a different antigenic determinant. As used herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. However, also these terms do not exclude that said individual antibody can bind with comparable affinity to the same antigen determinants from different species. In its most general form (and when no defined reference is mentioned), "specific binding" is referring to the ability of the antibody to discriminate between the target of interest and an unrelated molecule, as determined, for example, in accordance with specificity assay methods known in the art. Such methods comprise, but are not limited to Western blots, ELISA, RIA, ECL, IRMA, SPR (Surface plasmon resonance) tests and peptide scans. For example, a standard ELISA assay can be carried out. The scoring may be carried out by standard color development (e. g. secondary antibody with horseradish peroxide and tetramethyl benzidine with hydrogen peroxide). The reaction in certain wells is scored by the optical density, for example, at 450 nm. Typical background (= negative reaction) may be about 0.1 OD; typical positive reaction may be about 1 OD. This means the ratio between a positive and a negative score can be 10-fold or higher. In a further example, an SPR assay can be carried out, wherein at least 10-fold, particularly at least 100-fold difference between a background and signal indicates on specific binding. Typically, determination of binding specificity is performed by using not a single reference molecule, but a set of about three to five unrelated molecules, such as milk powder, transferrin or the like.

The antibody variable domains of the present invention bind to a target antigen, which can be any target antigen. Examples of target antigens include, but are not limited to: a transmembrane molecule; a receptor; a ligand; a growth factor; a growth hormone; a clotting factor; an anti-clotting factor; a plasminogen activator; a serum albumin; a receptor for a hormone or a growth factor; a neurotrophic factor; a nerve growth factor; a fibroblast growth factor; a CD protein; an interferon; a colony stimulating factor (CSF); an interleukin (IL); a T-cell receptor; a T-cell co-stimulatory receptor, such as CD137; a surface membrane protein; a viral protein; a tumor associated antigen; an integrin or an interleukin; VEGF; a renin; a human growth hormone; a bovine growth hormone; a growth hormone releasing factor; parathyroid hormone; thyroid stimulating hormone; a lipoprotein; alpha-1-antitrypsin; insulin A-chain; insulin B-chain; proinsulin; follicle stimulating hormone; calcitonin; luteinizing hormone; glucagon; clotting factor VIIIC; clotting factor IX; tissue factor (TP); von Willebrand's factor; Protein C; atrial natriuretic factor; a lung surfactant; urokinase; human urine; tissue-type plasminogen activator (t-PA); bombesin; thrombin; hemopoietic growth factor; tumor necrosis factor-alpha or -beta; enkephalinase; RANTES (Regulated on Activation Normally T-cell Expressed and Secreted); human macrophage inflammatory protein (MIP-I)-alpha; Muellerian-inhibiting substance; relaxin A-chain; relaxin B-chain; prorelaxiri; mouse gonadotropin-associated peptide; a microbial protein, beta-lactamase; DNase; IgE; a cytotoxic T-lymphocyte associated antigen (CTLA); CTLA-4; inhibin; activin; vascular endothelial growth factor (VEGF); protein A or D; a rheumatoid factor; bone-derived neurotrophic factor (BDNF); neurotrophin-3, -4, -5, or -6 (NT-3, NT-4, NT-5, or NT-6); NGF-beta; platelet-derived growth factor (PDGF); aFGF; bFGF; epidermal growth factor (EGF); TGF-alpha; TGF-beta, including TGF-beta1, TGF-beta2, TGF-beta3, TGF-betaI4, or TGF-beta5; insulin-like growth factor-I or -II (IGF-I or IGF-II); des(I-3)-IGF-I (brain IGF-I), an insulin-like growth factor binding protein, erythropoietin; an osteoinductive factor; an immunotoxin; a bone morphogenetic protein (BMP); interferon-alpha, -beta, or -gamma; M-CSF, GM-CSF or G-CSF; IL-1 to IL-10; superoxide dismutase; decay accelerating factor; an AIDS envelope protein; a transport protein; a homing receptor; an addressin; a regulatory protein; CD3, CD4, CD8, CDI Ia, CDI Ib, CDI Ic, CD18, CD19, CD20, CD34, CD40, or CD46, an ICAM, VLA-4 or VCAM; or HER2, HER3 or HER4 receptor; a member of the ErbB receptor family; an EGF receptor; HER2, HER3 or HER4 receptor; a cell adhesion molecule; LFA-1, Mac1, pI50.95, VLA-4, ICAM-1, VCAM, alpha4/beta7 integrin or alphav/beta3 integrin; an alpha or beta subunit of a cell adhesion molecule; antibodies); a growth factor, VEGF; tissue factor (TF); TGF-beta; alpha interferon (alpha-IFN); IL-8; IgE; blood group antigens Apo2; death receptor, such as PD-1; death receptor ligands, such as PD-L1; flk2/flt3 receptor; obesity (OB) receptor; mpl receptor; CTLA4 or protein C.

The term "tumor-associated antigen (TAA)" refers to an antigen that is expressed on the surface of a tumor cell. In particular embodiments, a TAA is an antigen that is preferentially expressed on a tumor cell when compared to non-tumor cells, particularly wherein expression of the TAA on a tumor cell is at least more than 5-fold, at least more than 10-fold, at least more than 20-fold, at least more than 50- fold, or at least more than 100-fold higher than on non-tumor cells from the same organism or patient.

Examples of tumor associated antigen targets include, but are not limited to: ADRB3, AFP, ALK, BCMA, beta human chorionic gonadotropin, CA-125 (MUC16), CAIX, CD123, CD133, CD135, CD135 (FLT3), CD138, CD171, CD19, CD20, CD22, CD24, CD276, CD33, CD33, CD38, CD44v6, CD79b, CD97, CDH3 (cadherin 3), CEA, CEACAM6, CLDN6, CLEC12A (CLL1), CSPG4, CYP1B1, EGFR, EGFRvIII, EPCAM, EPHA2, Ephrin B2, ERBBs (e. g. ERBB2), FAP, FGFR1, folate receptor alpha, folate receptor beta, Fos-related antigen, GA733, GD2, GD3, GFRalpha4, globoH, GPC3, GPR20, GPRC5D, HAVCR1, Her2/neu (HER2), HLA-A2, HMWMAA, HPV E6 or E7, human telomerase reverse transcriptase, IL-11Ra, IL-13Ra2, intestinal carboxyl esterase, KIT, Legumain, LewisY, LMP2, Ly6k, MAD-CT-1, MAD-CT-2, ML-IAP, MN-CA IX, MSLN, MUC1, mut hsp 70-2, NA-17, NCAM, neutrophil elastase, NY-BR-1, NY-ESO-1, o-acetyl-GD2, OR51E2, PANX3, PDGFR-beta, PLAC1, Polysialic acid, PSCA, PSMA, RAGE1, ROR1, sLe, sperm protein 17, SSEA-4, SSTR2, sTn antigen, sTn-OGlycopeptides, TAG72, TARP, TEM1/CD248, TEM7R, thyroglobulin, Tn antigen, Tn-O-Glycopeptides, TPBG (5T4), TRP-2, TSHR, UPK2 and VEGFR2. Preferred examples are: CD138, CD79b, TPBG (5T4), HER2, MSLN, MUC1, CA-125 (MUC16), PSMA, BCMA, CD19, EpCAM, CLEC12A (CLL1), CD20, CD22, CEA, CD33, EGFR, GPC3, CD123, CD38, CD33, CD276, CDH3 (cadherin 3), FGFR1, SSTR2, CD133, EPHA2, HLA-A2, IL13RA2, ROR1, CEACAM6, CD135, GD-2, GA733, CD135 (FLT3), CSPG4 and TAG-72. Particular examples are: CD138, CD79b, CD123, MSLN, PSMA, BCMA, CD19, CD20, CEA, CD38, CD33, CLEC12a, and ROR1.

In preferred embodiments, the HFW1, HFW2, HFW3 and HFW4 comprised in the antibody variable domains of the invention have a lysine (K), aspartate (D), glutamate (E) arginine (R), or glutamine (Q) at amino acid position 146 and one or two substitutions selected from the group consisting of (AHo numbering):

- an alanine (A), serine (S), lysine (K), arginine (R), asparagine (N) or glutamine (Q) at amino acid position 101; and
- a lysine (K) at amino acid position 148.

In other preferred embodiments, the HFW1, HFW2, HFW3 and HFW4 comprised in the antibody variable domains of the invention additionally have one or two substitutions selected from the group consisting of (AHo numbering):

- an alanine (A), lysine (K) or arginine (R) at amino acid position 12, particularly an alanine (A) or arginine (R) at amino acid position 12; and
- an alanine (A), lysine (K) or arginine (R) at amino acid position 144.

In particular embodiments, the HFW1, HFW2, HFW3 and HFW4 comprised in the antibody variable domains of the invention have one of the following substitutions (AHo numbering):

a. an alanine (A), serine (S), lysine (K), arginine (R), asparagine (N) or glutamine (Q) at amino acid position 101;
b. an alanine (A), serine (S), lysine (K), arginine (R), aspartate (D), glutamate (E), asparagine (N) or glutamine (Q) at amino acid position 146;
c. an alanine (A), serine (S), lysine (K), arginine (R), asparagine (N) or glutamine (Q) at amino acid position 101; and an alanine (A), serine (S), lysine (K), arginine (R), aspartate (D), glutamate (E), asparagine (N) or glutamine (Q) at amino acid position 146;
d. an alanine (A), lysine (K) or arginine (R) at amino acid position 144; and a leucine (L), lysine (K) or asparagine (N) at amino acid position 148;
e. an alanine (A), lysine (K) or arginine (R) at amino acid position 144;

an alanine (A), serine (S), lysine (K), arginine (R), aspartate (D), glutamate (E), asparagine (N) or glutamine (Q) at amino acid position 146; and

a leucine (L), lysine (K) or asparagine (N) at amino acid position 148;

    f. an alanine (A), serine (S), lysine (K), arginine (R), asparagine (N) or glutamine (Q) at amino acid position 101;

        an alanine (A), lysine (K) or arginine (R) at amino acid position 144; and
        an alanine (A), serine (S), lysine (K), arginine (R), aspartate (D), glutamate (E), asparagine (N) or glutamine (Q) at amino acid position 146.

    g. an alanine (A) or arginine (R) at amino acid position 12;
    h. an alanine (A), serine (S), lysine (K), arginine (R), asparagine (N) or glutamine (Q) at amino acid position 101;
    i. an alanine (A), lysine (K) or arginine (R) at amino acid position 144; or
    j. an alanine (A), serine (S), lysine (K), arginine (R), aspartate (D), glutamate (E), asparagine (N) or glutamine (Q) at amino acid position 146.

In other particular embodiments, the HFW1, HFW2, HFW3 and HFW4 comprised in the antibody variable domains of the invention have one of the following substitutions (AHo numbering):

    a. an alanine (A), lysine (K), arginine (R) or asparagine (N) at amino acid position 101;
    b. a lysine (K), arginine (R), aspartate (D), glutamate (E) or glutamine (Q) at amino acid position 146;
    c. a serine (S), lysine (K), arginine (R), asparagine (N) or glutamine (Q) at amino acid position 101; and
    a lysine (K), aspartate (D), glutamate (E) or glutamine (Q) at amino acid position 146;
    d. an alanine (A) or lysine (K) at amino acid position 144; and
    a leucine (L) or asparagine (N) at amino acid position 148;
    e. a lysine (K) at amino acid position 144;

        a glutamate (E) at amino acid position 146; and
        a lysine (K) at amino acid position 148;

    f. a serine (S), arginine (R) or glutamine (Q) at amino acid position 101;

        an alanine (A) or lysine (K) at amino acid position 144; and
        an arginine (R), or glutamine (Q) at amino acid position 146; or

    g. an alanine (A) at amino acid position 12;

        a serine (S), arginine (R) or glutamine (Q) at amino acid position 101;
        an alanine (A) or lysine (K) at amino acid position 144; and
        an arginine (R), or glutamine (Q) at amino acid position 146.

Suitably, the framework regions HFW1, HFW2, HFW3 and HFW4 comprised in the antibody variable domain of the invention are selected from a human VH framework. In particular, the framework regions HFW1, HFW2, HFW3 and HFW4 comprised in the antibody variable domain of the invention are selected from the human VH framework subtypes VH1a, VH1b, VH3, VH4, VH5 or VH6, particularly from the human VH framework subtypes VH1a, VH1b, VH3 or VH4. In particular embodiments, the framework regions HFW1, HFW2, HFW3 and HFW4 are selected from the human VH framework subtype VH3.

In the context of the present invention, the term "belonging to or selected from a VHx framework subtype (or Vκ/Vλ framework subtype)" means that the framework sequences HFW1 to HFW4 (or LF1 to LFW4) show the highest degree of homology to said human antibody VH or VL framework subtype. In the context of the present invention, the sequences of human VH domains are grouped into seven distinct human subfamilies (VH1a, VH1b, VH2, VH3, VH4, VH5 and VH6) based on sequence homology, as shown in Knappik et al., J. Mol. Biol. 296 (2000) 57-86 or in WO 2019/057787. Specific example of VH domains belonging to the VH3 framework subtype are represented by SEQ ID NOs: 157 to 170 (i. e. the non-italicized residues in Table 5). Specific examples of a VH domain belonging to the VH1a, VH1b, VH4, VH5 and VH6 framework subtypes are represented by SEQ ID NOs: 171 - 175 (i. e. the non-italicized residues in Table 5). Alternative examples of VH1a, VH1b, VH3 and VH4 sequences, and examples of other VHx sequences, may be found in Knappik et al., J. Mol. Biol. 296 (2000) 57-86 or in WO 2019/057787.

In particular embodiments, the variable heavy chain framework regions HFW1, HFW2, HFW3 and HFW4 of the antibody variable domain of the present invention are selected from the combination of framework regions (i. e. the non-italicized residues in Tables 1 and 3, i. e. all residues that are not marked as CDR residues) of any one of the SEQ ID NOs: 5 - 36 and 83 - 114, particularly of any one of the SEQ ID NOs: 8 - 36 and 86 - 114; and from the combination of framework regions (*i. e.* the non-italicized residues in Tables 1 and 3) of variants of SEQ ID NOs: 5 - 36 and 83 - 114, particularly of variants of SEQ ID NOs: 8 - 36 and 86 - 114, wherein no more than 5 amino acids, particularly no more than 4 amino acids, particularly no more than 3 amino acids, particularly no more than 2 amino acids, particularly no more than 1 amino acid within the framework regions at positions different from 12, 101, 144, 146 and 148 (AHo numbering) have been mutated. In this connection, the term "mutation" means, as various non-limiting examples, an addition, substitution or deletion. The VH regions further include VH domains comprising at least positions 5 to 140 (AHo numbering), particularly at least positions 3 to 145, more particularly at least positions 2 to 147 of one of the sequences shown in the SEQ ID NOs: 35 - 36 and 83 - 114, provided that such VH domains exhibit the functional features defined above in items 22 and 23.

In preferred embodiments, the variable light chain frameworks LFW1, LFW2, LFW3 and LFW4 of the antibody variable domain of the present invention are selected from a human antibody Vκ (e.g. a Vκ1, Vκ2, Vκ3 or Vκ4 framework subtype) framework or a human antibody Vλ framework (e.g. a Vλ1, Vλ2 or Vλ3 framework subtype), in particular a human antibody Vκ framework. In the context of the present invention, the sequences of human VL domains are grouped into four distinct human Vκ subfamilies (Vκ1, Vκ2, Vκ3, and Vκ4), and three distinct human Vλ subfamilies (Vλ1, Vλ2 and Vλ3) based on sequence homology, as shown in Knappik et al., J. Mol. Biol. 296 (2000) 57-86 or in WO 2019/057787. In particular embodiments, the variable light chain frameworks LFW1, LFW2, LFW3 and LFW4 of the antibody variable domain of the present invention are of the Vκ1 framework subtype. Specific examples of Vκ1 framework subtypes are represented by SEQ ID NOs: 176, 177, 178 and 179 (the non-italicized residues in Table 5). Alternative examples of Vκ1 sequences, and examples of Vκ2, Vκ3 or Vκ4 sequences, may be found in Knappik et al., J. Mol. Biol. 296 (2000) 57-86.

In other preferred embodiments, the variable light chain frameworks LFW1, LFW2 and LFW3 are selected from a human antibody Vκ framework, preferably a Vκ1 framework subtype, and the variable light chain framework LFW4 is selected from a Vλ framework. In particular embodiments, the variable light chain framework LFW4 of the antibody variable domain of the present invention is selected from the group consisting of the Vλ framework 4 sequences of SEQ ID NOs: 188, 189, 190, 191, 192, 193, 194, 195 and 196. Vλ framework 4 sequence of SEQ ID NO: 195 comprises a single cysteine residue at the variable light (VL) chain position 144 (AHo numbering) and is in particular applied in cases where a second single cysteine is present in the corresponding variable heavy (VH) chain, particularly in position 51 (AHo numbering) of VH, for the formation of an inter-domain disulfide bond.

In particular embodiments, the antibody variable domains of the present invention comprise a variable light chain (VL), wherein

a. the variable light chain framework regions LFW1, LFW2, LFW3 and LFW4 are selected from a human antibody Vκ framework, in particular are of the Vκ1 framework subtype; or

b. the variable light chain framework regions LFW1, LFW2 and LFW3 are selected from a human antibody Vκ framework, in particular are of the Vκ1 framework subtype, and said variable light chain framework region LFW4 is selected from a Vλ framework,

and wherein said LFW3 has the following substitution (AHo numbering):

- a glutamate (E), arginine (R) or a glutamine (Q) at amino acid position 101.

In other particular embodiments, the LFW1, LFW2 and LFW3, and optionally also LFW4, if LFW4 is selected from a human antibody Vκ framework subtype, are selected from the combination of framework regions LFW1, LFW2, LFW3 and optionally LFW4 (*i. e.* the non-italicized residues in Table 5) of any one of the SEQ ID NOs: 176, 177, 178 and 179; and the combination of framework regions LFW1, LFW2, LFW3 and optionally LFW4 (*i. e.* the non-italicized residues in Table 5) of any one of the SEQ ID NOs: 176, 177, 178 and 179, wherein no more than 5 amino acids, particularly no more than 4 amino acids, particularly no more than 3 amino acids, particularly no more than 2 amino acids, particularly no more than 1 amino acid within the framework regions at positions different from 101 (AHo numbering) have been mutated; and wherein said LFW3 optionally has a glutamate (E), arginine (R) or a glutamine (Q) at amino acid position 101.

In yet other particular embodiments, the variable light chain frameworks LFW1, LFW2, LFW3 and LFW4 of the antibody variable domain of the present invention are selected from the combination of framework regions LFW1, LFW2, LFW3 and LFW4 (*i. e.* the non-italicized residues in Tables 1, 3 and 5) of any one of the SEQ ID NOs: 37, 38, 39, 115, 116, 117, 180, 181, 182, 183, 184, 185, 186 and 187; and the combination of framework regions LFW1, LFW2, LFW3 and LFW4 (*i. e.* the non-italicized residues in Tables 1, 3 and 5) of any one of the SEQ ID NOs: 37, 38, 39, 115, 116, 117, 180, 181, 182, 183, 184, 185, 186 and 187, wherein no more than 5 amino acids, particularly no more than 4 amino acids, particularly no more than 3 amino acids, particularly no more than 2 amino acids, particularly no more than 1 amino acid within the framework regions at positions different from 101 (AHo numbering) have been mutated. In this connection, the term "mutation" means, as various non-limiting examples, an addition, substitution or deletion. The VL regions further include VL domains comprising at least positions 5 to 140 (AHo numbering), particularly at least positions 3 to 145, more particularly at least positions 2 to 147 of one of the sequences shown in the SEQ ID NOs: 37, 38, 39, 115, 116, 117, 180, 181, 182, 183, 184, 185, 186 and 187, provided that such VL domains exhibit the functional features defined above in items 22 and 23.

In preferred embodiments, the antibody variable domain of the present invention is in a format selected from an Fv fragment consisting of the VL and VH domains of a single arm of an antibody; a disulfide stabilized Fv fragment (dsFv); and a single chain Fv fragment (scFv). In particular embodiments, the antibody variable domain of the present invention is selected from an Fv fragment and a single-chain Fv fragment (scFv). In other particular embodiments, the VL and VH domains of the scFv fragment are stabilized by an interdomain disulfide bond, in particular said VH domain comprises a single cysteine residue in position 51 (AHo numbering) and said VL domain comprises a single cysteine residue in position 141 (AHo numbering).

The antibody variable domain in accordance with the present invention, when being in scFv format, exhibits a reduced immunogenicity, when compared to a version of said antibody variable domain that does not comprise the above defined substitutions in the VH framework regions. More specifically, the antibody variable domain of the present invention, when being in scFv format, exhibits a reduced binding to pre-existing anti-drug antibodies (ADA) present in human sera, in particular reduced binding to pre-existing ADAs when compared to a version of said antibody variable domain that does not comprise the above defined substitutions in the VH framework regions, as determined in a pre-existing ADA-binding assay, in particular as determined in a pre-existing ADA-binding assay as described in Example 2.

Immunogenicity, i.e. the tendency of a therapeutic protein to induce an antibody response within the patient's body, can e.g. be predicted by its capacity to be recognized by anti-drug antibodies (ADAs) that are already present in human sera of healthy and untreated individuals, herein referred to as "pre-existing ADAs".

Thus, for the purpose of the present invention, the term "immunogenicity", as used herein, refers to the capacity of a therapeutic protein, e.g. an antibody, an antibody fragment or an antibody binding domain, to be recognized by pre-existing ADAs in human serum samples. Without being bound to theory, it is believed that pre-existing ADA-binding as well as the induction of the formation of ADAs during therapeutic treatment is linked with the occurrence of B cell and/or T cell epitopes on a therapeutic protein. The extent of such immunogenicity can be determined by an ELISA assay and can be expressed by the percentage or the number of human serum samples, which contain measurable amounts of pre-existing ADAs and/or ADAs formed during therapeutic treatment, that recognize, i. e. bind to, the therapeutic protein in question, relative to the total number of tested human sera (percentage or number of positive serum samples). A reduction of immunogenicity between a therapeutic protein and a corresponding therapeutic protein being modified with the goal to reduce its immunogenicity can be measured by comparing the percentage of positive serum samples against the modified therapeutic protein, with the percentage of positive serum samples against the original therapeutic protein. A lower number or percentage of positive serum samples for the modified therapeutic protein indicates a reduction of immunogenicity relative to the original therapeutic protein.

A serum sample is deemed to contain measurable amounts of pre-existing ADAs, when the ELISA signal surpasses a certain threshold. This threshold is herein also referred to as the screening cut-point (SCP). The SCP can be calculated as defined below or set to an arbitrary value relative to the maximum ELISA signal obtained for the tested sera (e.g. 20 %, 15 %, 10 % or 5 % of the maximum ELISA signal obtained for the tested sera). Preferably, the SCP is calculated as defined below.

The antibody variable domains of the present invention, when being in scFv format, further have advantageous biophysical properties, in particular an excellent stability. Suitably, the antibody variable domain of the present invention, when being in scFv format, is further characterized by one or more of the following features:

    a. has a melting temperature (Tm), determined by differential scanning fluorimetry (DSF), of at least 63°C, particularly of at least 64°C, particularly of at least 65°C, when formulated in 20 mM Histidine, pH 6.0;
    b. has a loss in monomer content, after storage for 28 days at 4°C, of less than 5 %, particularly of less than 3 %, particularly of less than 2 %, when formulated at a concentration of 10 mg/ml in 20 mM Histidine at pH 6.0;
    c. has a loss in monomer content, after storage for 28 days at 40°C, of less than 15 %, particularly of less than 10 %, particularly of less than 8 %, when formulated at a concentration of 10 mg/ml in 20 mM Histidine at pH 6.0;
    d. has a loss in protein content, after storage for 28 days at 4°C, of less than 15 %, particularly of less than 10 %, when formulated at a concentration of 10 mg/ml in 20 mM Histidine, pH 6.0;
    e. has a loss in protein content, after storage for 28 days at 40°C, of less than 10 %, particularly of less than 5 %, when formulated at a concentration of 10 mg/ml in 20 mM Histidine, pH 6.0
    f. binds its target antigen with a dissociation constant (KD) that is lower or not greater than 3-folds, when compared with the KD of a version of said antibody variable domain that does not comprise the substitutions defined in item 1, as measured by surface plasmon resonance (SPR).

DSF is described earlier (Egan, et al., MAbs, 9(1) (2017), 68-84; Niesen, et al., Nature Protocols, 2(9) (2007) 2212-2221). The midpoint of transition for the thermal unfolding of the scFv constructs is determined by nano Differential Scanning Fluorimetry as described in detail in Example 4. In brief, $1 \pm 0.1$ mg/ml and $10 \pm 1$ mg/ml solution in 20 mM Histidine buffer, pH 6, are prepared and subjected to a temperature ramp of from 20 °C to 95 °C with a 1 °C/min increase. The unfolding event is monitored by using the intrinsic fluorescence of proteins, i.e. by the change of the fluorescence emission spectra of Tryptophan (Trp). The midpoint of unfolding (Tm) is defined at the inflection point (thermal midpoint) of the unfolding curve observed as a local maximum or minimum of the first derivative.

The loss in monomer content is as determined by area under the curve calculation of SE-HPLC chromatograms. SE-HPLC is a separation technique based on a solid stationary phase and a liquid mobile phase as outlined by the US Pharmacopeia (USP), chapter 621. This method separates molecules based on their size and shape utilizing a hydrophobic stationary phase and aqueous mobile phase. The separation of molecules is occurring between the void volume ($V_0$) and the total permeation volume ($V_T$) of a specific column. The monomeric content of the scFv constructs is determined using SE-HPLC with a Shodex 402.5-4F KW column. 5 $\mu$g of scFv molecules at the given sample concentration, e. g. about 1 mg/ml or about 10 mg/ml, are injected for the analysis. The monomeric content is then determined by integrating the monomeric peak of the scFv. A standard running buffer consisting of 50 mM Sodium acetate and 250 mM sodium chloride at pH 6.0 is used for the analysis.

It was further found by the inventors that the antibody variable domains of the present invention maintain their binding affinity to their respective target antigens, when compared to their unsubstituted versions. In this regard, the expression "maintain their binding affinity" means that the monovalent dissociation constant (KD) for binding of the antibody variable domains of the invention to their target antigens, as measured by surface plasmon resonance (SPR), is either lower or less than 3 times greater than the KD of their respective unsubstituted versions (i.e. versions of said antibody variable domains that do not comprise the substitutions defined herein.) The determination of the individual KD values by SPR is described in detail in Example 3.

As used herein, the term "affinity" refers to the strength of interaction between the antibody or the antibody variable domain and the antigen at single antigenic sites. Within each antigenic site, the variable region of the antibody variable domain or the antibody "arm" interacts through weak non-covalent forces with antigen at numerous sites; the more interactions, the stronger the affinity.

"Binding affinity" generally refers to the strength of the total sum of non-covalent interactions between a single binding site of a molecule (e. g., of an antibody or an antibody variable domain) and its binding partner (e. g., an antigen or, more specifically, an epitope on an antigen). Unless indicated otherwise, as used herein, "binding affinity", "bind to", "binds to" or "binding to" refers to intrinsic binding affinity that reflects a 1:1 interaction between members of a binding pair (e. g., an antibody variable domain and an antigen). The affinity of a molecule X for its partner Y can generally be represented by the dissociation constant ($K_D$). Affinity can be measured by common methods known in the art, including those described herein. Low-affinity antibodies and antibody variable domains generally bind antigens slowly and tend to dissociate readily, whereas high-affinity antibodies generally bind antigens faster and tend to remain bound longer. A variety of methods of measuring binding affinity are known in the art, any of which can be used for purposes of the present invention. Specific illustrative and exemplary embodiments for measuring binding affinity, i. e. binding strength are described in the following.

The term "$K_{assoc}$", "$K_a$" or "$K_{on}$", as used herein, are intended to refer to the association rate of a particular antibody-antigen interaction, whereas the term "$K_{dis}$", "$K_d$" or "$K_{off}$", as used herein, is intended to refer to the dissociation rate of a particular antibody-antigen interaction. In one embodiment, the term "$K_D$", as used herein, is intended to refer to the dissociation constant, which is obtained from the ratio of $K_d$ to $K_a$ (i. e. $K_d/K_a$) and is expressed as a molar concentration (M). The "$K_D$" or "$K_D$ value" or "KD" or "KD value" according to this invention is in one embodiment measured by using surface plasmon resonance assays.

In a second aspect, the present invention relates to an antibody comprising one or more antibody variable domains of the present invention.

In certain embodiments, the antibody of the present invention further comprises antibody variable domains that differ from the antibody variable domains of the present invention. More specifically, the antibody of the present invention further comprises antibody variable domains that do not have the substitutions in the framework regions as defined herein.

In preferred embodiments, the antibody of the present invention exclusively comprises antibody variable domains of the present invention. More specifically, in these preferred embodiments, the antibody of the present invention exclusively comprises antibody variable domains that have the substitutions in the framework regions as defined herein.

The term "monovalent antibody" or "antibody that is monovalent for its target antigen", as used herein, refers to an antibody that binds to a single target molecule, and more specifically to a single epitope on a target molecule. Also, the term "binding domain" or "monovalent binding domain", as used herein, refers to a binding domain that binds to a single epitope on a target molecule.

Furthermore, the term "binding domain" of an antibody, as used herein, or the terms "antigen-binding fragment thereof' or "antigen-binding portion" of an antibody, and the like, refer to one or more parts of an intact antibody that have the ability to bind to a given antigen, in particular to specifically bind to a given antigen. Antigen-binding functions of an antibody can be performed by fragments of an intact antibody. Specifically, in case of the antibodies of the present invention, the terms "binding domain", as used herein, or the terms "antigen-binding fragment thereof" or "antigen-binding portion", and the like, refer to a Fab fragment, *i. e.* a monovalent fragment consisting of the VL, VH, CL and CH1 domains; an Fv fragment consisting of the VL and VH domains of a single arm of an antibody; a disulfide stabilized Fv fragment (dsFv); and a single chain Fv fragment (scFv). Preferably, the binding domains of the antibodies of the present invention are independently of each other selected from an Fv fragment, a disulfide stabilized Fv fragment (dsFv) and a single-chain Fv fragment (scFv). In particular embodiments, the binding domains of the antibodies of the present invention are independently of each other selected from an Fv fragment and a single-chain Fv fragment (scFv). In other particular embodiments, the VL and VH domains of the scFv fragment are stabilized by an interdomain disulfide bond, in particular said VH domain comprises a single cysteine residue in position 51 (AHo numbering) and said VL domain comprises a single cysteine residue in position 141 (AHo numbering).

The term "bivalent antibody" or "antibody that is bivalent for its target antigen", as used herein, refers to a single antibody with two valencies, where "valency" is described as the number of antigen-binding moieties that binds to epitopes on a specific target molecule. As such, the single antibody can bind to two binding sites on a target molecule and/or to two target molecules due to the presence of two copies of the corresponding antigen-binding moieties.

Likewise, the term "trivalent antibody" or "antibody that is trivalent for its target antigen", as used herein, refers to a single antibody with three valencies. As such, the single antibody can bind to three binding sites on a target molecule and/or can bind up to three target molecules due to the presence of three copies of the corresponding antigen-binding moieties.

In case the antibodies of the invention comprise two or three binding domains, said two or three binding domains either bind the same epitope or different epitopes on the target molecules. Preferably, the two or three binding domains bind the same epitope on the target molecule.

The term "same epitope", as used herein, refers to an individual protein determinant on the protein capable of specific binding to more than one antibody, where that individual protein determinant is identical, *i. e.* consist of identical chemically active surface groupings of molecules such as amino acids or sugar side chains having identical three-dimensional structural characteristics, as well as identical charge characteristics for each of said antibodies.

The term "different epitope", as used herein in connection with a specific protein target, refers to individual protein determinants on the protein, each capable of specific binding to a different antibody, where these individual protein determinants are not identical for the different antibodies, *i. e.* consist of non-identical chemically active surface groupings of molecules such as amino acids or sugar side chains having different three-dimensional structural characteristics, as well as different charge characteristics. These different epitopes can be overlapping or non-overlapping.

In one group of embodiments, the format of the antibody is selected from bivalent bispecific IgG formats, trivalent bispecific IgG formats and tetravalent bispecific IgG formats, wherein said formats comprise one or more Fv, scFv or disulfide stabilized scFv. In particular, the format of said antibody is selected from KiH-based IgGs, such as DuoBodies (bispecific IgGs prepared by the Duobody technology) (MAbs. 2017 Feb/Mar;9(2):182-212. doi: 10.1080/19420862.2016.1268307); DVD-Ig; IgG-scFv fusions, such as CODV-IgG, Morrison (IgG CH$_3$-scFv fusion (Morrison-H) or IgG CL-scFv fusion (Morrison-L)), bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain) and Ts2Ab (dsscFv linked to C-terminus of heavy chain). More particularly, the format of said antibody is selected from KiH-based IgGs, such as DuoBodies; DVD-Ig; CODV-IgG and Morrison (IgG CH$_3$-scFv fusion (Morrison-H) or IgG CL-scFv fusion (Morrison-L)), even more particularly from DVD-Ig and Morrison (IgG CH$_3$-scFv fusion (Morrison-H) or IgG CL-scFv fusion (Morrison-L)).

In this group of embodiments, the IgG is preferably selected from the IgG subclasses IgG1 and IgG4, in particular IgG4.

In particular embodiments, the format of said antibody is selected from a Morrison format, *i. e.* a Morrison-L and a Morrison-H format. The Morrison-L and Morrison-H format used in the present invention are tetravalent and bispecific molecular formats bearing an IgG Fc region, in particular an IgG4 Fc region. Two highly stable scFv binding domains, wherein the light chain comprises Vκ FR1 to FR3 in combination with a Vλ FR4, and which are based on the antibody variable domains of the present invention, are fused via a linker L1 to the heavy chain (Morrison-H) or light chain (Morrison-L) C-termini.

The linker L1 is a peptide of 2-30 amino acids, more particularly 5-25 amino acids, and most particularly 10-20 amino acids. In particular embodiments, said linker L1 comprises one or more units of four (4) glycine amino acid residues and one (1) serine amino acid residue (GGGGS)$_n$, wherein n=1, 2, 3, 4 or 5 (SEQ ID NO: 197), particularly n=2 (SEQ ID NO: 198).

The VH regions and the VL regions of the two scFv domains are connected by a linker L2. The linker L2 is a peptide of 10-40 amino acids, more particularly 15-30 amino acids, and most particularly 20-25 amino acids. Particularly, said linker L2 comprises one or more units of four (4) glycine amino acid residues and one (1) serine amino acid residue

(GGGGS)$_n$, wherein n=1, 2, 3, 4, 5, 6, 7 or 8 (SEQ ID NO: 199), particularly n=4 (SEQ ID NO: 200).

In another group of embodiments, the antibody of the invention does not comprise an immunoglobulin Fc region.

The term "immunoglobulin Fc region" or "Fc region", as used herein, is used to define a C-terminal region of an immunoglobulin heavy chain, i. e. the CH2 and CH3 domains of the heavy chain constant regions. The term "Fc region" includes native-sequence Fc regions and variant Fc regions, i. e. Fc regions that are engineered to exhibit certain desired properties, such as for example altered Fc receptor binding function and/or reduced or suppressed Fab arm exchange. An example of such an engineered Fc region is the knob-into-hole (KiH) technology (see for example Ridgway et al., Protein Eng. 9:617-21 (1996) and Spiess et al., J Biol Chem. 288(37):26583-93 (2013)). Native-sequence Fc regions include human IgG1, IgG2 (IgG2A, IgG2B), IgG3 and IgG4. "Fc receptor" or "FcR" describes a receptor that binds to the Fc region of an antibody. Particularly, the FcR is a native sequence human FcR, which binds an IgG antibody (a gamma receptor) and includes receptors of the FcγRI, FcγRII, and FcγRIII subclasses, including allelic variants and alternatively spliced forms of these receptors, FcγRII receptors including FcγRIIA (an "activating receptor") and FcγRI IB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain, (see M. Daeron, Annu. Rev. Immunol. 5:203-234 (1997). FcRs are reviewed in Ravetch and Kinet, Annu. Rev. Immunol. 9: 457-92 (1991); Capet et al, Immunomethods 4: 25-34 (1994); and de Haas et al, J. Lab. Clin. Med. 126: 330-41 (1995). Other FcRs, including those to be identified in the future, are encompassed by the term "FcR" herein. The term "Fc receptor" or "FcR" also includes the neonatal receptor, FcRn, which is responsible for the transfer of maternal IgGs to the fetus. Guyer et al., J. Immunol. 117: 587 (1976) and Kim et al., J. Immunol. 24: 249 (1994). Methods of measuring binding to FcRn are known (see, e. g., Ghetie and Ward, Immunol. Today 18: (12): 592-8 (1997); Ghetie et al., Nature Biotechnology 15 (7): 637-40 (1997); Hinton et al., J. Biol. Chem. TJI (8): 6213-6 (2004); WO 2004/92219 (Hinton et al). Binding to FcRn in vivo and serum half-life of human FcRn high-affinity binding polypeptides can be assayed, e. g., in transgenic mice or transfected human cell lines expressing human FcRn, or in primates to which the polypeptides having a variant Fc region are administered. WO 2004/42072 (Presta) describes antibody variants which improved or diminished binding to FcRs. See also, e. g., Shields et al., J. Biol. Chem. 9(2): 6591-6604 (2001).

In this group of embodiments, the antibody is preferably in a format selected from the group consisting of: a tandem scDb (Tandab), a linear dimeric scDb (LD-scDb), a circular dimeric scDb (CD-scDb), a tandem tri-scFv, a tribody (Fab-(scFv)$_2$), a Fab-Fv$_2$, a triabody, an scDb-scFv, a tetrabody, a di-diabody, a tandem-di-scFv and a MATCH (described in WO 2016/202457; Egan T., et al., MABS 9 (2017) 68-84).

In particular embodiments, the antibody of the invention does further not comprise CH1 and/or CL regions. In these particular embodiments, the antibody is in a scDb-scFv, a triabody, a tetrabody or a MATCH format, particularly in a MATCH or scDb-scFv format. More particularly, the antibody of the invention is in a MATCH3 or a MATCH4 format.

In specific embodiments, the antibody of the invention is trispecific and tetravalent.

In further specific embodiments, the antibody of the invention is trispecific and trivalent.

The antibody variable domains comprised in the bispecific, trispecific tetraspecific or pentaspecific, antibodies of the invention are capable of binding to their respective antigens or receptors simultaneously. The term "simultaneously", as used in this connection refers to the simultaneous binding of one of the antibody variable domains, which for example specifically binds to MSLN, and of one or two further antibody variable domains, which for example have specificity for CD3 and hSA.

Suitably, the antibody variable domains comprised in the bispecific, trispecific tetraspecific or pentaspecific, antibodies of the invention are operably linked.

The term "operably linked", as used herein, indicates that two molecules (e. g., polypeptides, domains, binding domains) are attached in a way that each molecule retains functional activity. Two molecules can be "operably linked" whether they are attached directly or indirectly (e. g., via a linker, via a moiety, via a linker to a moiety). The term "linker" refers to a peptide or other moiety that is optionally located between binding domains or antibody variable domains used in the invention. A number of strategies may be used to covalently link molecules together. These include, but are not limited to, polypeptide linkages between N- and C-termini of proteins or protein domains, linkage via disulfide bonds, and linkage via chemical cross-linking reagents. In one aspect of this embodiment, the linker is a peptide bond, generated by recombinant techniques or peptide synthesis. Choosing a suitable linker for a specific case where two polypeptide chains are to be connected depends on various parameters, including but not limited to the nature of the two polypeptide chains (e. g., whether they naturally oligomerize), the distance between the N- and the C-termini to be connected if known, and/or the stability of the linker towards proteolysis and oxidation. Furthermore, the linker may contain amino acid residues that provide flexibility.

In the context of the present invention, the term "polypeptide linker" refers to a linker consisting of a chain of amino acid residues linked by peptide bonds that is connecting two domains, each being attached to one end of the linker. The polypeptide linker should have a length that is adequate to link two molecules in such a way that they assume the correct conformation relative to one another so that they retain the desired activity. In particular embodiments, the polypeptide linker has a continuous chain of between 2 and 30 amino acid residues (e. g., 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acid residues). In addition, the amino acid residues selected for inclusion in the polypeptide linker should exhibit properties that do not interfere significantly with the activity of the polypeptide. Thus, the linker peptide on the whole should not exhibit a charge that would be inconsistent with the activity of the polypeptide, or interfere with internal folding, or form bonds or other interactions with amino acid residues in one or more of the monomers that would seriously impede the binding of receptor monomer domains. In particular embodiments, the polypeptide linker is non-structured polypeptide. Useful linkers include glycine-serine, or GS linkers. By "Gly-Ser" or "GS" linkers is meant a polymer of glycines and serines in series (including, for example, $(Gly\text{-}Ser)_n$, $(GSGGS)_n$ (SEQ ID NO: 201), $(GGGGS)_n$ (SEQ ID NO: 202) and $(GGGS)_n$ (SEQ ID NO: 203), where n is an integer of at least one), glycine-alanine polymers, alanine-serine polymers, and other flexible linkers such as the tether for the shaker potassium channel, and a large variety of other flexible linkers, as will be appreciated by those in the art. Glycine-serine polymers are preferred since oligopeptides comprising these amino acids are relatively unstructured, and therefore may be able to serve as a neutral tether between components. Secondly, serine is hydrophilic and therefore able to solubilize what could be a globular glycine chain. Third, similar chains have been shown to be effective in joining subunits of recombinant proteins such as single-chain antibodies.

Suitably, the antibody variable domain of the invention is an isolated variable domain. Likewise, the antibodies of the invention are isolated antibodies. The term "isolated variable domain" or "isolated antibody", as used herein, refers to an variable domain or an antibody that is substantially free of other variable domains or other antibodies having different antigenic specificities (e. g., an isolated antibody variable domain that specifically binds mesothelin is substantially free of antibody variable domains that specifically bind antigens other than mesothelin). Moreover, an isolated antibody variable domain or isolated antibody may be substantially free of other cellular material and/or chemicals.

Suitably, the antibody variable domains and antibodies of the invention are monoclonal antibody variable domains and antibodies. The term "monoclonal antibody variable domains" or "monoclonal antibody" as used herein refers to variable domains or antibodies that have substantially identical amino acid sequences or are derived from the same genetic source. A monoclonal variable domain or antibody displays a binding specificity and affinity for a particular epitope, or binding specificities and affinities for specific epitopes.

The antibody variable domains and antibodies of the invention include, but are not limited to, chimeric, human and humanized antibody variable domains and antibodies.

The term "chimeric antibody" or "chimeric antibody variable domain", as used herein, refers to an antibody molecule or antibody variable domain in which (a) the constant region, or a portion thereof, is altered, replaced or exchanged so that the antigen-binding site (variable region) is linked to a constant region of a different or altered class, effector function and/or species; or (b) the variable region, or a portion thereof, is altered, replaced or exchanged with a variable region having a different or altered antigen specificity. For example, a mouse antibody can be modified by replacing its constant region with the constant region from a human immunoglobulin. Due to the replacement with a human constant region, the chimeric antibody can retain its specificity in recognizing the antigen while having reduced antigenicity in human as compared to the original mouse antibody.

The term "human antibody" or "human antibody variable domain" , as used herein, is intended to include antibodies or antibody variable domains having variable regions in which both the framework and CDR regions are derived from sequences of human origin. Furthermore, if the antibody or antibody variable domain contains a constant region, the constant region also is derived from such human sequences, e. g., human germline sequences, or mutated versions of human germline sequences. The human antibodies and antibody variable domains of the invention may include amino acid residues not encoded by human sequences (e. g., mutations introduced by random or site-specific mutagenesis in vitro or by somatic mutation in vivo). This definition of a human antibody or antibody variable domain specifically excludes a humanized antibody or antibody variable domain comprising non-human antigen-binding residues. Human antibodies and antibody variable domains can be produced using various techniques known in the art, including phage-display libraries (Hoogenboom and Winter, J. Mol. Biol, 227:381 (1992); Marks et al, J. Mol. Biol, 222:581 (1991)). Also available for the preparation of human monoclonal antibodies and human monoclonal antibody variable domains are methods described in Cole et al, Monoclonal Antibodies and Cancer Therapy, Alan R. Liss, p. 77 (1985); Boemer et al, J. Immunol, 147(I):86-95 (1991). See also van Dijk and van de Winkel, Curr. Opin. Pharmacol, 5: 368-74 (2001). Human antibodies and human antibody variable domains can be prepared by administering the antigen to a transgenic animal that has been modified to produce such antibodies and antibody variable domains in response to antigenic challenge, but whose endogenous loci have been disabled, e. g., immunized xenomice (see, e. g., U.S. Pat. Nos. 6,075,181 and 6,150,584 regarding XENOMOUSE™ technology). See also, for example, Li et al, Proc. Natl. Acad. Sci. USA, 103:3557- 3562 (2006) regarding human antibodies generated via a human B-cell hybridoma technology.

The term "humanized" antibody or "humanized" antibody variable domain, as used herein, refers to an antibody or antibody variable domain that retains the reactivity of a non-human antibody or antibody variable domain while being less immunogenic in humans. This can be achieved, for instance, by retaining the non-human CDR regions and replacing the remaining parts of the antibody or antibody variable domain with their human counterparts (i. e., the constant region as well as the framework portions of the variable region). Additional framework region modifications may be made within the human framework sequences as well as within the CDR sequences derived from the germline of another mammalian species. The humanized antibodies and antibody variable domains of the invention may include amino acid residues not encoded by human sequences (e. g., mutations introduced by random or site-specific mutagenesis *in vitro* or by somatic mutation *in vivo,* or a conservative substitution to promote stability or manufacturing). See, e. g., Morrison et al., Proc. Natl. Acad. Sci. USA, 81:6851-6855, 1984; Morrison and Oi, Adv. Immunol., 44:65-92, 1988; Verhoeyen et al., Science, 239: 1534-1536, 1988; Padlan, Molec. Immun., 28:489-498, 1991; and Padlan, Molec. Immun., 31: 169-217, 1994. Other examples of human engineering technology include but are not limited to the Xoma technology disclosed in US 5,766,886.

The term "recombinant humanized antibody" or "recombinant humanized antibody variable domain" as used herein, includes all human antibodies and human antibody variable domains that are prepared, expressed, created or isolated by recombinant means, such as antibodies and antibody variable domains isolated from a host cell transformed to express the humanized antibody or humanized antibody variable domain, e. g., from a transfectoma, and antibodies and antibody variable domains prepared, expressed, created or isolated by any other means that involve splicing of all or a portion of a human immunoglobulin gene, sequences to other DNA sequences.

Preferably, the antibody variable domains and antibodies of the invention are humanized. More preferably, the antibody variable domains and antibodies of the invention are humanized and comprise rabbit-derived CDRs.

The term "bispecific antibody", "trispecific antibody", "tetraspecific antibody", "pentaspecific antibody" or the more general term "multispecific antibody" as used herein, refers to an antibody that binds to two or more different epitopes on at least two or more different targets, for example 2 different targets (bispecific), 3 different targets (trispecific), 4 different targets (tetraspecific), or 5 different targets (pentaspecific). Preferably, the antibodies of the invention are bispecific, trispecific or tetraspecific, particularly bispecific or trispecific, more particularly trispecific. As indicated above, the term trispecific antibody refers to an antibody that binds to at least three different epitopes on three different targets (e. g., mesothelin, CD3 and hSA or PD-L1, CD137 and hSA).

The term "epitope" means a protein determinant capable of specific binding to an antibody. Epitopes usually consist of chemically active surface groupings of molecules such as amino acids or sugar side chains and usually have specific three-dimensional structural characteristics, as well as specific charge characteristics. "Conformational" and "linear" epitopes are distinguished in that the binding to the former but not the latter is lost in the presence of denaturing solvents.

The term "conformational epitope" as used herein refers to amino acid residues of an antigen that come together on the surface when the polypeptide chain folds to form the native protein.

The term "linear epitope" refers to an epitope, wherein all points of interaction between the protein and the interacting molecule (such as an antibody) occurring linearly along the primary amino acid sequence of the protein (continuous).

The term "recognize" as used herein refers to an antibody or antibody variable domain that finds and interacts (e. g., binds) with its conformational epitope.

The inventors found that the antibody variable domain of the invention can be successfully applied in the construction of divers antibody fragments, e. g. scFv fragments, and multispecific antibodies, e. g. bispecific and trispecific antibodies, which exhibits significantly reduced immunogenicity, when compared to their unmodified counterparts, and which have an excellent stability.

The antibody variable domains and antibodies of the invention can be produced using any convenient antibody-manufacturing method known in the art (see, e. g., Fischer, N. & Leger, O., Pathobiology 74 (2007) 3-14 with regard to the production of bispecific constructs; Hornig, N. & Färber-Schwarz, A., Methods Mol. Biol. 907 (2012)713-727, and WO 99/57150 with regard to bispecific diabodies and tandem scFvs). Specific examples of suitable methods for the preparation of the multispecific constructs further include, *inter alia,* the Genmab (see Labrijn et al., Proc. Natl. Acad. Sci. USA 110 (2013) 5145-5150) and Merus (see de Kruif et al., Biotechnol. Bioeng. 106 (2010) 741-750) technologies.

These methods typically involve the generation of monoclonal antibodies or monoclonal antibody variable domains, for example by means of fusing myeloma cells with the spleen cells from a mouse that has been immunized with the desired antigen using the hybridoma technology (see, e. g., Yokoyama et al., Curr. Protoc. Immunol. Chapter 2, Unit 2.5, 2006) or by means of recombinant antibody engineering (repertoire cloning or phage display/yeast display) (see, e. g., Chames & Baty, FEMS Microbiol. Letters 189 (2000) 1-8), and the combination of the antigen-binding domains or fragments or parts thereof of two or more different monoclonal antibodies to give a bispecific or multispecific construct using known molecular cloning techniques.

The antibodies of the invention that are multispecific, e.g. bispecific, trispecific, tetraspecific or pentaspecific, and/or multivalent, can be prepared by conjugating the constituent binding specificities, using methods known in the art. For example, each binding specificity of these antibodies can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-5-acetyl-thioacetate (SATA), 5,5'-dithiobis (2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl)-cyclohexane-1-carboxylate (sulfo-SMCC) (see e. g., Karpovsky et al., 1984 J. Exp. Med. 160: 1686; Liu, M A et al., 1985 Proc. Natl. Acad. Sci. USA 82:8648). Other methods include those described in Paulus, 1985 Behring Ins. Mitt. No. 78, 118-132; Brennan et al., 1985 Science 229:81-83, and Glennie et al., 1987 J. Immunol. 139: 2367-2375. Conjugating agents are SATA and sulfo-SMCC, both available from Pierce Chemical Co. (Rockford, III).

Alternatively, two or more binding specificities can be encoded in the same vector and expressed and assembled in the same host cell. This method is particularly useful where the bispecific molecule is a mAb x Fab, a mAb x scFv, a mAb x dsFv or a mAb x Fv fusion protein. Methods for preparing multispecific and/or multivalent antibodies and molecules are described for example in US 5,260,203; US 5,455,030; US 4,881,175; US 5,132,405; US 5,091,513; US 5,476,786; US 5,013,653; US 5,258,498; and US 5,482,858.

Binding of the antibody variable domains and multispecific antibodies to their specific targets can be confirmed by, for example, enzyme-linked immunosorbent assay (ELISA), radioimmunoassay (REA), FACS analysis, bioassay (e. g., growth inhibition), or Western Blot assay. Each of these assays generally detects the presence of protein-antibody complexes of particular interest by employing a labeled reagent (e. g., an antibody) specific for the complex of interest.

In a further aspect, the invention provides a nucleic acid or two nucleic acids encoding the antibody variable domain or the antibody of the invention. Such nucleic acids can be optimized for expression in mammalian cells.

The term "nucleic acid" is used herein interchangeably with the term "polynucleotide(s)" and refers to one or more deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphorates, 2-O-methyl ribonucleotides, peptide-nucleic acids (PNAs). Unless otherwise indicated, a particular nucleic acid sequence also implicitly encompasses conservatively modified variants thereof (e. g., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., Nucleic Acid Res. 19:5081, 1991; Ohtsuka et al., J. Biol. Chem. 260:2605-2608, 1985; and Rossolini et al., Mol. Cell. Probes 8:91-98, 1994).

The invention provides substantially purified nucleic acid molecules which encode polypeptides comprising segments or domains of the antibody variable domains or the antibodies described above. When expressed from appropriate expression vectors, polypeptides encoded by these nucleic acid molecules are capable of exhibiting antigen-binding capacities of the antibody variable domains or the antibodies of the present invention.

The polynucleotide sequences can be produced by *de novo* solid-phase DNA synthesis or by PCR mutagenesis of an existing sequence (e. g., sequences as described in the Examples below) encoding the antibody variable domain or the antibody of the invention. Direct chemical synthesis of nucleic acids can be accomplished by methods known in the art, such as the phosphotriester method of Narang et al., 1979, Meth. Enzymol. 68:90; the phosphodiester method of Brown et al., Meth. Enzymol. 68: 109, 1979; the diethylphosphoramidite method of Beaucage et al., Tetrahedron Lett., 22: 1859, 1981; and the solid support method of US 4,458,066. Introducing mutations to a polynucleotide sequence by PCR can be performed as described in, e. g., PCR Technology: Principles and Applications for DNA Amplification, H. A. Erlich (Ed.), Freeman Press, NY, N.Y., 1992; PCR Protocols: A Guide to Methods and Applications, Innis et al. (Ed.), Academic Press, San Diego, Calif, 1990; Mattila et al., Nucleic Acids Res. 19:967, 1991; and Eckert et al., PCR Methods and Applications 1:17, 1991.

Also provided in the invention are expression vectors and host cells for producing the antibody variable domain or the antibody of the invention.

The term "vector" is intended to refer to a polynucleotide molecule capable of transporting another polynucleotide to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e. g., bacterial vectors having a bacterial origin of replication and episomal mammalian vectors). Other vectors (e. g., non-episomal mammalian vectors) can be integrated into the genome of a host cell upon introduction into the host cell, and thereby are replicated along with the host genome.

Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (e. g., replication defective retroviruses, adenoviruses and adeno- associated viruses), which serve equivalent functions. In this particular context, the term "operably linked" refers to a functional relationship between two or more polynucleotide (e. g., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, *i.* e., they are cis-acting. However, some transcriptional regulatory sequences, such as enhancers, need not be physically contiguous or located in close proximity to the coding sequences whose transcription they enhance.

Various expression vectors can be employed to express the polynucleotides encoding the antibody variable domain or the antibody chain(s). Both viral-based and non-viral expression vectors can be used to produce the antibodies or antibody variable domains in a mammalian host cell. Non-viral vectors and systems include plasmids, episomal vectors, typically with an expression cassette for expressing a protein or RNA, and human artificial chromosomes (see, e. g., Harrington et al., Nat Genet. 15:345, 1997). For example, non-viral vectors useful for expression of the PD-L1- or MSLN-binding polypeptides, or of polynucleotides encoding such polypeptides, in mammalian (e. g., human) cells include pThioHis A, B and C, pcDNA3.1/His, pEBVHis A, B and C, (Invitrogen, San Diego, Calif.), MPS V vectors, and numerous other vectors known in the art for expressing other proteins. Useful viral vectors include vectors based on retroviruses, adenoviruses, adeno-associated viruses, herpes viruses, vectors based on SV40, papilloma virus, HBP Epstein Barr virus, Vaccinia virus vectors and Semliki Forest virus (SFV). See, Brent et al., supra; Smith, Annu. Rev. Microbiol. 49:807, 1995; and Rosenfeld et al., Cell 68: 143, 1992.

The choice of expression vector depends on the intended host cells in which the vector is to be expressed. Typically, the expression vectors contain a promoter and other regulatory sequences (e. g., enhancers) that are operably linked to the polynucleotides encoding a multispecific antibody chain or a variable domain. In one embodiment, an inducible promoter is employed to prevent expression of inserted sequences except under inducing conditions. Inducible promoters include, e. g., arabinose, lacZ, metallothionein promoter or a heat shock promoter. Cultures of transformed organisms can be expanded under non-inducing conditions without biasing the population for coding sequences whose expression products are better tolerated by the host cells. In addition to promoters, other regulatory elements may also be required or desired for efficient expression of a multispecific antibody chain or a variable domain. These elements typically include an ATG initiation codon and adjacent ribosome binding site or other sequences. In addition, the efficiency of expression may be enhanced by the inclusion of enhancers appropriate to the cell system in use (see, e. g., Scharf et al., Results Probl. Cell Differ. 20: 125, 1994; and Bittner et al., Meth. Enzymol., 153:516, 1987). For example, the SV40 enhancer or CMV enhancer may be used to increase expression in mammalian host cells.

Vectors to be used typically encode the antibody variable domain or the antibody light and heavy chain including constant regionsor parts thereof, if present. Such vectors allow expression of the variable regions as fusion proteins with the constant regions thereby leading to production of intact antibodies and antibody variable domains thereof. Typically, such constant regions are human.

The term "recombinant host cell" (or simply "host cell") refers to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The host cells for harboring and expressing the antibody variable domain or the antibody of the invention can be either prokaryotic or eukaryotic. *E. coli* is one prokaryotic host useful for cloning and expressing the polynucleotides of the present invention. Other microbial hosts suitable for use include bacilli, such as *Bacillus subtilis,* and other enterobacteriaceae, such as Salmonella, Serratia, and various Pseudomonas species. In these prokaryotic hosts, one can also make expression vectors, which typically contain expression control sequences compatible with the host cell (e. g., an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express the antibody variable domain or multispecific antibodies of the invention. Insect cells in combination with baculovirus vectors can also be used.

In one embodiment, mammalian host cells are used to express and produce the antibody variable domain or the antibody of the invention. For example, they can be either a hybridoma cell line expressing endogenous immunoglobulin genes or a mammalian cell line harboring an exogenous expression vector. These include any normal mortal or normal or abnormal immortal animal or human cell. For example, a number of suitable host cell lines capable of secreting intact immunoglobulins have been developed including the CHO cell lines, various COS cell lines, HeLa cells, myeloma cell lines, transformed B-cells and hybridomas. The use of mammalian tissue cell culture to express polypeptides is discussed generally in, e. g., Winnacker, FROM GENES TO CLONES, VCH Publishers, N.Y., N.Y., 1987. Expression vectors for mammalian host cells can include expression control sequences, such as an origin of replication, a promoter, and an enhancer (see, e. g., Queen, et al., Immunol. Rev. 89:49-68, 1986), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP polIII promoter, the constitutive MPS V promoter, the tetracycline-inducible CMV promoter (such as the human immediate-early CMV promoter), the constitutive CMV promoter, and promoter-enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Green, M. R., and Sambrook, J., Molecular Cloning: A Laboratory Manual (Fourth Edition), Cold Spring Harbor Laboratory Press (2012)). Other methods include, e. g., electroporation, calcium phosphate treatment, liposome-mediated transformation, injection and microinjection, ballistic methods, virosomes, immunoliposomes, polycation-nucleic acid conjugates, naked DNA, artificial virions, fusion to the herpes virus structural protein VP22 (Elliot and O'Hare, Cell 88:223, 1997), agent-enhanced uptake of DNA, and ex vivo transduction. For long-term, high-yield production of recombinant proteins, stable expression will often be desired. For example, cell lines which stably express the antibody variable domain or the antibody of the invention can be prepared using expression vectors of the invention which contain viral origins of replication or endogenous expression elements and a selectable marker gene. Following the introduction of the vector, cells may be allowed to grow for 1 to 2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type. The present invention thus provides a method of producing the antibody variable domain or the antibody of the invention, wherein said method comprises the step of culturing a host cell comprising a nucleic acid or a vector encoding the antibody variable domain or the antibody of the invention, whereby said antibody variable domain or said antibody of the disclosure is expressed.

In one aspect, the present invention relates to a method of producing the antibody variable domain or the antibody of the invention, the method comprising the step of culturing a host cell expressing a nucleic acid or two nucleic acids encoding the antibody variable domain or the antibody of the invention. In particular, the present invention relates to a method of producing the antibody variable domain or the antibody of the invention, the method comprising (i) providing a nucleic acid or two nucleic acids encoding the antibody variable domain or the antibody of the invention or one or two vectors encoding the antibody variable domain or the antibody of the invention, expressing said nucleic acid or nucleic acids, or said vector or vectors, and collecting said antibody variable domain or said antibody from the expression system, or (ii) providing a host cell or host cells expressing a nucleic acid or two nucleic acids encoding the antibody variable domain or the antibody of the invention, culturing said host cell or said host cells; and collecting said antibody variable domain or said multispecific antibody from the cell culture.

In a further aspect, the present invention relates to a pharmaceutical composition comprising the antibody of the invention, and a pharmaceutically acceptable carrier. "Pharmaceutically acceptable carrier" means a medium or diluent that does not interfere with the structure of the antibodies. Pharmaceutically acceptable carriers enhance or stabilize the composition, or facilitate preparation of the composition. Pharmaceutically acceptable carriers include solvents, dispersion media, coatings, antibacterial and antifungal agents, isotonic and absorption delaying agents, and the like that are physiologically compatible.

Certain of such carriers enable pharmaceutical compositions to be formulated as, for example, tablets, pills, dragees, capsules, liquids, gels, syrups, slurries, suspension and lozenges for the oral ingestion by a subject. Certain of such carriers enable pharmaceutical compositions to be formulated for injection, infusion or topical administration. For example, a pharmaceutically acceptable carrier can be a sterile aqueous solution.

Pharmaceutical compositions in accordance with the present disclosure may further routinely contain pharmaceutically acceptable concentrations of salt, buffering agents, preservatives, supplementary immune potentiating agents such as adjuvants and cytokines and optionally other therapeutic agents. The composition may also include antioxidants and/or preservatives. As antioxidants may be mentioned thiol derivatives (e. g. thioglycerol, cysteine, acetylcysteine, cystine, dithioerythreitol, dithiothreitol, glutathione), tocopherols, butylated hydroxyanisole, butylated hydroxytoluene, sulfurous acid salts (e. g. sodium sulfate, sodium bisulfite, acetone sodium bisulfite, sodium metabisulfite, sodium sulfite, sodium formaldehyde sulfoxylate, sodium thiosulfate) and nordihydroguaiaretic acid. Suitable preservatives may for instance be phenol, chlorobutanol, benzylalcohol, methyl paraben, propyl paraben, benzalkonium chloride and cetylpyridinium chloride.

The pharmaceutical composition of the invention can be administered by a variety of methods known in the art. The route and/or mode of administration vary depending upon the desired results. Administration can be intravenous, intramuscular, intraperitoneal, or subcutaneous, or administered proximal to the site of the target. The pharmaceutically acceptable carrier should be suitable for intravenous, intramuscular, subcutaneous, parenteral, spinal or epidermal administration (e. *g.*, by injection or infusion). Depending on the route of administration, the active compound, *i.* e., the antibody of the invention, may be coated in a material to protect the compound from the action of acids and other natural conditions that may inactivate the compound.

The pharmaceutical compositions of the invention can be prepared in accordance with methods well known and routinely practiced in the art. See, e. g., Remington: The Science and Practice of Pharmacy, Mack Publishing Co., 20th ed., 2000; and Sustained and Controlled Release Drug Delivery Systems, J. R. Robinson, ed., Marcel Dekker, Inc., New York, 1978. Pharmaceutical compositions are preferably manufactured under GMP conditions. Typically, a therapeutically effective dose or efficacious dose of the antibody of the invention is employed in the pharmaceutical compositions of the invention. The antibodies of the invention are formulated into pharmaceutically acceptable dosage forms by conventional methods known to those of skill in the art. Dosage regimens are adjusted to provide the optimum desired response (e. *g.*, a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions of the invention can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

In one aspect, the present invention relates to the antibody of the invention or the pharmaceutical composition of the invention for use as a medicament. In a suitable embodiment, the present invention provides the multispecific antibody or the pharmaceutical composition for use in the treatment of a proliferative disease, such as cancer, or a disease selected from allergic, inflammatory and autoimmune diseases.

In another aspect, the present invention provides the pharmaceutical composition of the invention for use in the manufacture of a medicament for the treatment of a proliferative disease, such as cancer, or a disease selected from allergic, inflammatory and autoimmune diseases.

In another aspect, the present invention relates to the use of the antibody or the pharmaceutical composition of the present invention for treating a proliferative disease, such as cancer, or a disease selected from allergic, inflammatory and autoimmune diseases, in a subject in need thereof.

In another aspect, the present invention relates to a method of treating a subject comprising administering to the subject a therapeutically effective amount of the antibody of the present invention. In a suitable embodiment, the present invention relates to a method for the treatment of a proliferative disease, such as cancer, or a disease selected from allergic, inflammatory and autoimmune diseases, in a subject comprising administering to the subject a therapeutically effective amount of the antibody of the present invention.

The term "subject" includes human and non-human animals.

The term "animals" include all vertebrates, e. g., non-human mammals and non-mammals, such as non-human primates, sheep, dog, cow, chickens, amphibians, and reptiles. Except when noted, the terms "patient" or "subject" are used herein interchangeably.

The terms "treatment", "treating", "treat", "treated", and the like, as used herein, refer to obtaining a desired pharmacologic and/or physiologic effect. The effect may be therapeutic in terms of a partial or complete cure for a disease and/or adverse effect attributable to the disease or delaying the disease progression. "Treatment", as used herein, covers any treatment of a disease in a mammal, e. *g.*, in a human, and includes: (a) inhibiting the disease, *i.* e., arresting its development; and (b) relieving the disease, *i.* e., causing regression of the disease.

The term "therapeutically effective amount" or "efficacious amount" refers to the amount of an agent that, when administered to a mammal or other subject for treating a disease, is sufficient to affect such treatment for the disease. The "therapeutically effective amount" will vary depending on the agent, the disease and its severity and the age, weight, etc., of the subject to be treated.

In a final aspect, the present invention relates to a method for modifying an antibody, where the antibody is fragment-based or is an antibody comprising one or more scFv fragments, the method comprises the step of introducing one or more of the following substitutions (AHo numbering) in the VH sequence(s) of said fragment-based antibody or in the VH sequence(s) of the scFv fragment(s) of said antibody:

- an alanine (A), serine (S), lysine (K), arginine (R), aspartate (D), glutamate (E), asparagine (N) or glutamine (Q) at amino acid position 101;
- an alanine (A), serine (S), lysine (K), arginine (R), aspartate (D), glutamate (E), asparagine (N) or glutamine (Q) at amino acid position 146;
- a leucine (L), lysine (K), or asparagine (N) at amino acid position 148

  to obtain a modified antibody;
  wherein the modified antibody exhibits a decreased binding to pre-existing anti-drug-antibodies (ADAs) present in human sera from healthy donors when compared to its unmodified version, and wherein the decrease in binding is determined by an ELISA-based pre-existing anti-drug-antibody binding assay.

In particular embodiments of said final aspect, said modified antibody comprises an antibody variable domain in accordance with the present invention, *i.* e. as defined in the claims, in items 1 to 23 or in the detailed description of the invention.

**Sequence listing (residues designated according to AHo numbering scheme; the CDRs defined according to Numab CDR definition, unless specified otherwise)**

Table 1. Examples of *VH/VL* sequences of *PRO1922* variants according to the present invention and *VH/VL* sequences of original (unmodified) *PRO1922* and reference / comparative *PRO1922* variants (modifications relative to original *PRO1922* are shown in bold; CDR residues are shown in bold and italic letters).

| Original (unmodified) PRO1922 | | |
|---|---|---|
| **SEQ ID NO:** | **Description:** | **Sequence:** |
| 1 | **VH** 54-21-H03-sc01 | EVQLVESGGGLVQPGGSLRLSCAAS*GFSFSTTYYMC*WVRQAPGKGLEWIG*C TNTASSVRTYYATWAKG*RFTISRDNSKNTVYLQMNSLRAEDTAVYYCA*RDMG FADYALNL*WGQGTLVTVSS |
| 2 | **VH** 54-21-H03-sc01_(**G51C**) | EVQLVESGGGLVQPGGSLRLSCAAS*GFSFSTTYYMC*WVRQAPGK**C**LEWIG*CT NTASSVRTYYATWAKG*RFTISRDNSKNTVYLQMNSLRAEDTAVYYCA*RDMGF ADYALNL*WGQGTLVTVSS |
| 3 | **VL** 54-21-H03-sc01 | DIQMTQSPSSLSASVGDRVTITC*QASESIYSSLA*WYQQKPGKAPKLLIY*LASTL AS*GVPSRFSGSGSGTDFTLTISSLQPEDFATYYC*QSTDYTTSTHRNS*FGTGTK VTVLG |
| 4 | **VL** 54-21-H03-sc01_(**T141C**) | DIQMTQSPSSLSASVGDRVTITC*QASESIYSSLA*WYQQKPGKAPKLLIY*LASTL AS*GVPSRFSGSGSGTDFTLTISSLQPEDFATYYC*QSTDYTTSTHRNS*FG**C**GTK VTVLG |
| PRO1922 variants (according to the invention) | | |
| 7 | **VH** 54-21-H03-sc01_(**T101S**) | EVQLVESGGGLVQPGGSLRLSCAAS*GFSFSTTYYMC*WVRQAPGKGLEWIG*C TNTASSVRTYYATWAKG*RFTISRDNSKNTVYLQMNSLRAED**S**AVYYCA*RDMG FADYALNL*WGQGTLVTVSS |
| 8 | **VH** 54-21-H03-sc01_(**T101A**) | EVQLVESGGGLVQPGGSLRLSCAAS*GFSFSTTYYMC*WVRQAPGKGLEWIG*C TNTASSVRTYYATWAKG*RFTISRDNSKNTVYLQMNSLRAED**A**AVYYCA*RDMG FADYALNL*WGQGTLVTVSS |
| 9 | **VH** 54-21 - H 03-sc0 1 _(T 1 01 R) | EVQLVESGGGLVQPGGSLRLSCAAS*GFSFSTTYYMC*WVRQAPGKGLEWIG*C TNTASSVRTYYATWAKG*RFTISRDNSKNTVYLQMNSLRAED**R**AVYYCA*RDMG FADYALNL*WGQGTLVTVSS |

(continued)

| PRO1922 variants (according to the invention) | | |
|---|---|---|
| 10 | **VH** 54-21-H03-sc01_(T101Q) | EVQLVESGGGLVQPGGSLRLSCAAS*GFSFSTTYYMC*WVRQAPGKGLEWIG*C TNTASSVRTYYATWAKG*RFTISRDNSKNTVYLQMNSLRAEDQAVYYCA*RDMG FADYALNL*WGQGTLVTVSS |
| 11 | **VH** 54-21-H03-sc01_(T146A) | EVQLVESGGGLVQPGGSLRLSCAAS*GFSFSTTYYMC*WVRQAPGKGLEWIG*C TNTASSVRTYYATWAKG*RFTISRDNSKNTVYLQMNSLRAEDTAVYYCA*RDMG FADYALNL*WGQGTLVAVSS |
| 12 | **VH** 54-21-H03-sc01_(T146K) | EVQLVESGGGLVQPGGSLRLSCAAS*GFSFSTTYYMC*WVRQAPGKGLEWIG*C TNTASSVRTYYATWAKG*RFTISRDNSKNTVYLQMNSLRAEDTAVYYCA*RDMG FADYALNL*WGQGTLVKVSS |
| 13 | **VH** 54-21-H03-sc01_(T146R) | EVQLVESGGGLVQPGGSLRLSCAAS*GFSFSTTYYMC*WVRQAPGKGLEWIG*C TNTASSVRTYYATWAKG*RFTISRDNSKNTVYLQMNSLRAEDTAVYYCA*RDMG FADYALNL*WGQGTLVRVSS |
| 14 | **VH** 54-21-H03-sc01_(T146E) | EVQLVESGGGLVQPGGSLRLSCAAS*GFSFSTTYYMC*WVRQAPGKGLEWIG*C TNTASSVRTYYATWAKG*RFTISRDNSKNTVYLQMNSLRAEDTAVYYCA*RDMG FADYALNL*WGQGTLVEVSS |
| 15 | **VH** 54-21-H03-sc01_(T146Q) | EVQLVESGGGLVQPGGSLRLSCAAS*GFSFSTTYYMC*WVRQAPGKGLEWIG*C TNTASSVRTYYATWAKG*RFTISRDNSKNTVYLQMNSLRAEDTAVYYCA*RDMG FADYALNL*WGQGTLVQVSS |
| 16 | **VH** 54-21-H03-sc01_(T101S, T146Q) | EVQLVESGGGLVQPGGSLRLSCAAS*GFSFSTTYYMC*WVRQAPGKGLEWIG*C TNTASSVRTYYATWAKG*RFTISRDNSKNTVYLQMNSLRAEDSAVYYCA*RDMG FADYALNL*WGQGTLVQVSS |
| 17 | **VH** 54-21- H 03-sc0 1 _(T101R, T146Q) | EVQLVESGGGLVQPGGSLRLSCAAS*GFSFSTTYYMC*WVRQAPGKGLEWIG*C TNTASSVRTYYATWAKG*RFTISRDNSKNTVYLQMNSLRAEDRAVYYCA*RDMG FADYALNL*WGQGTLVQVSS |

| PRO1922 variants (according to the invention) | | |
|---|---|---|
| 18 | VH 54-21-H03-sc01_(T101S, T146R) | EVQLVESGGGLVQPGGSLRLSCAAS*GFSFSTTYYMC*WVRQAPGKGLEWIG*C* *TNTASSVRTYYATWAKG*RFTISRDNSKNTVYLQMNSLRAEDSAVYYCA*RDMG* *FADYALNL*WGQGTLVRVSS |
| 19 | VH 54-21-H03-sc01_(T101Q, T146Q) | EVQLVESGGGLVQPGGSLRLSCAAS*GFSFSTTYYMC*WVRQAPGKGLEWIG*C* *TNTASSVRTYYATWAKG*RFTISRDNSKNTVYLQMNSLRAEDQAVYYCA*RDMG* *FADYALNL*WGQGTLVQVSS |
| 20 | VH 54-21-H03-sc01_(T101K, T146Q) | EVQLVESGGGLVQPGGSLRLSCAAS*GFSFSTTYYMC*WVRQAPGKGLEWIG*C* *TNTASSVRTYYATWAKG*RFTISRDNSKNTVYLQMNSLRAEDKAVYYCA*RDMG* *FADYALNL*WGQGTLVQVSS |
| 21 | VH 54-21-H03-sc01_(T101N, T146Q) | EVQLVESGGGLVQPGGSLRLSCAAS*GFSFSTTYYMC*WVRQAPGKGLEWIG*C* *TNTASSVRTYYATWAKG*RFTISRDNSKNTVYLQMNSLRAEDNAVYYCA*RDMG* *FADYALNL*WGQGTLVQVSS |
| 22 | VH 54-21-H03-sc01_(T101S, T146K) | EVQLVESGGGLVQPGGSLRLSCAAS*GFSFSTTYYMC*WVRQAPGKGLEWIG*C* *TNTASSVRTYYATWAKG*RFTISRDNSKNTVYLQMNSLRAEDSAVYYCA*RDMG* *FADYALNL*WGQGTLVKVSS |
| 23 | VH 54-21-H03-sc01_(T101S, T146S) | EVQLVESGGGLVQPGGSLRLSCAAS*GFSFSTTYYMC*WVRQAPGKGLEWIG*C* *TNTASSVRTYYATWAKG*RFTISRDNSKNTVYLQMNSLRAEDSAVYYCA*RDMG* *FADYALNL*WGQGTLVSVSS |
| 24 | VH 54-21-H03-sc01_(T101K, T146D) | EVQLVESGGGLVQPGGSLRLSCAAS*GFSFSTTYYMC*WVRQAPGKGLEWIG*C* *TNTASSVRTYYATWAKG*RFTISRDNSKNTVYLQMNSLRAEDKAVYYCA*RDMG* *FADYALNL*WGQGTLVDVSS |
| 25 | VH 54-21-H03-sc01_(T101R, T146E) | EVQLVESGGGLVQPGGSLRLSCAAS*GFSFSTTYYMC*WVRQAPGKGLEWIG*C* *TNTASSVRTYYATWAKG*RFTISRDNSKNTVYLQMNSLRAEDRAVYYCA*RDMG* *FADYALNL*WGQGTLVEVSS |

| PRO1922 variants (according to the invention) | | |
|---|---|---|
| 26 | **VH** 54-21-H03-sc01_(L144K, S148L) | EVQLVESGGGLVQPGGSLRLSCAAS*GFSFSTTYYMC*WVRQAPGKGLEWIG*C* *TNTASSVRTYYATWAKG*RFTISRDNSKNTVYLQMNSLRAEDTAVYYCA*RDMG* *FADYALNL*WGQGTKVTVLS |
| 27 | **VH** 54-21-H03-sc01_(L144A, S148N) | EVQLVESGGGLVQPGGSLRLSCAAS*GFSFSTTYYMC*WVRQAPGKGLEWIG*C* *TNTASSVRTYYATWAKG*RFTISRDNSKNTVYLQMNSLRAEDTAVYYCA*RDMG* *FADYALNL*WGQGTAVTVNS |
| 28 | **VH** 54-21-H03-sc01_(L144K, T146E, S148K) | EVQLVESGGGLVQPGGSLRLSCAAS*GFSFSTTYYMC*WVRQAPGKGLEWIG*C* *TNTASSVRTYYATWAKG*RFTISRDNSKNTVYLQMNSLRAEDTAVYYCA*RDMG* *FADYALNL*WGQGTKVEVKS |
| 29 | **VH** 54-21-H03-sc01_(T101S, L144A, T146Q) | EVQLVESGGGLVQPGGSLRLSCAAS*GFSFSTTYYMC*WVRQAPGKGLEWIG*C* *TNTASSVRTYYATWAKG*RFTISRDNSKNTVYLQMNSLRAED**S**AVYYCA*RDMG* *FADYALNL*WGQGTAVQVSS |
| 30 | **VH** 54-21-H03-sc01_(L12A, T101S, L144A, T146Q) | EVQLVESGGG**A**VQPGGSLRLSCAAS*GFSFSTTYYMC*WVRQAPGKGLEWIG*C* *TNTASSVRTYYATWAKG*RFTISRDNSKNTVYLQMNSLRAED**S**AVYYCA*RDMG* *FADYALNL*WGQGTAVQVSS |
| 31 | **VH** 54-21-H03-sc01_(L12A, T101R, L144A, T146Q) | EVQLVESGGG**A**VQPGGSLRLSCAAS*GFSFSTTYYMC*WVRQAPGKGLEWIG*C* *TNTASSVRTYYATWAKG*RFTISRDNSKNTVYLQMNSLRAED**R**AVYYCA*RDMG* *FADYALNL*WGQGTAVQVSS |
| 32 | **VH** 54-21-H03-sc01_(L12A, T101S, L144A, T146R) | EVQLVESGGG**A**VQPGGSLRLSCAAS*GFSFSTTYYMC*WVRQAPGKGLEWIG*C* *TNTASSVRTYYATWAKG*RFTISRDNSKNTVYLQMNSLRAED**S**AVYYCA*RDMG* *FADYALNL*WGQGTAVRVSS |
| 33 | **VH** 54-21-H03-sc01_(L12A, T101S, L144K, T146Q) | EVQLVESGGG**A**VQPGGSLRLSCAAS*GFSFSTTYYMC*WVRQAPGKGLEWIG*C* *TNTASSVRTYYATWAKG*RFTISRDNSKNTVYLQMNSLRAED**S**AVYYCA*RDMG* *FADYALNL*WGQGTKVQVSS |

| PRO1922 variants (according to the invention) | | |
|---|---|---|
| 34 | **VH** 54-21-H03-sc01_(L12R, T101S, L144A, T146Q) | EVQLVESGGG**R**VQPGGSLRLSCAAS*GFSFSTTYYMC*WVRQAPGKGLEWIG*C TNTASSVRTYYATWAKG*RFTISRDNSKNTVYLQMNSLRAED**S**AVYYCA*RDMG FADYALNL*WGQGTA**VQ**VSS |
| 35 | **VH** 54-21-H03-sc01_(L12R, T101R, L144A, T146Q) | EVQLVESGGG**R**VQPGGSLRLSCAAS*GFSFSTTYYMC*WVRQAPGKGLEWIG*C TNTASSVRTYYATWAKG*RFTISRDNSKNTVYLQMNSLRAED**R**AVYYCA*RDMG FADYALNL*WGQGTA**VQ**VSS |
| 36 | **VH** 54-21-H03-sc01_(L12A, T101Q, L144A, T146Q) | EVQLVESGGG**A**VQPGGSLRLSCAAS*GFSFSTTYYMC*WVRQAPGKGLEWIG*C TNTASSVRTYYATWAKG*RFTISRDNSKNTVYLQMNSLRAED**Q**AVYYCA*RDMG FADYALNL*WGQGTA**VQ**VSS |
| 37 | **VL** 54-21-H03-sc01_(F101E) | DIQMTQSPSSLSASVGDRVTITC*QASESIYSSLA*WYQQKPGKAPKLLIY*LASTL AS*GVPSRFSGSGSGTDFTLTISSLQPED**E**ATYYC*QSTDYTTSTHRNS*FGTGTK VQVLG |
| 38 | **VL** 54-21-H03-sc01_(F1 01R) | DIQMTQSPSSLSASVGDRVTITC*QASESIYSSLA*WYQQKPGKAPKLLIY*LASTL AS*GVPSRFSGSGSGTDFTLTISSLQPED**R**ATYYC*QSTDYTTSTHRNS*FGTGTK VTVLG |
| 39 | **VL** 54-21-H03-sc01_(F101Q) | DIQMTQSPSSLSASVGDRVTITC*QASESIYSSLA*WYQQKPGKAPKLLIY*LASTL AS*GVPSRFSGSGSGTDFTLTISSLQPED**Q**ATYYC*QSTDYTTSTHRNS*FGTGTK VTVLG |
| Reference / comparative PRO1922 variants (not according to the invention) | | |
| **SEQ ID NO:** | **Description:** | Sequence: |
| 5 | **VH** 54-21-H03-sc01_(P15A) | EVQLVESGGGLVQ**A**GGSLRLSCAAS*GFSFSTTYYMC*WVRQAPGKGLEWIG*C TNTASSVRTYYATWAKG*RFTISRDNSKNTVYLQMNSLRAEDTAVYYCA*RDMG FADYALNL*WGQGTLVTVSS |
| 6 | **VH** 54-21-H03-sc01_(P48A) | EVQLVESGGGLVQPGGSLRLSCAAS*GFSFSTTYYMC*WVRQA**A**GKGLEWIG*C TNTASSVRTYYATWAKG*RFTISRDNSKNTVYLQMNSLRAEDTAVYYCA*RDMG FADYALNL*WGQGTLVTVSS |

EP 4 273 162 A1

31

| Reference / comparative PRO1922 variants (not according to the invention) | | |
|---|---|---|
| **SEQ ID NO:** | **Description:** | Sequence: |
| 40 | **VH** 54-21-H03-sc01_(L12R, V103T, L144Q) | EVQLVESGGG**R**VQPGGSLRLSCAAS*GFSFSTTYYMC*WVRQAPGKGLEWIG*C TNTASSVRTYYATWAKG*RFTISRDNSKNTVYLQMNSLRAEDTATYYCA*RDMG FADYALNL*WGQGT**Q**VTVSS |
| 41 | **VH** 54-21-H03-sc01_(L12R, G51C, V103T, L144Q) | EVQLVESGGG**R**VQPGGSLRLSCAAS*GFSFSTTYYMC*WVRQAPGK**C**LEWIG*C TNTASSVRTYYATWAKG*RFTISRDNSKNTVYLQMNSLRAEDTATYYCA*RDMG FADYALNL*WGQGT**Q**VTVSS |
| 42 | **VH** 54-21-H03-sc01_(L12S, V103T, L144T) | EVQLVESGGG**S**VQPGGSLRLSCAAS*GFSFSTTYYMC*WVRQAPGKGLEWIG*C TNTASSVRTYYATWAKG*RFTISRDNSKNTVYLQMNSLRAEDTATYYCA*RDMG FADYALNL*WGQGTTVTVSS |

*Table 2. Examples of scFv sequences of **PRO1922** variants according to the present invention and scFv sequences of original (unmodified) **PRO1922** and reference / comparative **PRO1922** variants (modifications relative to original **PRO1922** and linker residues, if present, are shown in bold).*

| Original (unmodified) PRO1922 | | |
|---|---|---|
| **SEQ ID NO:** | **Description:** | **Sequence:** |
| 43 | **scFv** 54-21-H03-sc01 (PRO1922) | DIQMTQSPSSLSASVGDRVTITC***QASESIYSSLA***WYQQKPGKAPKLLIY***LASTLAS***GVPSRFSGSGSGTDFTLTISSLQPEDFATYYC***QSTDYTTSTHRNS***FGTGTKVTVLG**GGGGSGGGGSGGGGSGGGGS**EVQLVESGGGLVQPGGSLRLSCAAS***GFSFSTTYYMC***WVRQAPGKGLEWIG***CTNTASSVRTYYATWAKG***RFTISRDNSKNTVYLQMNSLRAEDTAVYYCA***RDMGFADYALNL***WGQGTLVTVSS |
| 44 | **scFv** 54-21-H03-sc01_(VL_G141C, VH_G51C) | DIQMTQSPSSLSASVGDRVTITC***QASESIYSSLA***WYQQKPGKAPKLLIY***LASTLAS***GVPSRFSGSGSGTDFTLTISSLQPEDFATYYC***QSTDYTTSTHRNS***FGCGTKVTVLG**GGGGSGGGGSGGGGS**EVQLVESGGGLVQPGGSLRLSCAAS***GFSFSTTYYMC***WVRQAPGKCLEWIG***CTNTASSVRTYYATWAKG***RFTISRDNSKNTVYLQMNSLRAEDTAVYYCA***RDMGFADYALNL***WGQGTLVTVSS |
| **PRO1922 variants (according to the invention)** | | |
| **SEQ ID NO:** | **Description:** | **Sequence:** |
| 45 | **scFv** 54-21-H03-sc01_(P15A) PRO2993 | DIQMTQSPSSLSASVGDRVTITC***QASESIYSSLA***WYQQKPGKAPKLLIY***LASTLAS***GVPSRFSGSGSGTDFTLTISSLQPEDFATYYC***QSTDYTTSTHRNS***FGTGTKVTVLG**GGGGSGGGGSGGGGS**EVQLVESGGGLVQ**A**GGSLRLSCAAS***GFSFSTTYYMC***WVRQAPGKGLEWIG***CTNTASSVRTYYATWAKG***RFTISRDNSKNTVYLQMNSLRAEDTAVYYCA***RDMGFADYALNL***WGQGTLVTVSS |
| 46 | **scFv** 54-21-H03-sc01_(P48A) PRO2994 | DIQMTQSPSSLSASVGDRVTITC***QASESIYSSLA***WYQQKPGKAPKLLIY***LASTLAS***GVPSRFSGSGSGTDFTLTISSLQPEDFATYYC***QSTDYTTSTHRNS***FGTGTKVTVLG**GGGGSGGGGSGGGGS**EVQLVESGGGLVQPGGSLRLSCAAS***GFSFSTTYYMC***WVRQA**A**GKGLEWIG***CTNTASSVRTYYATWAKG***RFTISRDNSKNTVYLQMNSLRAEDTAVYYCA***RDMGFADYALNL***WGQGTLVTVSS |
| 47 | **scFv** 54-21-H03-sc01_(T101S) PRO2946 | DIQMTQSPSSLSASVGDRVTITC***QASESIYSSLA***WYQQKPGKAPKLLIY***LASTLAS***GVPSRFSGSGSGTDFTLTISSLQPEDFATYYC***QSTDYTTSTHRNS***FGTGTKVTVLG**GGGGSGGGGSGGGGS**EVQLVESGGGLVQPGGSLRLSCAAS***GFSFSTTYYMC***WVRQAPGKGLEWIG***CTNTASSVRTYYATWAKG***RFTISRDNSKNTVYLQMNSLRAED**S**AVYYCA***RDMGFADYALNL***WGQGTLVTVSS |

EP 4 273 162 A1

| PRO1922 variants (according to the invention) | | |
|---|---|---|
| **SEQ ID NO:** | **Description:** | **Sequence:** |
| 48 | **scFv 54-21-H03-sc01_(T101A)** PRO2991 | DIQMTQSPSSLSASVGDRVTITC*QASESIYSSLA*WYQQKPGKAPKLLIY*LASTLAS*GVPSRFSGSGSGTDFTLTISSLQPEDFATYYCQSTDYTTSTHRNSFGTGTKVTVLG**GGGGSGGGGSGGGGSGGGGS**EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDAAVYYCA*RDMGFADYALNL*WGQGTLVTVSS |
| 49 | **scFv 54-21-H03-sc01_(T101R)** PRO2945 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG**GGGGSGGGGSGGGGSGGGGS**EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDRAVYYCA*RDMGFADYALNL*WGQGTLVTVSS |
| 50 | **scFv 54-21-H03-sc01_(T101Q)** PRO3329 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG**GGGGSGGGGSGGGGSGGGGS**EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDQAVYYCA*RDMGFADYALNL*WGQGTLVTVSS |
| 51 | **scFv 54-21-H03-sc01_(T146A)** PRO2992 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG**GGGGSGGGGSGGGGSGGGGS**EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDTAVYYCA*RDMGFADYALNL*WGQGTLVAVSS |
| 52 | **scFv 54-21-H03-sc01_(T146K)** PRO2941 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG**GGGGSGGGGSGGGGSGGGGS**EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDTAVYYCA*RDMGFADYALNL*WGQGTLVKVSS |

EP 4 273 162 A1

(continued)

| PRO1922 variants (according to the invention) | | |
|---|---|---|
| SEQ ID NO: | Description: | Sequence: |
| 53 | **scFv** 54-21-H03-sc01_(T146R) PRO2942 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG**GGGGSGGGGSGGGGSGGGGS**EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDTAVYYCA*RDMGFADYALNL*WGQGTLV**R**VSS |
| 54 | **scFv** 54-21-H03-sc01_(T146E) PRO2943 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG**GGGGSGGGGSGGGGSGGGGS**EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDTAVYYCA*RDMGFADYALNL*WGQGTLV**E**VSS |
| 55 | **scFv** 54-21-H03-sc01_(T146Q) PRO2944 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG**GGGGSGGGGSGGGGSGGGGS**EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDTAVYYCA*RDMGFADYALNL*WGQGTLV**Q**VSS |
| 56 | **scFv** 54-21-H03-sc01_(T101S, T146Q) PRO2936 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG**GGGGSGGGGSGGGGSGGGGS**EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAED**S**AVYYCA*RDMGFADYALNL*WGQGTLV**Q**VSS |
| 57 | **scFv** 54-21-H03-sc01_(T101R, T146Q) PRO3324 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG**GGGGSGGGGSGGGGSGGGGS**EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAED**R**AVYYCA*RDMGFADYALNL*WGQGTLV**Q**VSS |

| PRO1922 variants (according to the invention) | | |
|---|---|---|
| SEQ ID NO: | Description: | Sequence: |
| 58 | scFv 54-21-H03-sc01_(T101S, T146R) PRO3325 | DIQMTQSPSSLSASVGDRVTITC*QASESIYSSLA*WYQQKPGKAPKLLIY*LASTLAS*GVP SRFSGSGSGTDFTLTISSLQPEDFATYYC*QSTDYTTSTHRNS*FGTGTKVTVLG**GGGGS GGGGSGGGGSGGGGS**EVQLVESGGGLVQPGGSLRLSCAAS*GFSFSTTYYMC*WVR QAPGKGLEWIG*CTNTASSVRTYYATWAKG*RFTISRDNSKNTVYLQMNSLRAEDSAVY YCA*RDMGFADYALNL*WGQGTLV**R**VSS |
| 59 | scFv 54-21-H03-sc01_(T101Q, T146Q) PRO3326 | DIQMTQSPSSLSASVGDRVTITC*QASESIYSSLA*WYQQKPGKAPKLLIY*LASTLAS*GVP SRFSGSGSGTDFTLTISSLQPEDFATYYC*QSTDYTTSTHRNS*FGTGTKVTVLG**GGGGS GGGGSGGGGSGGGGS**EVQLVESGGGLVQPGGSLRLSCAAS*GFSFSTTYYMC*WVR QAPGKGLEWIG*CTNTASSVRTYYATWAKG*RFTISRDNSKNTVYLQMNSLRAED**Q**AVY YCA*RDMGFADYALNL*WGQGTLV**Q**VSS |
| 60 | scFv 54-21-H03-sc01_(T101K, T146Q) PRO3344 | DIQMTQSPSSLSASVGDRVTITC*QASESIYSSLA*WYQQKPGKAPKLLIY*LASTLAS*GVP SRFSGSGSGTDFTLTISSLQPEDFATYYC*QSTDYTTSTHRNS*FGTGTKVTVLG**GGGGS GGGGSGGGGSGGGGS**EVQLVESGGGLVQPGGSLRLSCAAS*GFSFSTTYYMC*WVR QAPGKGLEWIG*CTNTASSVRTYYATWAKG*RFTISRDNSKNTVYLQMNSLRAED**K**AVY YCA*RDMGFADYALNL*WGQGTLV**Q**VSS |
| 61 | scFv 54-21-H03-sc01_(T101N, T146Q) PRO3345 | DIQMTQSPSSLSASVGDRVTITC*QASESIYSSLA*WYQQKPGKAPKLLIY*LASTLAS*GVP SRFSGSGSGTDFTLTISSLQPEDFATYYC*QSTDYTTSTHRNS*FGTGTKVTVLG**GGGGS GGGGSGGGGSGGGGS**EVQLVESGGGLVQPGGSLRLSCAAS*GFSFSTTYYMC*WVR QAPGKGLEWIG*CTNTASSVRTYYATWAKG*RFTISRDNSKNTVYLQMNSLRAED**N**AVY YCA*RDMGFADYALNL*WGQGTLV**Q**VSS |
| 62 | scFv 54-21-H03-sc01_(T101S, T146K) PRO3346 | DIQMTQSPSSLSASVGDRVTITC*QASESIYSSLA*WYQQKPGKAPKLLIY*LASTLAS*GVP SRFSGSGSGTDFTLTISSLQPEDFATYYC*QSTDYTTSTHRNS*FGTGTKVTVLG**GGGGS GGGGSGGGGSGGGGS**EVQLVESGGGLVQPGGSLRLSCAAS*GFSFSTTYYMC*WVR QAPGKGLEWIG*CTNTASSVRTYYATWAKG*RFTISRDNSKNTVYLQMNSLRAEDSAVY YCA*RDMGFADYALNL*WGQGTLV**K**VSS |

EP 4 273 162 A1

| SEQ ID NO: | Description: | Sequence: |
|---|---|---|
| **PRO1922 variants (according to the invention)** | | |
| 63 | **scFv** 54-21-H03-sc01_(T101S, T146S) PRO3347 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG*GGGGSGGGGSGGGGSGGGGS*EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDSAVYYCA*RDMGFADYALNL*WGQGTLVSVSS |
| 64 | **scFv** 54-21-H03-sc01_(T101K, T146D) PRO3348 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG*GGGGSGGGGSGGGGSGGGGS*EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDKAVYYCA*RDMGFADYALNL*WGQGTLVDVSS |
| 65 | **scFv** 54-21-H03-sc01_(T101R, T146E) PRO3349 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG*GGGGSGGGGSGGGGSGGGGS*EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDRAVYYCA*RDMGFADYALNL*WGQGTLVEVSS |
| 66 | **scFv** 54-21-H03-sc01_(L144K, S148L) PRO2931 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG*GGGGSGGGGSGGGGSGGGGS*EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDTAVYYCA*RDMGFADYALNL*WGQGTKVTVLS |
| 67 | **scFv** 54-21-H03-sc01_(L144A, S148N) PRO2932 | DIQMTQSPSSLSASVGDRVTITC*QASESIYSSLA*WYQQKPGKAPKLLIY*LASTLAS*GVPSRFSGSGSGTDFTLTISSLQPEDFATYYC*QSTDYTTSTHRNS*FGTGTKVTVLG*GGGGSGGGGSGGGGSGGGGS*EVQLVESGGGLVQPGGSLRLSCAAS*GFSFSTTYYMC*WVRQAPGKGLEWIG*CTNTASSVRTYYATWAKG*RFTISRDNSKNTVYLQMNSLRAEDTAVYYCA*RDMGFADYALNL*WGQGTAVTVNS |

EP 4 273 162 A1

(continued)

EP 4 273 162 A1

| PRO1922 variants (according to the invention) | | |
|---|---|---|
| SEQ ID NO: | Description: | Sequence: |
| 68 | **scFv** 54-21-H03-sc01_(L144K, T146E, S148K) PRO2930 | DIQMTQSPSSLSASVGDRVTITC***QASESIYSSLA***WYQQKPGKAPKLLIY***LASTLAS***GVPSRFSGSGSGTDFTLTISSLQPEDFATYYC***QSTDYTTSTHRNS***FGTGTKVTVLG**GGGGSGGGGSGGGGSGGGGS**EVQLVESGGGLVQPGGSLRLSCAAS***GFSFSTTYYMC***WVRQAPGKGLEWIG***CTNTASSVRTYYATWAKG***RFTISRDNSKNTVYLQMNSLRAEDTAVYYCA***RDMGFADYALNL***WGQGTK**V**E**V**K**S** |
| 69 | **scFv** 54-21-H03-sc01_(T101S, L144A, T146Q) PRO2937 | DIQMTQSPSSLSASVGDRVTITC***QASESIYSSLA***WYQQKPGKAPKLLIY***LASTLAS***GVPSRFSGSGSGTDFTLTISSLQPEDFATYYC***QSTDYTTSTHRNS***FGTGTKVTVLG**GGGGSGGGGSGGGGSGGGGS**EVQLVESGGGLVQPGGSLRLSCAAS***GFSFSTTYYMC***WVRQAPGKGLEWIG***CTNTASSVRTYYATWAKG***RFTISRDNSKNTVYLQMNSLRAEDSAVYYCA***RDMGFADYALNL***WGQGTA**V**Q**V**SS |
| 70 | **scFv** 54-21-H03-sc01_(L12A, T101S, L144A, T146Q) PRO2938 | DIQMTQSPSSLSASVGDRVTITC***QASESIYSSLA***WYQQKPGKAPKLLIY***LASTLAS***GVPSRFSGSGSGTDFTLTISSLQPEDFATYYC***QSTDYTTSTHRNS***FGTGTKVTVLG**GGGGSGGGGSGGGGSGGGGS**EVQLVESGGG**A**VQPGGSLRLSCAAS***GFSFSTTYYMC***WVRQAPGKGLEWIG***CTNTASSVRTYYATWAKG***RFTISRDNSKNTVYLQMNSLRAEDSAVYYCA***RDMGFADYALNL***WGQGTA**V**Q**V**SS |
| 71 | **scFv** 54-21-H03-sc01_(L12A, T101R, L144A, T146Q) PRO3318 | DIQMTQSPSSLSASVGDRVTITC***QASESIYSSLA***WYQQKPGKAPKLLIY***LASTLAS***GVPSRFSGSGSGTDFTLTISSLQPEDFATYYC***QSTDYTTSTHRNS***FGTGTKVTVLG**GGGGSGGGGSGGGGSGGGGS**EVQLVESGGG**A**VQPGGSLRLSCAAS***GFSFSTTYYMC***WVRQAPGKGLEWIG***CTNTASSVRTYYATWAKG***RFTISRDNSKNTVYLQMNSLRAED**R**AVYYCA***RDMGFADYALNL***WGQGTA**V**Q**V**SS |
| 72 | **scFv** 54-21-H03-sc01_(L12A, T101S, L144A, T146R) PRO3319 | DIQMTQSPSSLSASVGDRVTITC***QASESIYSSLA***WYQQKPGKAPKLLIY***LASTLAS***GVPSRFSGSGSGTDFTLTISSLQPEDFATYYC***QSTDYTTSTHRNS***FGTGTKVTVLG**GGGGSGGGGSGGGGSGGGGS**EVQLVESGGG**A**VQPGGSLRLSCAAS***GFSFSTTYYMC***WVRQAPGKGLEWIG***CTNTASSVRTYYATWAKG***RFTISRDNSKNTVYLQMNSLRAEDSAVYYCA***RDMGFADYALNL***WGQGTA**V**RVSS |
| 73 | **scFv** 54-21-H03-sc01_(L12A, T101S, L144K, T146Q) | DIQMTQSPSSLSASVGDRVTITC***QASESIYSSLA***WYQQKPGKAPKLLIY***LASTLAS***GVPSRFSGSGSGTDFTLTISSLQPEDFATYYC***QSTDYTTSTHRNS***FGTGTKVTVLG**GGGGSGGGGSGGGGSGGGGS**EVQLVESGGG**A**VQPGGSLRLSCAAS***GFSFSTTYYMC***WVR |

(continued)

| PRO1922 variants (according to the invention) | | |
|---|---|---|
| **SEQ ID NO:** | **Description:** | **Sequence:** |
| | PRO3320 | QAPGKGLEWIG*CTNTASSVRTYYATWAKG*RFTISRDNSKNTVYLQMNSLRAEDSAVY YCA*RDMGFADYALNL*WGQGTKV**Q**VSS |
| 74 | **scFv** 54-21-H03-sc01_(L12R, T101S, L144A, T146Q) PRO3321 | DIQMTQSPSSLSASVGDRVTITC*QASESIYSSLA*WYQQKPGKAPKLLIY*LASTLAS*GVP SRFSGSGSGTDFTLTISSLQPEDFATYYC*QSTDYTTSTHRNS*FGTGTKVTVLGGGGGS GGGGSGGGGSGGGGSEVQLVESGGGRVQPGGSLRLSCAAS*GFSFSTTYYMC*WVR QAPGKGLEWIG*CTNTASSVRTYYATWAKG*RFTISRDNSKNTVYLQMNSLRAEDSAVY YCA*RDMGFADYALNL*WGQGTA**V**QVSS |
| 75 | **scFv** 54-21-H03-sc01_(L12R, T101R, L144A, T146Q) PRO3322 | DIQMTQSPSSLSASVGDRVTITC*QASESIYSSLA*WYQQKPGKAPKLLIY*LASTLAS*GVP SRFSGSGSGTDFTLTISSLQPEDFATYYC*QSTDYTTSTHRNS*FGTGTKVTVLGGGGGS GGGGSGGGGSGGGGSEVQLVESGGGRVQPGGSLRLSCAAS*GFSFSTTYYMC*WVR QAPGKGLEWIG*CTNTASSVRTYYATWAKG*RFTISRDNSKNTVYLQMNSLRAEDRAVY YCA*RDMGFADYALNL*WGQGTA**V**QVSS |
| 76 | **scFv** 54-21-H03-sc01_(L12A, T101Q, L144A, T146Q) PRO3323 | DIQMTQSPSSLSASVGDRVTITC*QASESIYSSLA*WYQQKPGKAPKLLIY*LASTLAS*GVP SRFSGSGSGTDFTLTISSLQPEDFATYYC*QSTDYTTSTHRNS*FGTGTKVTVLGGGGGS GGGGSGGGGSGGGGSEVQLVESGGGAVQPGGSLRLSCAAS*GFSFSTTYYMC*WVR QAPGKGLEWIG*CTNTASSVRTYYATWAKG*RFTISRDNSKNTVYLQMNSLRAEDQAVY YCA*RDMGFADYALNL*WGQGTA**V**QVSS |
| Reference / comparative PRO1922 variants (not according to the invention) | | |
| **SEQ ID NO:** | **Description:** | **Sequence:** |
| 77 | **scFv** 54-21-H03-sc01_(L12R, V103T, L144Q) (PRO2918) | DIQMTQSPSSLSASVGDRVTITC*QASESIYSSLA*WYQQKPGKAPKLLIY*LASTLAS*GVP SRFSGSGSGTDFTLTISSLQPEDFATYYC*QSTDYTTSTHRNS*FGTGTKVTVLGGGGGS GGGGSGGGGSGGGGSEVQLVESGGGRVQPGGSLRLSCAAS*GFSFSTTYYMC*WVR QAPGKGLEWIG*CTNTASSVRTYYATWAKG*RFTISRDNSKNTVYLQMNSLRAEDTATY YCA*RDMGFADYALNL*WGQGT**Q**VTVSS |

EP 4 273 162 A1

(continued)

| Reference / comparative PRO1922 variants (not according to the invention) | | |
|---|---|---|
| **SEQ ID NO:** | **Description:** | **Sequence:** |
| 78 | **scFv** 54-21-H03-sc01_(L12S, V103T, L144T) (PRO2990) | DIQMTQSPSSLSASVGDRVTITC***QASESIYSSLA***WYQQKPGKAPKLLIY***LASTLAS***GVP SRFSGSGSGTDFTLTISSLQPEDFATYYC***QSTDYTTSTHRNS***FGTGTKVTVLG**GGGGS GGGGSGGGGSGGGGS**EVQLVESGGG**S**VQPGGSLRLSCAAS***GFSFSTTYYMC***WVR QAPGKGLEWIG***CTNTASSVRTYYATWAKG***RFTISRDNSKNTVYLQMNSLRAEDTATY YCA***RDMGFADYALNL***WGQGTTVTVSS |

40

EP 4 273 162 A1

**Table 3. Examples of VH/VL sequences of PRO2230 variants according to the present invention (modifications relative to original PRO2230 are shown in bold; CDR residues are shown in bold and italic letters).**

| Original (unmodified) PRO2230 | | |
|---|---|---|
| **SEQ ID NO:** | **Description:** | **Sequence:** |
| 79 | **VH** 37-20-B03-sc09.1 | EVQLVESGGGLVQPGGSLRLSCAAS***GFSFNSDYWIY***WVRQAPGKGLEWIA***SIYGGSS GNTQYASWAQG***RFTISRDNSKNTVYLQMNSLRAEDTAVYFCA***RGYVDYGGATDL***WG QGTLVTVSS |
| 80 | **VH** 37-20-B03-sc09.1_(G51C) | EVQLVESGGGLVQPGGSLRLSCAAS***GFSFNSDYWIY***WVRQAPGK**C**LEWIA***SIYGGSS GNTQYASWAQG***RFTISRDNSKNTVYLQMNSLRAEDTAVYFCA***RGYVDYGGATDL***WG QGTLVTVSS |
| 81 | **VL** 37-20-B03-sc09.1 | DIQMTQSPASLSASVGDRVTITC***QASQSIGTYLA***WYQQKPGKPPKLLIY***RAFILAS***GVP SRFSGSGSGTDFTLTISSLQPEDFATYYC***QSNFYSDSTTIGPNA***FGTGTKVTVLG |
| 82 | **VL** 37-20-B03-sc09.1_(T141C) | DIQMTQSPASLSASVGDRVTITC***QASQSIGTYLA***WYQQKPGKPPKLLIY***RAFILAS***GVP SRFSGSGSGTDFTLTISSLQPEDFATYYC***QSNFYSDSTTIGPNA***FG**C**GTKVTVLG |
| PRO2230 variants (according to the invention) | | |
| 83 | **VH** 37-20-B03-sc09.1_(P15A) | EVQLVESGGGLVQ**A**GGSLRLSCAAS***GFSFNSDYWIY***WVRQAPGKGLEWIA***SIYGGSS GNTQYASWAQG***RFTISRDNSKNTVYLQMNSLRAEDTAVYFCA***RGYVDYGGATDL***WG QGTLVTVSS |
| 84 | **VH** 37-20-B03-sc09.1_(P48A) | EVQLVESGGGLVQPGGSLRLSCAAS***GFSFNSDYWIY***WVRQA**A**GKGLEWIA***SIYGGSS GNTQYASWAQG***RFTISRDNSKNTVYLQMNSLRAEDTAVYFCA***RGYVDYGGATDL***WG QGTLVTVSS |
| 85 | **VH** 37-20-B03-sc09.1_(T101S) | EVQLVESGGGLVQPGGSLRLSCAAS***GFSFNSDYWIY***WVRQAPGKGLEWIA***SIYGGSS GNTQYASWAQG***RFTISRDNSKNTVYLQMNSLRAED**S**AVYFCA***RGYVDYGGATDL***WG QGTLVTVSS |
| 86 | **VH** 37-20-B03-sc09.1_(T101A) | EVQLVESGGGLVQPGGSLRLSCAAS***GFSFNSDYWIY***WVRQAPGKGLEWIA***SIYGGSS GNTQYASWAQG***RFTISRDNSKNTVYLQMNSLRAED**A**AVYFCA***RGYVDYGGATDL***W GQGTLVTVSS |

| SEQ ID NO: | Description: | Sequence: |
|---|---|---|
| | **Original (unmodified) PRO2230** | |
| 87 | **VH** 37-20-B03-sc09.1_(T101R) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDRAVYFCA*RGYVDYGGATDL*W GQGTLVTVSS |
| 88 | **VH** 37-20-B03-sc09.1_(T101Q) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDQAVYFCA*RGYVDYGGATDL*W GQGTLVTVSS |
| 89 | **VH** 37-20-B03-sc09.1_(T146A) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDTAVYFCA*RGYVDYGGATDL*WG QGTLVAVSS |
| 90 | **VH** 37-20-B03-sc09.1_(T146K) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDTAVYFCA*RGYVDYGGATDL*WG QGTLVKVSS |
| 91 | **VH** 37-20-B03-sc09.1_(T146R) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDTAVYFCA*RGYVDYGGATDL*WG QGTLVRVSS |
| 92 | **VH** 37-20-B03-sc09.1_(T146E) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDTAVYFCA*RGYVDYGGATDL*WG QGTLVEVSS |
| 93 | **VH** 37-20-B03-sc09.1_(T146Q) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDTAVYFCA*RGYVDYGGATDL*WG QGTLVQVSS |
| 94 | **VH** 37-20-B03-SC09.1_(T101S, T146Q) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDSAVYFCA*RGYVDYGGATDL*WG QGTLVQVSS |

(continued)

**Original (unmodified) PRO2230**

| SEQ ID NO: | Description: | Sequence: |
|---|---|---|
| 95 | **VH** 37-20-B03-sc09.1_(T101R, T146Q) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDRAVYFCA*RGYVDYGGATDL*W GQGTLV**Q**VSS |
| 96 | **VH** 37-20-B03-SC09.1_(T101S, T146R) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDSAVYFCA*RGYVDYGGATDL*WG QGTLV**R**VSS |
| 97 | **VH** 37-20-B03-SC09.1_(T101Q, T146Q) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDQAVYFCA*RGYVDYGGATDL*W GQGTLV**Q**VSS |
| 98 | **VH** 37-20-B03-sc09.1_(T101K, T146Q) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDKAVYFCA*RGYVDYGGATDL*W GQGTLV**Q**VSS |
| 99 | **VH** 37-20-B03-sc09.1_(T101N, T146Q) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDNAVYFCA*RGYVDYGGATDL*W GQGTLV**Q**VSS |
| 100 | **VH** 37-20-B03-sc09.1_(T101S, T146K) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDSAVYFCA*RGYVDYGGATDL*WG QGTLV**K**VSS |
| 101 | **VH** 37-20-B03-sc09.1_(T101S, T146S) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDSAVYFCA*RGYVDYGGATDL*WG QGTLV**S**VSS |
| 102 | **VH** 37-20-B03-sc09.1_(T101K, T146D) | EVQLVESGGGLVQPGGSLRLSCAAS*GFSFNSDYWIY*WVRQAPGKGLEWIA*SIYGGSS GNTQYASWAQG*RFTISRDNSKNTVYLQMNSLRAEDKAVYFCA*RGYVDYGGATDL*W GQGTLV**D**VSS |

EP 4 273 162 A1

(continued)

**Original (unmodified) PRO2230**

| SEQ ID NO: | Description: | Sequence: |
|---|---|---|
| 103 | VH 37-20-B03-sc09.1_(T101R, T146E) | EVQLVESGGGLVQPGGSLRLSCAAS*GFSFNSDYWIY*WVRQAPGKGLEWIA*SIYGGSSGNTQYASWAQ*GRFTISRDNSKNTVYLQMNSLRAEDRAVYFCA*RGYVDYGGATDL*WGQGTLVEVSS |
| 104 | VH 37-20-B03-sc09.1_(L144K, S148L) | EVQLVESGGGLVQPGGSLRLSCAAS*GFSFNSDYWIY*WVRQAPGKGLEWIA*SIYGGSSGNTQYASWAQ*GRFTISRDNSKNTVYLQMNSLRAEDTAVYFCA*RGYVDYGGATDL*WGQGTKVTVLS |
| 105 | VH 37-20-B03-sc09.1_(L144A, S148N) | EVQLVESGGGLVQPGGSLRLSCAAS*GFSFNSDYWIY*WVRQAPGKGLEWIA*SIYGGSSGNTQYASWAQ*GRFTISRDNSKNTVYLQMNSLRAEDTAVYFCA*RGYVDYGGATDL*WGQGTAVTVNS |
| 106 | VH 37-20-B03-sc09.1_(L144K, T146E, S148K) | EVQLVESGGGLVQPGGSLRLSCAAS*GFSFNSDYWIY*WVRQAPGKGLEWIA*SIYGGSSGNTQYASWAQ*GRFTISRDNSKNTVYLQMNSLRAEDTAVYFCA*RGYVDYGGATDL*WGQGTKVEVKS |
| 107 | VH 37-20-B03-SC09.1_(T101S, L144A, T146Q) | EVQLVESGGGLVQPGGSLRLSCAAS*GFSFNSDYWIY*WVRQAPGKGLEWIA*SIYGGSSGNTQYASWAQ*GRFTISRDNSKNTVYLQMNSLRAEDSAVYFCA*RGYVDYGGATDL*WGQGTAVQVSS |
| 108 | VH 37-20-B03-sc09.1_(L12A, T101S, L144A, T146Q) | EVQLVESGGGAVQPGGSLRLSCAAS*GFSFNSDYWIY*WVRQAPGKGLEWIA*SIYGGSSGNTQYASWAQ*GRFTISRDNSKNTVYLQMNSLRAEDSAVYFCA*RGYVDYGGATDL*WGQGTAVQVSS |
| 109 | VH 37-20-B03-sc09.1_(L12A, T101R, L144A, T146Q) | EVQLVESGGGAVQPGGSLRLSCAAS*GFSFNSDYWIY*WVRQAPGKGLEWIA*SIYGGSSGNTQYASWAQ*GRFTISRDNSKNTVYLQMNSLRAEDRAVYFCA*RGYVDYGGATDL*WGGQGTAVQVSS |
| 110 | VH 37-20-B03-sc09.1_(L12A, T101S, L144A, T146R) | EVQLVESGGGAVQPGGSLRLSCAAS*GFSFNSDYWIY*WVRQAPGKGLEWIA*SIYGGSSGNTQYASWAQ*GRFTISRDNSKNTVYLQMNSLRAEDSAVYFCA*RGYVDYGGATDL*WGQGTAVRVSS |

(continued)

EP 4 273 162 A1

**Original (unmodified) PRO2230**

| SEQ ID NO: | Description: | Sequence: |
|---|---|---|
| 111 | **VH** 37-20-B03-sc09.1_(L12A, T101S, L144K, T146Q) | EVQLVESGGGAVQPGGSLRLSCAAS*GFSFNSDYWIY*WVRQAPGKGLEWIA*SIYGGSS GNTQYASWAQG*RFTISRDNSKNTVYLQMNSLRAEDSAVYFCA*RGYVDYGGATDL*WG QGTK**V**QVSS |
| 112 | **VH** 37-20-B03-sc09.1_(L12R, T101S, L144A, T146Q) | EVQLVESGGGRVQPGGSLRLSCAAS*GFSFNSDYWIY*WVRQAPGKGLEWIA*SIYGGSS GNTQYASWAQG*RFTISRDNSKNTVYLQMNSLRAEDSAVYFCA*RGYVDYGGATDL*WG QGTA**V**QVSS |
| 113 | **VH** 37-20-B03-sc09.1_(L12R, T101R, L144A, T146Q) | EVQLVESGGGRVQPGGSLRLSCAAS*GFSFNSDYWIY*WVRQAPGKGLEWIA*SIYGGSS GNTQYASWAQG*RFTISRDNSKNTVYLQMNSLRAEDRAVYFCA*RGYVDYGGATDL*W GQGTA**V**QVSS |
| 114 | VH 37-20-B03-sc09.1_(L12A, T101Q, L144A, T146Q) | EVQLVESGGGAVQPGGSLRLSCAAS*GFSFNSDYWIY*WVRQAPGKGLEWIA*SIYGGSS GNTQYASWAQG*RFTISRDNSKNTVYLQMNSLRAEDQAVYFCA*RGYVDYGGATDL*W GQGTA**V**QVSS |
| 115 | **VL** 37-20-B03-sc09.1_(F101E) | DIQMTQSPASLSASVGDRVTITC*QASQSIGTYLA*WYQQKPGKPPKLLIY*RAFILAS*GVP SRFSGSGSGTDFTLTISSLQPEDEATYYC*QSNFYSDSTTIGPNA*FGTGTKVTVLG |
| 116 | **VL** 37-20-B03-sc09.1_(F101R) | DIQMTQSPASLSASVGDRVTITC*QASQSIGTYLA*WYQQKPGKPPKLLIY*RAFILAS*GVP SRFSGSGSGTDFTLTISSLQPEDRATYYC*QSNFYSDSTTIGPNA*FGTGTKVTVLG |
| 117 | **VL** 37-20-B03-sc09.1_(F101Q) | DIQMTQSPASLSASVGDRVTITC*QASQSIGTYLA*WYQQKPGKPPKLLIY*RAFILAS*GVP SRFSGSGSGTDFTLTISSLQPEDQATYYC*QSNFYSDSTTIGPNA*FGTGTKVTVLG |
| **Reference / comparative PRO2230 variants (not according to the invention)** | | |
| 118 | **VH** 37-20-B03-sc09.1_(L12R, V103T, L144Q) | EVQLVESGGGRVQPGGSLRLSCAAS*GFSFNSDYWIY*WVRQAPGKGLEWIA*SIYGGSS GNTQYASWAQG*RFTISRDNSKNTVYLQMNSLRAEDTATYFCA*RGYVDYGGATDL*WG QGT**Q**VTVSS |
| 119 | **VH** 37-20-B03-sc09.1_(L12R, G51C, V103T, L144Q) | EVQLVESGGGRVQPGGSLRLSCAAS*GFSFNSDYWIY*WVRQAPGKCLEWIA*SIYGGSS GNTQYASWAQG*RFTISRDNSKNTVYLQMNSLRAEDTATYFCA*RGYVDYGGATDL*WG QGT**Q**VTVSS |

| Reference / comparative PRO2230 variants (not according to the invention) | | |
|---|---|---|
| 120 | **VH** 37-20-B03-sc09.1_(L12S, V103T, L144T) | EVQLVESGGG**S**VQPGGSLRLSCAAS***GFSFNSDYWIY***WVRQAPGKGLEWIA***SIYGGSS GNTQYASWAQG***RFTISRDNSKNTVYLQMNSLRAEDTATYFCA***RGYVDYGGATDL***WG QGTTVTVSS |

**Table 4. Examples of scFv sequences of PRO2230 variants according to the present invention and scFv sequences of original (unmodified) PRO2230 and reference / comparative PRO2230 variants (modifications relative to original PRO2230 and linker residues, if present, are shown in bold).**

| Original (unmodified) PRO2230 | | |
|---|---|---|
| **SEQ ID NO:** | **Description:** | **Sequence:** |
| 121 | **scFv** 37-20-B03-sc09.1 PRO2230 | DIQMTQSPASLSASVGDRVTITC***QASQSIGTYLA***WYQQKPGKPPKLLIY***RAFILAS***GVPSRFSGSGSGTDFTLTISSLQPEDFATYYC***QSNFYSDSTTIGPNA***FGTGTKVTVLG**GGGGSGGGGSGGGGSGGGGS**EVQLVESGGGLVQPGGSLRLSCAAS***GFSFNSDYWIY***WVRQAPGKGLEWIA***SIYGGSSGNTQYASWAQG***RFTISRDNSKNTVYLQMNSLRAEDTAVYFCA***RGYVDYGGATDL***WGQGTLVTVSS |
| 122 | **scFv** 37-20-B03-sc09.1_ (VL_G141C, VH_G51C) | DIQMTQSPASLSASVGDRVTITC***QASQSIGTYLA***WYQQKPGKPPKLLIY***RAFILAS***GVPSRFSGSGSGTDFTLTISSLQPEDFATYYC***QSNFYSDSTTIGPNA***FG**C**GTKVTVLG**GGGGSGGGGSGGGGSGGGGS**EVQLVESGGGLVQPGGSLRLSCAAS***GFSFNSDYWIY***WVRQAPGK**C**LEWIA***SIYGGSSGNTQYASWAQG***RFTISRDNSKNTVYLQMNSLRAEDTAVYFCA***RGYVDYGGATDL***WGQGTLVTVSS |

| PRO2230 variants (according to the invention) | | |
|---|---|---|
| **SEQ ID NO:** | **Description:** | **Sequence:** |
| 123 | **scFv** 37-20-B03-sc09.1_ (P15A) PRO2987 | DIQMTQSPASLSASVGDRVTITC***QASQSIGTYLA***WYQQKPGKPPKLLIY***RAFILAS***GVPSRFSGSGSGTDFTLTISSLQPEDFATYYC***QSNFYSDSTTIGPNA***FGTGTKVTVLG**GGGGSGGGGSGGGGSGGGGS**EVQLVESGGGLVQ**A**GGSLRLSCAAS***GFSFNSDYWIY***WVRQAPGKGLEWIA***SIYGGSSGNTQYASWAQG***RFTISRDNSKNTVYLQMNSLRAEDTAVYFCA***RGYVDYGGATDL***WGQGTLVTVSS |
| 124 | **scFv** 37-20-B03-sc09.1_ (P48A) PRO2988 | DIQMTQSPASLSASVGDRVTITC***QASQSIGTYLA***WYQQKPGKPPKLLIY***RAFILAS***GVPSRFSGSGSGTDFTLTISSLQPEDFATYYC***QSNFYSDSTTIGPNA***FGTGTKVTVLG**GGGGSGGGGSGGGGSGGGGS**EVQLVESGGGLVQPGGSLRLSCAAS***GFSFNSDYWIY***WVRQA**A**GKGLEWIA***SIYGGSSGNTQYASWAQG***RFTISRDNSKNTVYLQMNSLRAEDTAVYFCA***RGYVDYGGATDL***WGQGTLVTVSS |
| 125 | **scFv** 37-20-B03-SC09.1_ (T101S) PRO2952 | DIQMTQSPASLSASVGDRVTITC***QASQSIGTYLA***WYQQKPGKPPKLLIY***RAFILAS***GVPSRFSGSGSGTDFTLTISSLQPEDFATYYC***QSNFYSDSTTIGPNA***FGTGTKVTVLG**GGGGSGGGGSGGGGSGGGGS**EVQLVESGGGLVQPGGSLRLSCAAS***GFSFNSDYWIY***WVRQAPGKGLEWIA***SIYGGSSGNTQYASWAQG***RFTISRDNSKNTVYLQMNSLRAED**S**AVYFCA***RGYVDYGGATDL***WGQGTLVTVSS |

EP 4 273 162 A1

47

(continued)

**PRO2230 variants (according to the invention)**

| SEQ ID NO: | Description: | Sequence: |
|---|---|---|
| 126 | scFv 37-20-B03-sc09.1_ (T101A) PRO2985 | DIQMTQSPASLSASVGDRVTITC*QASQSIGTYLA*WYQQKPGKPPKLLIY***RAFILA**SGVPSRFSGSGSGTDFTLTISSLQPEDFATYYC**QSNFYSDSTTIGPNA**FGTGTKVTVLGGGGGSGGGGSGGGGSGGGGSEVQLVESGGGLVQPGGSLRLSCAAS**GFSFNSDYWIY**WVRQAPGKGLEWIAS*IYGGSSGNTQYASWA*QGRFTISRDNSKNTVYLQMNSLRAEDAAVYFCAR***GYVDYGGATDL***WGQGTLVTVSS |
| 127 | scFv 37-20-B03-sc09.1_ (T101R) PRO2951 | DIQMTQSPASLSASVGDRVTITC*QASQSIGTYLA*WYQQKPGKPPKLLIY***RAFILA**SGVPSRFSGSGSGTDFTLTISSLQPEDFATYYC**QSNFYSDSTTIGPNA**FGTGTKVTVLGGGGGSGGGGSGGGGSGGGGSEVQLVESGGGLVQPGGSLRLSCAAS**GFSFNSDYWIY**WVRQAPGKGLEWIAS*IYGGSSGNTQYASWA*QGRFTISRDNSKNTVYLQMNSLRAEDRAVYFCAR***GYVDYGGATDL***WGQGTLVTVSS |
| 128 | scFv 37-20-B03-sc09.1_ (T101Q) PRO3317 | DIQMTQSPASLSASVGDRVTITC*QASQSIGTYLA*WYQQKPGKPPKLLIY***RAFILA**SGVPSRFSGSGSGTDFTLTISSLQPEDFATYYC**QSNFYSDSTTIGPNA**FGTGTKVTVLGGGGGSGGGGSGGGGSGGGGSEVQLVESGGGLVQPGGSLRLSCAAS**GFSFNSDYWIY**WVRQAPGKGLEWIAS*IYGGSSGNTQYASWA*QGRFTISRDNSKNTVYLQMNSLRAEDQAVYFCAR***GYVDYGGATDL***WGQGTLVTVSS |
| 129 | scFv 37-20-B03-sc09.1_ (T146A) PRO2986 | DIQMTQSPASLSASVGDRVTITC*QASQSIGTYLA*WYQQKPGKPPKLLIY***RAFILA**SGVPSRFSGSGSGTDFTLTISSLQPEDFATYYC**QSNFYSDSTTIGPNA**FGTGTKVTVLGGGGGSGGGGSGGGGSGGGGSEVQLVESGGGLVQPGGSLRLSCAAS**GFSFNSDYWIY**WVRQAPGKGLEWIAS*IYGGSSGNTQYASWA*QGRFTISRDNSKNTVYLQMNSLRAEDTAVYFCAR***GYVDYGGATDL***WGQGTLVAVSS |
| 130 | scFv 37-20-B03-sc09.1_ (T146K) PRO2947 | DIQMTQSPASLSASVGDRVTITC*QASQSIGTYLA*WYQQKPGKPPKLLIY***RAFILA**SGVPSRFSGSGSGTDFTLTISSLQPEDFATYYC**QSNFYSDSTTIGPNA**FGTGTKVTVLGGGGGSGGGGSGGGGSGGGGSEVQLVESGGGLVQPGGSLRLSCAAS**GFSFNSDYWIY**WVRQAPGKGLEWIAS*IYGGSSGNTQYASWA*QGRFTISRDNSKNTVYLQMNSLRAEDTAVYFCAR***GYVDYGGATDL***WGQGTLVKVSS |

(continued)

| SEQ ID NO: | Description: | Sequence: |
|---|---|---|
| PRO2230 variants (according to the invention) | | |
| 131 | **scFv** 37-20-B03-sc09.1_ (T146R) PRO2948 | DIQMTQSPASLSASVGDRVTITC***QASQSIGTYLA***WYQQKPGKPPKLLIY***RAFILAS***GVP SRFSGSGSGTDFTLTISSLQPEDFATYYC***QSNFYSDSTTIGPNA***FGTGTKVTVLG**GGG GSGGGGSGGGGSGGGGS**EVQLVESGGGLVQPGGSLRLSCAAS***GFSFNSDYWIY***WV RQAPGKGLEWIA***SIYGGSSGNTQYASWAQG***RFTISRDNSKNTVYLQMNSLRAEDTAV YFCA***RGYVDYGGATDL***WGQGTLV**R**VSS |
| 132 | **scFv** 37-20-B03-sc09.1_ (T146E) PRO2949 | DIQMTQSPASLSASVGDRVTITC***QASQSIGTYLA***WYQQKPGKPPKLLIY***RAFILAS***GVP SRFSGSGSGTDFTLTISSLQPEDFATYYC***QSNFYSDSTTIGPNA***FGTGTKVTVLG**GGG GSGGGGSGGGGSGGGGS**EVQLVESGGGLVQPGGSLRLSCAAS***GFSFNSDYWIY***WV RQAPGKGLEWIA***SIYGGSSGNTQYASWAQG***RFTISRDNSKNTVYLQMNSLRAEDTAV YFCA***RGYVDYGGATDL***WGQGTLV**E**VSS |
| 133 | **scFv** 37-20-B03-sc09.1_ (T146Q) PRO2950 | DIQMTQSPASLSASVGDRVTITC***QASQSIGTYLA***WYQQKPGKPPKLLIY***RAFILAS***GVP SRFSGSGSGTDFTLTISSLQPEDFATYYC***QSNFYSDSTTIGPNA***FGTGTKVTVLG**GGG GSGGGGSGGGGSGGGGS**EVQLVESGGGLVQPGGSLRLSCAAS***GFSFNSDYWIY***WV RQAPGKGLEWIA***SIYGGSSGNTQYASWAQG***RFTISRDNSKNTVYLQMNSLRAEDTAV YFCA***RGYVDYGGATDL***WGQGTLV**Q**VSS |
| 134 | **scFv** 37-20-B03-sc09.1_ (T101S, T146Q) PRO2913 | DIQMTQSPASLSASVGDRVTITC***QASQSIGTYLA***WYQQKPGKPPKLLIY***RAFILAS***GVP SRFSGSGSGTDFTLTISSLQPEDFATYYC***QSNFYSDSTTIGPNA***FGTGTKVTVLG**GGG GSGGGGSGGGGSGGGGS**EVQLVESGGGLVQPGGSLRLSCAAS***GFSFNSDYWIY***WV RQAPGKGLEWIA***SIYGGSSGNTQYASWAQG***RFTISRDNSKNTVYLQMNSLRAED**S**AV YFCA***RGYVDYGGATDL***WGQGTLV**Q**VSS |
| 135 | **scFv** 37-20-B03-sc09.1_ (T101R, T146Q) PRO3312 | DIQMTQSPASLSASVGDRVTITC***QASQSIGTYLA***WYQQKPGKPPKLLIY***RAFILAS***GVP SRFSGSGSGTDFTLTISSLQPEDFATYYC***QSNFYSDSTTIGPNA***FGTGTKVTVLG**GGG GSGGGGSGGGGSGGGGS**EVQLVESGGGLVQPGGSLRLSCAAS***GFSFNSDYWIY***WV RQAPGKGLEWIA***SIYGGSSGNTQYASWAQG***RFTISRDNSKNTVYLQMNSLRAED**R**AV YFCA***RGYVDYGGATDL***WGQGTLV**Q**VSS |

| PRO2230 variants (according to the invention) | | |
|---|---|---|
| SEQ ID NO: | Description: | Sequence: |
| 136 | scFv 37-20-B03-SC09.1_ (T101S, T146R) PRO3313 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGLVQPGGSLRLSCAAS*GFSFNSDYWIY*WVRQAPGKGLEWIA*SIYGGSSGNTQYASWAQG*RFTISRDNSKNTVYLQMNSLRAEDSAVYFCA*RGYVDYGGATDL*WGQGTLV**R**VSS |
| 137 | scFv 37-20-B03-SC09.1_ (T101Q, T146Q) PRO3314 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGLVQPGGSLRLSCAAS*GFSFNSDYWIY*WVRQAPGKGLEWIA*SIYGGSSGNTQYASWAQG*RFTISRDNSKNTVYLQMNSLRAED**Q**AVYFCA*RGYVDYGGATDL*WGQGTLV**Q**VSS |
| 138 | scFv 37-20-B03-sc09.1_ (T101K, T146Q) PRO3338 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGLVQPGGSLRLSCAAS*GFSFNSDYWIY*WVRQAPGKGLEWIA*SIYGGSSGNTQYASWAQG*RFTISRDNSKNTVYLQMNSLRAED**K**AVYFCA*RGYVDYGGATDL*WGQGTLV**Q**VSS |
| 139 | scFv 37-20-B03-sc09.1_ (T101N, T146Q) PRO3339 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGLVQPGGSLRLSCAAS*GFSFNSDYWIY*WVRQAPGKGLEWIA*SIYGGSSGNTQYASWAQG*RFTISRDNSKNTVYLQMNSLRAED**N**AVYFCA*RGYVDYGGATDL*WGQGTLV**Q**VSS |
| 140 | scFv 37-20-B03-sc09.1_ (T101S, T146K)<br><br>PRO3340 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGLVQPGGSLRLSCAAS*GFSFNSDYWIY*WVRQAPGKGLEWIA*SIYGGSSGNTQYASWAQG*RFTISRDNSKNTVYLQMNSLRAEDSAVYFCA*RGYVDYGGATDL*WGQGTLV**K**VSS |

**PRO2230 variants (according to the invention)**

| SEQ ID NO: | Description: | Sequence: |
|---|---|---|
| 141 | **scFv** 37-20-B03-sc09.1_ (T101S, T146S) PRO3341 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGLVQPGGSLRLSCAAS*GFSFNSDYWIY*WVRQAPGKGLEWIA*SIYGGSSGNTQYASWAQG*RFTISRDNSKNTVYLQMNSLRAEDSAVYFCA*RGYVDYGGATDL*WGQGTLVSVSS |
| 142 | **scFv** 37-20-B03-sc09.1_ (T101K, T146D) PRO3342 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGLVQPGGSLRLSCAAS*GFSFNSDYWIY*WVRQAPGKGLEWIA*SIYGGSSGNTQYASWAQG*RFTISRDNSKNTVYLQMNSLRAEDKAVYFCA*RGYVDYGGATDL*WGQGTLVDVSS |
| 143 | **scFv** 37-20-B03-sc09.1_ (T101R, T146E) PRO3343 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGLVQPGGSLRLSCAAS*GFSFNSDYWIY*WVRQAPGKGLEWIA*SIYGGSSGNTQYASWAQG*RFTISRDNSKNTVYLQMNSLRAEDRAVYFCA*RGYVDYGGATDL*WGQGTLVEVSS |
| 144 | **scFv** 37-20-B03-sc09.1_ (L144K, S148L) PRO2934 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGLVQPGGSLRLSCAAS*GFSFNSDYWIY*WVRQAPGKGLEWIA*SIYGGSSGNTQYASWAQG*RFTISRDNSKNTVYLQMNSLRAEDTAVYFCA*RGYVDYGGATDL*WGQGTKVTVLS |
| 145 | **scFv** 37-20-B03-sc09.1_ (L144A, S148N) PRO2935 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGLVQPGGSLRLSCAAS*GFSFNSDYWIY*WVRQAPGKGLEWIA*SIYGGSSGNTQYASWAQG*RFTISRDNSKNTVYLQMNSLRAEDTAVYFCA*RGYVDYGGATDL*WGQGTAVTVNS |

EP 4 273 162 A1

| PRO2230 variants (according to the invention) | | |
|---|---|---|
| SEQ ID NO: | Description: | Sequence: |
| 146 | **scFv** 37-20-B03-sc09.1_ (L144K, T146E, S148K) PRO2933 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGLVQPGGSLRLSCAAS*GFSFNSDYWIY*WVRQAPGKGLEWIA*SIYGGSSGNTQYASWAQG*RFTISRDNSKNTVYLQMNSLRAEDTAVYFCA*RGYVDYGGATDL*WGQGT**K**VEV**KS** |
| 147 | **scFv** 37-20-B03-sc09.1_ (T101S, L144A, T146Q)<br><br>PRO2914 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGLVQPGGSLRLSCAAS*GFSFNSDYWIY*WVRQAPGKGLEWIA*SIYGGSSGNTQYASWAQG*RFTISRDNSKNTVYLQMNSLRAED**S**AVYFCA*RGYVDYGGATDL*WGQGTA**VQ**VSS |
| 148 | **scFv** 37 -20-B03-sc09.1_(L 12A, T101S, L144A, T146Q) PRO2915 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGG**A**VQPGGSLRLSCAAS*GFSFNSDYWIY*WVRQAPGKGLEWIA*SIYGGSSGNTQYASWAQG*RFTISRDNSKNTVYLQMNSLRAED**S**AVYFCA*RGYVDYGGATDL*WGQGTA**VQ**VSS |
| 149 | **scFv** 37-20-B03-sc09.1_ (L12A, T101R, L144A, T146Q) PRO3306 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGG**A**VQPGGSLRLSCAAS*GFSFNSDYWIY*WVRQAPGKGLEWIA*SIYGGSSGNTQYASWAQG*RFTISRDNSKNTVYLQMNSLRAED**R**AVYFCA*RGYVDYGGATDL*WGQGTA**VQ**VSS |
| 150 | **scFv** 37-20-B03-sc09.1_ (L12A, T101S, L144A, T146R) PRO3307 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGG**A**VQPGGSLRLSCAAS*GFSFNSDYWIY*WVRQAPGKGLEWIA*SIYGGSSGNTQYASWAQG*RFTISRDNSKNTVYLQMNSLRAED**S**AVYFCA*RGYVDYGGATDL*WGQGTA**VR**VSS |

| PRO2230 variants (according to the invention) | | |
|---|---|---|
| **SEQ ID NO:** | **Description:** | **Sequence:** |
| 151 | **scFv** 37-20-B03-sc09.1_ (L12A, T101S, L144K, T146Q) PRO3308 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGAVQPGGSLRLSCAAS*GFSFNSDYWIY*WVRQAPGKGLEWIA*SIYGGSSGNTQYASWAQG*RFTISRDNSKNTVYLQMNSLRAEDSAVYFCA*RGYVDYGGATDL*WGQGT**K**VQVSS |
| 152 | **scFv** 37-20-B03-sc09.1_ (L12R, T101S, L144A, T146Q) PRO3309 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGRVQPGGSLRLSCAAS*GFSFNSDYWIY*WVRQAPGKGLEWIA*SIYGGSSGNTQYASWAQG*RFTISRDNSKNTVYLQMNSLRAEDSAVYFCA*RGYVDYGGATDL*WGQGTAVQVSS |
| 153 | **scFv** 37-20-B03-sc09.1_ (L12R, T101R, L144A, T146Q) PRO3310 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGRVQPGGSLRLSCAAS*GFSFNSDYWIY*WVRQAPGKGLEWIA*SIYGGSSGNTQYASWAQG*RFTISRDNSKNTVYLQMNSLRAED**R**AVYFCA*RGYVDYGGATDL*WGQGTAVQVSS |
| 154 | **scFv** 37-20-B03-sc09.1_ (L12A, T101Q, L144A, T146Q) | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGAVQPGGSLRLSCAAS*GFSFNSDYWIY*WVRQAPGKGLEWIA*SIYGGSSGNTQYASWAQG*RFTISRDNSKNTVYLQMNSLRAED**Q**AVYFCA*RGYVDYGGATDL*WGQGTAVQVSS |
| **Reference/comparative PRO2230 variamts (not according to the invention)** | | |
| **SEQ ID NO:** | **Description:** | **Sequence:** |
| 155 | **scFv** 37-20-B03-sc09.1_ (L12R, V103T, L144Q) PRO2903 | DIQMTQSPASLSASVGDRVTITC*QASQSIGTYLA*WYQQKPGKPPKLLIY*RAFILAS*GVPSRFSGSGSGTDFTLTISSLQPEDFATYYC*QSNFYSDSTTIGPNA*FGTGTKVTVLG**GGGGSGGGGSGGGGSGGGGS**EVQLVESGGGRVQPGGSLRLSCAAS*GFSFNSDYWIY*WVRQAPGKGLEWIA*SIYGGSSGNTQYASWAQG*RFTISRDNSKNTVYLQMNSLRAEDTA**T**YFCA*RGYVDYGGATDL*WGQGT**Q**VTVSS |

EP 4 273 162 A1

| Reference/comparative PRO2230 variamts (not according to the invention) | | |
|---|---|---|
| **SEQ ID NO:** | **Description:** | **Sequence:** |
| 156 | **scFv** 37-20-B03-sc09.1_ (L12S, V103T, L144T) PRO2984 | DIQMTQSPASLSASVGDRVTITC***QASQSIGTYLA***WYQQKPGKPPKLLIY***RAFILAS***GVP SRFSGSGSGTDFTLTISSLQPEDFATYYC***QSNFYSDSTTIGPNA***FGTGTKVTVLG**GGG GSGGGGSGGGGSGGGGS**EVQLVESGGGSVQPGGSLRLSCAAS***GFSFNSDYWIY***WV RQAPGKGLEWIA***SIYGGSSGNTQYASWAQG***RFTISRDNSKNTVYLQMNSLRAEDTA**T** YFCA***RGYVDYGGATDL***WGQGTTVTVSS |

**Table 5. Other sequences related to the present invention.**

| SEQ ID NO: | Description | Sequence |
|---|---|---|
| 157 | VH3 | EVQLVESGGGLVQPGGSLRLSCAAS*GFSFSANYYPC*WVRQAPGKGLEWIG*CIYGGS SDITYDANWTKG*RFTISRDNSKNTVYLQMNSLRAEDTAVYYCA*RSAWYSGWGGDL*W GQGTLVTVSS |
| 158 | VH3_(51C) | EVQLVESGGGLVQPGGSLRLSCAAS*GFSFSANYYPC*WVRQAPGKCLEWIG*CIYGGS SDITYDANWTKG*RFTISRDNSKNTVYLQMNSLRAEDTAVYYCA*RSAWYSGWGGDL*W GQGTLVTVSS |
| 159 | VH3_(56A) | EVQLVESGGGLVQPGGSLRLSCAAS*GFSFSANYYPC*WVRQAPGKGLEWIA*CIYGGS SDITYDANWTKG*RFTISRDNSKNTVYLQMNSLRAEDTAVYYCA*RSAWYSGWGGDL*W GQGTLVTVSS |
| 160 | VH3_(51C, 56A) | EVQLVESGGGLVQPGGSLRLSCAAS*GFSFSANYYPC*WVRQAPGKCLEWIA*CIYGGSS DITYDANWTKG*RFTISRDNSKNTVYLQMNSLRAEDTAVYYCA*RSAWYSGWGGDL*WG QGTLVTVSS |
| 161 | VH3_(105F) | EVQLVESGGGLVQPGGSLRLSCAAS*GFSFSANYYPC*WVRQAPGKGLEWIG*CIYGGS SDITYDANWTKG*RFTISRDNSKNTVYLQMNSLRAEDTAVYFCA*RSAWYSGWGGDL*W GQGTLVTVSS |
| 162 | VH3_(51C, 105F) | EVQLVESGGGLVQPGGSLRLSCAAS*GFSFSANYYPC*WVRQAPGKCLEWIG*CIYGGS SDITYDANWTKG*RFTISRDNSKNTVYLQMNSLRAEDTAVYFCA*RSAWYSGWGGDL*W GQGTLVTVSS |
| 163 | VH3_(89L) | EVQLVESGGGLVQPGGSLRLSCAAS*GFSFSANYYPC*WVRQAPGKGLEWIG*CIYGGS SDITYDANWTKG*RFTISRDNSKNTLYLQMNSLRAEDTAVYYCA*RSAWYSGWGGDL*W GQGTLVTVSS |
| 164 | VH3_(51C, 89L) | EVQLVESGGGLVQPGGSLRLSCAAS*GFSFSANYYPC*WVRQAPGKCLEWIG*CIYGGS SDITYDANWTKG*RFTISRDNSKNTLYLQMNSLRAEDTAVYYCA*RSAWYSGWGGDL*W GQGTLVTVSS |
| 165 | VH3_(56A, 89L) | EVQLVESGGGLVQPGGSLRLSCAAS*GFSFSANYYPC*WVRQAPGKGLEWIA*CIYGGS SDITYDANWTKG*RFTISRDNSKNTLYLQMNSLRAEDTAVYYCA*RSAWYSGWGGDL*W GQGTLVTVSS |

| SEQ ID NO: | Description | Sequence |
|---|---|---|
| 166 | VH3_(51C, 56A, 89L) | EVQLVESGGGLVQPGGSLRLSCAAS*GFSFSANYYPC*WVRQAPGKCLEWIA*CIYGGSS DITYDANWTKG*RFTISRDNSKNTLYLQMNSLRAEDTAVYYCA*RSAWYSGWGGDL*WG QGTLVTVSS |
| 167 | VH3_(56A, 105F) | EVQLVESGGGLVQPGGSLRLSCAAS*GFSFSANYYPC*WVRQAPGKGLEWIA*CIYGGS SDITYDANWTKG*RFTISRDNSKNTVYLQMNSLRAEDTAVYFCA*RSAWYSGWGGDL*W GQGTLVTVSS |
| 168 | VH3_(51C, 56A, 105F) | EVQLVESGGGLVQPGGSLRLSCAAS*GFSFSANYYPC*WVRQAPGKCLEWIA*CIYGGSS DITYDANWTKG*RFTISRDNSKNTVYLQMNSLRAEDTAVYFCA*RSAWYSGWGGDL*WG QGTLVTVSS |
| 169 | VH3_(56A, 89L, 105F) | EVQLVESGGGLVQPGGSLRLSCAAS*GFSFSANYYPC*WVRQAPGKGLEWIA*CIYGGS SDITYDANWTKG*RFTISRDNSKNTLYLQMNSLRAEDTAVYFCA*RSAWYSGWGGDL*W GQGTLVTVSS |
| 170 | VH3_(51C, 56A, 89L, 105F) | EVQLVESGGGLVQPGGSLRLSCAAS*GFSFSANYYPC*WVRQAPGKCLEWIA*CIYGGSS DITYDANWTKG*RFTISRDNSKNTLYLQMNSLRAEDTAVYFCA*RSAWYSGWGGDL*WG QGTLVTVSS |
| 171 | VH1a | QVQLVQSGAEVKKPGSSVKVSCKAS*GIDFNSNYYMC*WVRQAPGQGLEWMG*CIYVGS HVNTYYANWAKG*RVTITADESTSTAYMELSSLRSEDTAVYYCA*TSGSSVLYFKF*WGQ GTLVTVSS |
| 172 | VH1b | QVQLVQSGAEVKKPGASVKVSCKAS*GIDFNSNYYMC*WVRQAPGQGLEWMG*CIYVGS HVNTYYANWAKG*RVTMTRDTSISTAYMELSSLRSEDTAVYYCA*TSGSSVLYFKF*WGQ GTLVTVSS |
| 173 | VH4 | QVQLQESGPGLVKPSETLSLTCTVS*GIDFNSNYYMC*WIRQPPGKGLEWIG*CIYVGSHV NTYYANWAKG*RVTISVDTSKNQFSLKLSSVTAADTAVYYCA*TSGSSVLYFKF*WGQGT LVTVSS |
| 174 | VH5 | EVQLVQSGAEVKKPGESLKISCKGS*GIDFNSNYYM*CWVRQMPGKGLEWMG*CIYVGS HVNTYYANWAKG*QVTISADKSISTAYLQWSSLKASDTAMYYCA*TSGSSVLYFKF*WGQ GTLVTVSS |

| SEQ ID NO: | Description | Sequence |
|---|---|---|
| 175 | VH6 | QVQLQQSGPGLVKPSQTLSLTCAIS*GIDFNSNYYM*CWIRQSPGRGLEWLG*CIYVGSHV NTYYANWAKG*RITINPDTSKNQFSLQLNSVTPEDTAVYYCA*TSGSSVLYFKF*WGQGT LVTVSS |
| 176 | Vkappa1_V1 (L24 is defined here to belong to LFW1) | DIQMTQSPSSLSASVGDRVTITCQ*ASQSINNVLA*WYQQKPGKAPKLLIY*RASTLAS*GV PSRFSGSGSGTDFTLTISSLQPEDFATYYC*QSSYGNYGD*FGQGTKVEIKR |
| 177 | Vkappa1_V1_(Q24R) (L24 is defined here to belong to LFW1) | DIQMTQSPSSLSASVGDRVTITC**R**ASQSINNVLA*WYQQKPGKAPKLLIY*RASTLAS*GV PSRFSGSGSGTDFTLTISSLQPEDFATYYC*QSSYGNYGD*FGQGTKVEIKR |
| 178 | Vkappa1_V2 (L24 is defined here to belong to LFW1) | DIQMTQSPASLSASVGDRVTITCQ*ASQSIGTYLA*WYQQKPGKPPKLLIY*RAFILAS*GVP SRFSGSGSGTDFTLTISSLQPEDFATYYC*QSNFYSDSTTIGPNA*FGQGTKVEIKR |
| 179 | Vkappa1_V2_(Q24R) (L24 is defined here to belong to LFW1) | DIQMTQSPASLSASVGDRVTITC**R**ASQSIGTYLA*WYQQKPGKPPKLLIY*RAFILAS*GVP SRFSGSGSGTDFTLTISSLQPEDFATYYC*QSNFYSDSTTIGPNA*FGQGTKVEIKR |
| 180 | Vkappa1-V1_λ-LFW4 (L24 is defined here to belong to LFW1) | DIQMTQSPSSLSASVGDRVTITCQ*ASQSINNVLA*WYQQKPGKAPKLLIY*RASTLAS*GV PSRFSGSGSGTDFTLTISSLQPEDFATYYC*QSSYGNYGD*FGTGTKVTVLG |
| 181 | Vkappa1_V1_λ-LFW4_(141C) (L24 is defined here to belong to LFW1) | DIQMTQSPSSLSASVGDRVTITCQ*ASQSINNVLA*WYQQKPGKAPKLLIY*RASTLAS*GV PSRFSGSGSGTDFTLTISSLQPEDFATYYC*QSSYGNYGD*FGCGTKVTVLG |
| 182 | Vkappa1_V1_λ-LFW4_(Q24R) (L24 is defined here to belong to LFW1) | DIQMTQSPSSLSASVGDRVTITC**R**ASQSINNVLA*WYQQKPGKAPKLLIY*RASTLAS*GV PSRFSGSGSGTDFTLTISSLQPEDFATYYC*QSSYGNYGD*FGTGTKVTVLG |
| 183 | Vkappa1_V1_λ-LFW4_(Q24R, 141C) (L24 is defined here to belong to LFW1) | DIQMTQSPSSLSASVGDRVTITC**R**ASQSINNVLA*WYQQKPGKAPKLLIY*RASTLAS*GV PSRFSGSGSGTDFTLTISSLQPEDFATYYC*QSSYGNYGD*FGCGTKVTVLG |
| 184 (= 81) | Vkappa1_V2_λ-LFW4 (L24 is defined here to belong to LFW1) | DIQMTQSPASLSASVGDRVTITCQ*ASQSIGTYLA*WYQQKPGKPPKLLIY*RAFILAS*GVP SRFSGSGSGTDFTLTISSLQPEDFATYYC*QSNFYSDSTTIGPNA*FGTGTKVTVLG |
| 185 (= 82) | Vkappa1_V2_λ-LFW4_(141C) (L24 is defined here to belong to LFW1) | DIQMTQSPASLSASVGDRVTITCQ*ASQSIGTYLA*WYQQKPGKPPKLLIY*RAFILAS*GVP SRFSGSGSGTDFTLTISSLQPEDFATYYC*QSNFYSDSTTIGPNA*FGCGTKVTVLG |
| 186 | Vkappa1_V2_λ-LFW4_(Q24R) (L24 is defined here to belong to LFW1) | DIQMTQSPASLSASVGDRVTITC**R**ASQSIGTYLA*WYQQKPGKPPKLLIY*RAFILAS*GVP SRFSGSGSGTDFTLTISSLQPEDFATYYC*QSNFYSDSTTIGPNA*FGTGTKVTVLG |

| SEQ ID NO: | Description | Sequence |
|---|---|---|
| 187 | Vkappa1_V2_λ-LFW4_(Q24R, 141C) (L24 is defined here to belong to LFW1) | DIQMTQSPASLSASVGDRVTITC*RASQSIGTYLA*WYQQKPGKPPKLLIY*RAFILAS*GVP SRFSGSGSGTDFTLTISSLQPEDFATYYC*QSNFYSDSTTIGPNA*FGCGTKVTVLG |
| 188 | Vλ germline-based FR4 (Sk17) | FGTGTKVTVLG |
| 189 | Vλ germline-based FR4 (Sk12) | FGGGTKLTVLG |
| 190 | Vλ germline-based FR4 | FGGGTQLIILG |
| 191 | Vλ germline-based FR4 | FGEGTELTVLG |
| 192 | Vλ germline-based FR4 | FGSGTKVTVLG |
| 193 | Vλ germline-based FR4 | FGGGTQLTVLG |
| 194 | Vλ germline-based FR4 | FGGGTQLTALG |
| 195 | Vλ germline-based FR4 G141C | FGCGTKVTVLG |
| 196 | Vλ germline-based FR4_G141T | FGTGTKLTVLG |

Throughout the text of this application, should there be a discrepancy between the text of the specification (e. g., Tables 1 to 5) and the sequence listing, the text of the specification shall prevail.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all subcombinations of the various embodiments and elements thereof are also specifically embraced by the present invention and are disclosed herein just as if each and every such sub-combination was individually and explicitly disclosed herein.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

To the extent possible under the respective patent law, all patents, applications, publications, test methods, literature, and other materials cited herein are hereby incorporated by reference.

The following Examples illustrates the invention described above, but is not, however, intended to limit the scope of the invention in any way. Other test models known as such to the person skilled in the pertinent art can also determine the beneficial effects of the claimed invention.

**Examples**

**Example 1: Manufacturing of scFv variants according to the present invention and reference scFvs**

**1.1. Manufacturing of variants of PRO1922 (MSLN binding scFv) and variants of PRO2230 (PDL1 binding scFv):**

PRO1922, PRO2230, the variants of PRO1922 and PRO2230 of the present invention, as well as the respective references have been produced following the methods described in detail in the patent applications WO 2019/072868 and WO/2021/239987.

Briefly, the expression of the variants of PRO1922 and PRO2230 and the respective references, as defined herein, was performed in CHO cells using the ExpiCHO Expression System (ThermoFisher). Expression was conducted according to manufactural instructions. Proteins were purified from clarified harvest by affinity chromatography using a tandem affinity chromatography setup (in-line purification via Protein A and Protein L). If necessary, variant scFvs were polished by SE-chromatography to a final monomeric content > 95 %. For quality control of the manufactured material, standard analytical methods such as SE-HPLC, $UV_{280}$ and SDS-PAGE were applied.

PRO1922, PRO2230, the variants of PRO1922 and PRO2230 of the present invention, as well as the respective references that were produced are summarized in Table 6.

**Table 6: Variants of the MSLN binding scFvs PRO1922 and the PD-L1 binding scFv PRO2230.**

| Mutations | MSLN binding scFv variants | | | PD-L1 binding scFv variants | | |
|---|---|---|---|---|---|---|
| | PRO Number | Experimental Annotation | Titer Post Capture [mg/L] | PRO Number | Experimental Annotation | Titer Post Capture [mg/L] |
| VVT | PRO1992 | wt | 255 | PRO2330 | wt | 112 |
| L12R-V103T-L144Q (Reference) | PRO2918 | A-1.7 | 327 | PRO2903 | B-1.7 | 151 |
| L12S-V103T-L144T (Reference) | PRO2990 | A-1.9 | 222 | PRO2984 | B-1.9 | 127 |
| L144K-S148L | PRO2931 | A-1.14 | 398 | PRO2934 | B-1.14 | 136 |
| L144A-S148N | PRO2932 | A-1.15 | 118 | PRO2935 | B-1.15 | 94 |
| L144K-T146E-S148K | PRO2930 | A-1.16 | 420 | PRO2933 | B-1.16 | 191 |
| T146A | PRO2992 | A-2.1 | 75 | PRO2986 | B-2.1 | 103 |
| T146E | PRO2943 | A-2.2 | 203 | PRO2949 | B-2.2 | 48 |

(continued)

| Mutations | MSLN binding scFv variants | | | PD-L1 binding scFv variants | | |
|---|---|---|---|---|---|---|
| | PRO Number | Experimental Annotation | Titer Post Capture [mg/L] | PRO Number | Experimental Annotation | Titer Post Capture [mg/L] |
| T146K | PRO2941 | A-2.3 | 301 | PRO2947 | B-2.3 | 100 |
| T146R | PRO2942 | A-2.4 | 313 | PRO2948 | B-2.4 | 37 |
| T146Q | PRO2944 | A-2.5 | 155 | PRO2950 | B-2.5 | 67 |
| T101A | PRO2991 | A-2.6 | 304 | PRO2985 | B-2.6 | 32 |
| T101R | PRO2945 | A-2.7 | 576 | PRO2951 | B-2.7 | 188 |
| T101S | PRO2946 | A-2.8 | 165 | PRO2952 | B-2.8 | 169 |
| T101S-T146Q | PRO2936 | A-2.9 | 706 | PRO2913 | B-2.9 | 176 |
| T101S-L144A-T146Q | PRO2937 | A-3.1 | 21 | PRO2914 | B-3.1 | 156 |
| L12A-T101S-L144A-T146Q | PRO2938 | A-3.2 | 652 | PRO2915 | B-3.2 | 91 |
| P15A | PRO2993 | A-4.1 | 192 | PRO2987 | B-4.1 | 49 |
| P48A | PRO2994 | A-4.2 | 43 | PRO2988 | B-4.2 | 121 |
| T101Q | PRO3329 | A-2-2.1 | 157.2 | PRO3317 | B-2-2.1 | 36.1 |
| T101R-T146Q | PRO3324 | A-2-2.2 | 118.3 | PRO3312 | B-2-2.2 | 18.1 |
| T101R-T146E | PRO3349 | A-2-2.3 | 157.5 | PRO3343 | B-2-2.3 | 176.6 |
| T101S-T146R | PRO3325 | A-2-2.4 | 172.6 | PRO3313 | B-2-2.4 | 22.4 |
| T101S-T146K | PRO3346 | A-2-2.5 | 112.1 | PRO3340 | B-2-2.5 | 85.5 |
| T101Q-T146Q | PRO3326 | A-2-2.6 | 173.1 | PRO3314 | B-2-2.6 | 52.8 |
| T101K-T146Q | PRO3344 | A-2-2.7 | 173.1 | PRO3338 | B-2-2.7 | 143.8 |
| T101N-T146Q | PRO3345 | A-2-2.8 | 146.3 | PRO3339 | B-2-2.8 | 153.5 |
| T101S-T146S | PRO3347 | A-2-2.9 | 140.7 | PRO3341 | B-2-2.9 | 139.4 |
| T101K-T146D | PRO3348 | A-2-2.10 | 187.8 | PRO3342 | B-2-2.10 | 183.4 |
| L12A-T101R-L144A-T146Q | PRO3318 | A-2-3.1 | 97.7 | PRO3306 | B-2-3.1 | 37.3 |
| L12A-T101S-L144A-T146R | PRO3319 | A-2-3.2 | 130.3 | PRO3307 | B-2-3.2 | 83 |
| L12A-T101S-L144K-T146Q | PRO3320 | A-2-3.3 | 111.2 | PRO3308 | B-2-3.3 | 96.4 |
| L12R-T101S-L144A-T146Q | PRO3321 | A-2-3.4 | 96.1 | PRO3309 | B-2-3.4 | 55.2 |
| L12R-T101R-L144A-T146Q | PRO3322 | A-2-3.5 | 164.1 | PRO3310 | B-2-3.5 | 68.4 |
| L12A-T101Q-L144A-T146Q | PRO3323 | A-2-3.6 | 87.9 | PRO3311 | B-2-3.6 | 120.6 |

Example 2: pre-existing ADA-binding assay

2.1. General assay procedure:

A method was developed at Numab to detect pre-existing anti-drug-antibodies in human serum, using a direct assay format.

96 well half-area plates were coated with 100 ng/ml of the test molecule (e. g. scFv molecules) for 2 hours at room temperature. The plates were blocked for 1 hour with PBS containing 0.2% Tween and 1% BSA. Individual human sera were then added at a dilution of 1:20 (5% serum) or 1:100 (1% serum), either unspiked (screening assay) or spiked (confirmatory assay) with the same molecule as coated in the corresponding well. The spiking concentration ranged from 60 to 115 nM and spiked samples were pre-incubated for 1 hour. Antibodies bound to the molecules coated on the plate where then detected with 100 ng/ml rabbit anti-human IgG-HRP for 1 hour. TMB substrate was added as substrate and after a short incubation, the enzymatic reaction was stopped with 1M HCl. The optical density of each well was read at 450nm.

All steps were performed at room temperature. Between each step, plates were washed three times with 450 $\mu$l wash buffer. Except for the blocking and washing steps, all assay components were added in a volume of 25 $\mu$l/well and duplicates were used. For the incubation steps, the ELISA plates were placed on a rotating mixer (40 rpm).

Generally, a first round of measurement was performed with unspiked human sera (screening assay). Then a screening cut-point (SCP) was calculated for each plate. Unspiked samples with signal below the SCP were termed "screening negative" and were not taken into account in the confirmatory assay. Unspiked samples with signal above the SCP were termed "screening positive".

With most of these "screening positive" samples, a second round of measurement was performed with spiked human sera (confirmatory assay), to determine whether the initially detected binding of antibodies in the respective sera sample is specific to the test molecule. A decrease of the absorbance signal in the spiked wells indicates that the signal observed in the unspiked well of the initial screening assay is specific to the molecule coated on the plate. The resulting percent inhibition (% inhibition) required to confirm specificity was either set at 20 to 30% or a confirmatory cut-point was calculated in percent (%CCP). In the latter case, only samples where percent inhibition was above the CCP were confirmed positive. % inhibition was calculated as reduction of the initial signal obtained for unspiked serum (screening assay) as follows: %CCP = initial signal (1-(spiked serum/signal unspiked serum))*100.

In an alternative procedure, the initial screening assay was not performed. Instead, the test samples were directly analysed using the confirmatory assay procedure. For data analysis however, the same calculations were performed, which at least involves the calculation of the SCP and the %CCP.

**2.2. Determination/calculation of Screening Cut Point (SCP), Normalization Factor (NF) and Floating Cut Point (FCP)**

For each test compound, 20 individual human serum samples of healthy untreated subjects were analyzed.

***Screening Cut-Point (SCP):***

The screening cut point (SCP) is the threshold at which a signal is considered positive (screening positive). It is calculated such that 5% false positive sera are included.

The screening cut point (SCP) is calculated as follows:

$$SCP = mean\ N + 1.645 \times SDN$$

Wherein:

- "Mean N" corresponds to mean signal from all unspiked individual sera measured for a specific test compound;
- "SDN" corresponds to standard deviation calculated from all unspiked individual sera measured for a specific test compound.

***Normalization factor (NF):***

The normalization factor (NF) is calculated as follow:

$$NF = SCP - Negative\ control\ mean$$

Wherein:

- "SCP" is as defined above; and
- "negative control mean" corresponds to mean signal from the negative controls (pooled individual human sera, same on each plate) analyzed in duplicates (*i.* e. 2 wells) per plate.

At least two NC samples (*i.* e. 4 wells) must be taken into account for the calculation of the normalization factor.

*Calculation of the Floating Cut Point (FCP):*

After the determination of the SCP and the NF, the Floating Cut Point (FCP) for each plate was used as the reference cut point. The FCP takes into account the analytical variability of each analytical run, by normalizing the SCP with the negative controls of the plate. The FCP is calculated for each analytical run as follows:

$$FCP = NF + Mean\ NC$$

Wherein:

- "Mean NC" refers to the mean signal of the negative control samples;
- "NF" refers to the normalization factor, as defined above.

### 2.3. Pre-existing ADA-binding assay results for PRO1922, PRO2230, the variants scFvs of PRO1922 and PRO2230 of the present invention, as well as the respective references

PRO1922 wt, PRO2230 wt; 32 PRO1922 scFv variants according to the present invention, 32 PRO2230 scFv variants according to the present invention, as well as the references PRO1922-L12R-V103T-L144Q (PRO2918), PRO1922-L12S-V103T-L144T (PRO2990), PRO2230-L12R-V103T-L144Q (PRO2903) PRO2230-L12S-V103T-L144T (PRO2984) have been analyzed. The measurements were directly performed in the confirmatory assay setup using 20 human serum samples.

The detailed assay procedure for PRO1922-based and PRO2230-based **scFvs** was as follows. The **scFvs** were diluted in PBS to a concentration of 100 ng/ml, applied in a volume of 25 μl onto a 96-well plate (Greiner, half area, high binding) and incubated for 2 hours at RT. After incubation, the plate was washed three times and the wells are blocked for 1 hour at RT using 150 μl of blocking buffer per well (PBS + 0.1% Tween 20 + 1% BSA). 20 human sera from 10 females and 10 males have been ordered at Dunn Labortechnik for the analysis. For the unspiked samples, human sera are diluted to a concentration of 5% using commercial low cross buffer (Candor). The dilution was performed as follows:

First dilution: 80 μl human serum stock + 720 μl low cross buffer;
Second dilution: 250 μl of first dilution + 250 μl low cross buffer.

For spiked samples, human sera are diluted to a concentration of 10% using commercial low cross buffer (Candor). The scFv was also diluted in low cross buffer to a concentration of 67 nM. Diluted sera and scFv were combined in a 1:1 ratio, resulting in spiked 5% human sera. For example:

5 μl scFv stock solution was diluted to 67 nM with low cross buffer (volume depends on protein concentration);
50 μl of first diluted human serum (10%) + 50 μl diluted scFv (67 nM).

A positive control was incorporated into the assay that specifically binds to the human acceptor framework of the scFv and can be later detected. This control antibody was diluted to 50 μg/ml by using normal human serum (Merck Millipore) and was further diluted for spiked and non-spiked samples as follows.
For non-spiked samples: 1 μl of control antibody (50 μg/ml) + 49 μl normal human serum to yield a 1 μg/mL concentration; 30 μl of diluted antibody-human serum solution was further diluted 1:20 with 570 μl low cross buffer resulting in a final control antibody concentration of 0.05 μg/ml.
For spiked samples: 1 μl of scFv stock was diluted to 500 nM using normal human serum (volume depends on protein concentration); 1 μl control antibody (50 μg/ml) + 49 μl spiked normal human serum resulting in a concentration of 1 μg/ml; 30 μl of diluted antibody-spiked human serum solution was further diluted 1:20 with 570 μl low cross buffer resulting in a final control antibody concentration of 0.05 μg/ml.
Spiked samples (spiked sera and spiked positive control) were incubated for 1 hour prior to admission to the immobilized scFv. After blocking, the plates were washed again three times and row A-D were incubated with the unspiked human sera and positive control and row E to H with the spiked human sera and spiked positive control for 15 minutes at RT with a volume of 25 μl per well. Afterwards, plates were washed again three times and incubated with diluted rabbit anti-human Fc-IgG-HRP detection antibody at a concentration of 100ng/ml for 1 hour at RT and volume of 25 μl per well. The antibody was therefore diluted 1:8000 with low cross dilution buffer. The detection antibody binds to the Fc region of any potential human pre-existing ADA of each serum as well as to the positive control antibody.

Another three washing steps were performed and TMB peroxidase substrate in volume of 25 $\mu$l per well is added. For this step the 96-well plate was stored in the dark for 15 minutes at RT. HRP linked to the detection antibody catalyzed the oxidation of colorless TMB to blue TMB$^+$. The enzymatic reaction was stopped by adding 25 $\mu$l 1M hydrochloric acid which further oxidizes TMB$^+$ into yellow TMB$^{2+}$ after incubating in the dark. The concentration of TMB$^{2+}$ was measured at 450 nm and was proportional to the amount of bound pre-existing ADAs in the well.

The data were analyzed by first determining the number of serum samples that show more than 30% inhibition. These serum samples were accounted as "screening positives". The screening positives sera were then further analysed by calculating the SCP for each individual plate, as described above, and by taking into account a %CCP of 30%. The number of positive serum samples for the tested molecule are summarized in Tables 7 and 8. Some exemplary graphs of absorption levels of pre-existing ADAs in human serum and of reduction of absorbance level of spiked human serum for PRO1922 and PRO2230 are shown in Figure 1. A representative comparison of the positive serum samples of 10 individual PRO2330 variants (PRO2945, PRO2942, RPO2941, PRO3349, PRO3325, PRO3346, PRO2936, PRO2931, PRO2930, PRO2938) and 10 individual PRO1992 variants (PRO2947, PRO2948, PRO2951, PRO2913, PRO2915, PRO2933, PRO2934, PRO3313, PRO3340, PRO3343) in comparison to the wt and the references PRO2330-L12R-V103T-L144Q and PRO1992-L12R-V103T-L144Q, identified in two independent rounds of pre-existing ADA determination, is shown in Figure 2.

**Table 7: Number of positive serum samples for PRO1922 (MSLN binding) scFvs:**

| PRO Number | Parental scFv | Substitutions | No of screening positive serum % samples above 30 Inhibition | No of positive serum samples above 30 % CCP |
|---|---|---|---|---|
| PRO1922 | - | wt | 16 | 10 |
| PRO2918 (reference) | PRO1922 | L12R-V103T-L144Q | 5 | 1 |
| PRO2990 (reference) | PRO1922 | L12S-V103T-L144T | 5 | 3 |
| PRO2930 | PRO1922 | L144K-T146E-S148K | 0 6 | 0 0 |
| PRO2931 | PRO1922 | L144K-S148L | | |
| PRO2932 | PRO1922 | L144A-S148N | 5 | 1 |
| PRO2936 | PRO1922 | T101S-T146Q | 4 | 0 |
| PRO2937 | PRO1922 | T101S-L144A-T146Q | 5 | 0 |
| PRO2938 | PRO1922 | L12A-T101S-L144A-T146Q | 0 | 0 |
| PRO2941 | PRO1922 | T146K | 0 | 0 |
| PRO2942 | PRO1922 | T146R | 6 | 0 |
| PRO2943 | PRO1922 | T146E | 4 | 1 |
| PRO2944 | PRO1922 | T146Q | 3 | 0 |
| PRO2945 | PRO1922 | T101R | 0 | 0 |
| PRO2991 | PRO1922 | T101A | 0 | 0 |
| PRO2992 | PRO1922 | T146A- | 4 | 3 |
| PRO3318 | PRO1922 | L12A-T101R-L144A-T146Q | 0 | 0 |
| PRO3319 | PRO1922 | L12A-T101S-L144A-T146R | 0 | 0 |
| PRO3320 | PRO1922 | L12A-T101S-L144K-T146Q | 0 | 0 |
| PRO3321 | PRO1922 | L12R-T101S-L144A-T146Q | 0 | 0 |
| PRO3322 | PRO1922 | L12R-T101R-L144A-T146Q | 0 | 0 |
| PRO3323 | PRO1922 | L12A-T101Q-L144A-T146Q | 0 | 0 |
| PRO3324 | PRO1922 | T101R-T146Q | 2 | 1 |
| PRO3325 | PRO1922 | T101S-T146R | 4 | 1 |

(continued)

| PRO Number | Parental scFv | Substitutions | No of screening positive serum % samples above 30 Inhibition | No of positive serum samples above 30 % CCP |
|---|---|---|---|---|
| PRO3326 | PRO1922 | T101Q-T146Q | 0 | 0 |
| PRO3329 | PRO1922 | T101Q | 2 | 1 |
| PRO3344 | PRO1922 | T101K-T146Q | 0 | 0 |
| PRO3345 | PRO1922 | T101N-T146Q | 0 | 0 |
| PRO3346 | PRO1922 | T101S-T146K | 0 | 0 |
| PRO3347 | PRO1922 | T101S-T146S | 3 | 3 |
| PRO3348 | PRO1922 | T101K-T146D | 0 | 0 |
| PRO3349 | PRO1922 | T101R-T146E | 0 | 0 |
| PRO2946 | PRO1922 | T101S | 9 | 6 |
| PRO2993 | PRO1922 | P15A | 3 | 2 |
| PRO2994 | PRO1922 | P48A | 6 | 2 |

**Table 8: Number of positive serum samples for PRO2230 (PDL1 binding) scFvs:**

| PRO Number | Parental scFv | Substitutions | No of screening positive serum % samples above 30 Inhibition | No of positive serum samples above 30 % CCP |
|---|---|---|---|---|
| PRO2230 | - | wt | 18 | 6 |
| PRO2903 (reference) | PRO2230 | L12R-V103T-L144Q | 5 | 4 |
| PRO2984 (reference) | PRO2230 | L12S-V103T-L144T | 5 | 5 |
| PRO2913 | PRO2230 | T101S-T146Q | 2 | 2 |
| PRO2914 | PRO2230 | T101S-L144A-T146Q | 5 | 5 |
| PRO2915 | PRO2230 | L12A-T101S-L144A-T146Q | 1 | 1 |
| PRO2933 | PRO2230 | L144K-T146E-S148K | 0 | 0 |
| PRO2934 | PRO2230 | L144K-S148L | 2 | 2 |
| PRO2947 | PRO2230 | T146K | 2 | 2 |
| PRO2948 | PRO2230 | T146R | 0 | 0 |
| PRO2949 | PRO2230 | T146E | 2 | 0 |
| PRO2950 | PRO2230 | T146Q | 4 | 4 |
| PRO2951 | PRO2230 | T101R | 0 | 0 |
| PRO2985 | PRO2230 | T101A | 2 | 2 |
| PRO2986 | PRO2230 | T146A | 2 | 2 |
| PRO3306 | PRO2230 | L12A-T101R-L144A-T146Q | 7 | 0 |
| PRO3307 | PRO2230 | L12A-T101S-L144A-T146R | 7 | 4 |
| PRO3308 | PRO2230 | L12A-T101S-L144K-T146Q | 7 | 2 |
| PRO3309 | PRO2230 | L12R-T101S-L144A-T146Q | 4 | 1 |
| PRO3310 | PRO2230 | L12R-T101R-L144A-T146Q | 6 | 3 |

(continued)

| PRO Number | Parental scFv | Substitutions | No of screening positive serum % samples above 30 Inhibition | No of positive serum samples above 30 % CCP |
|---|---|---|---|---|
| PRO3311 | PRO2230 | L12A-T101Q-L144A-T146Q | 10 | 7 |
| PRO3312 | PRO2230 | T101R-T146Q | 2 | 1 |
| PRO3313 | PRO2230 | T101S-T146R | 5 | 0 |
| PRO3314 | PRO2230 | T101Q-T146Q | 4 | 1 |
| PRO3317 | PRO2230 | T101Q | 7 | 1 |
| PRO3338 | PRO2230 | T101K-T146Q | 0 | 0 |
| PRO3339 | PRO2230 | T101N-T146Q | 0 | 0 |
| PRO3340 | PRO2230 | T101S-T146K | 2 | 0 |
| PRO3341 | PRO2230 | T101S-T146S | 4 | 0 |
| PRO3342 | PRO2230 | T101K-T146D | 1 | 0 |
| PRO3343 | PRO2230 | T101R-T146E | 0 | 0 |
| PRO2935 | PRO2230 | L144A-S148N | 8 | 8 |
| PRO2952 | PRO2230 | T101S | 8 | 5 |
| PRO2987 | PRO2230 | P15A | 7 | 2 |
| PRO2988 | PRO2230 | P48A | 7 | 3 |

## Example 3: KD determination by SPR

For the determination of the binding kinetics and affinity of PRO1992, PRO2330 and the mutation variants thereof, human $\alpha$-PD-L1 Fc (Sino Biological, 10084-H02H, LC11NO2402) and human $\alpha$-Mesothelin (Acro Biosystems; MSN_H5223) were immobilized at the surface density of 100-200 Response Units (RU) on a new CMD200M SPR sensor prism chip (Xantec) in an SPR 24 system (Sierra Sensor - Bruker). The mutation variants were then injected at concentrations of 10 nM, 2 nM and 0.4 nM over the ligand-immobilized and reference spots. The wild type variants were used as a control, while an empty spot was used as reference.

The parameters for the immobilization of the ligands onto the sensor chip are as follows:

**Human MSLN:** Concentration: 4 ug/ mL; CT: 280s; FR: 15 ul/ min; Aimed immobilization level: 700 RU; Sodium Acetate: pH 4.0;

**Human PD-L1:** Concentration: 4 ug/ mL; CT: 120s; FR: 15 ul/ min; Aimed immobilization level: 500 RU; Sodium Acetate pH 5.0.

After each cycle with analyte, the surface was regenerated with a 3M $MgCl_2$ solution, thereby allowing the next analyte cycle to have only free ligand available. Binding affinity $K_D$ was globally calculated by the Bruker SPR software based on the binding association ($k_{on}$) and dissociation ($k_{off}$) rate, by fitting a 1:1 Langmuir model to the curves obtained by the cycles with the three different concentrations of the analyte.

The binding affinity of $\alpha$-PD-L1 variants to **PD-L1** shows little variation compared to the wt as seen in Figure 2. The binding affinity ranges between 0.5 to 3.5 nM while the wild type shows a binding affinity in average of 1.5 nM (n=4). The same can be observed for variants with an $\alpha$-MSLN binding specificity. The binding affinity for the MSLN targeting variants ranges between 0.4 to 4.5 nM and for the wild type in average 1.8 nM (n=2). In summary, no significant difference between the variants and the corresponding wild type in the binding affinity was identified.

## Example 4: Thermal stability measurement using nanoDSF

PRO1992, PRO2330 and the mutation variants thereof at a concentration of $1 \pm 0.1$ mg/ml were analyzed for their thermal stability using nanoDSF.

During nanoDSF measurement, molecules in solution were subjected to increasing temperature leading to the unfolding of the molecules. PRO1992, PRO2330 and the mutation at $1 \pm 0.1$ mg/ml and $10 \pm 1$ mg/ml were analyzed for their thermal stability. Melting curves were generated with a temperature ramp from 20 °C to 95 °C with a 1 °C/min increase. To monitor the unfolding event, the intrinsic fluorescence of proteins is used. The unfolding event leads to a shift of the fluorescence emission spectra of Tryptophan (Trp) by a change of its environment, e.g., a change to solvent-exposed Trp residues from the hydrophobic core. The spectral shift was recorded at two wavelengths, 330 nm and 350 nm, and the ratio of 350nm/330 nm taken to analyze the data. The midpoint of unfolding (Tm) is defined at the inflection point (thermal midpoint) of the unfolding curve observed as a local maximum or minimum of the first derivative. The inflection point or thermal midpoint (Tm) changes with the stability of the protein. The higher the thermal midpoint, the more stable is the protein. A protein can have multiple thermal midpoints based on the number of domains that unfold separately. The thermal stability is further influenced by the protein concentration and buffer conditions. Therefore, all proteins were measured after rebuffering into 20 mM Histidine, pH 6 before measurement. The protein unfolding was conducted using the Prometheus device (Nanotemper Technologies) and recorded as well as analyzed by the PR.ThermControl v2.3.1 and PR.Stability v1.1 software. The melting onset ($T_{onset}$ of unfolding) and midpoint of unfolding (Tm) are automatically determined by the analysis software. At some instances the onset was not correctly assigned by the software and was set manually.

Table 9 lists all measured thermal midpoints of PRO1992-based and PRO2330-based scFvs determined at 1 mg/ml in 20 mM Histidine, pH 6.0, as well as the onset of unfolding. Some PRO2330 variants showed two thermal midpoints in their first derivative curve but only one was calculated due to the second thermal midpoint being overlapped by a shoulder which has no minima or maxima. Therefore, thermal midpoints were calculated or set whenever the first derivative displayed a maximum.

The thermal onset of unfolding was used more as a quality measurement as it varied deeply depending on whether it was calculated or manually set. Many PRO2330-based variants show a very similar melting point $T_{m2}$ in a range of $\pm 2.5°C$ when compared to the wild type. Also many of the PRO1992-based variants showed very little variance in a range of $\pm 2.5 °C$ when compared to the wild type.

**Table 9: Overview of melting temperatures and thermal unfolding onsets for $\alpha$-PD-L1 & $\alpha$-MSLN variants of first selection round.**

| Mutation | Relative difference to wt | | | PRO2330-based | | | PRO1992-based | | |
|---|---|---|---|---|---|---|---|---|---|
| | $\Delta$ PRO2330 [°C] | $\Delta$ PRO1992 [°C] | Variant | $T_{on}$ [°C] | Tm 1 [°C] | Tm 2 [°C] | Variant | $T_{on}$ [°C] | Tm 1 [°C] |
| wt | | | wt | 60.5 | | 81.4 | wt | 60.4 | 69.0 |
| L12R-V103T-L144Q (Reference) | 1.1 | 0.6 | A-1.7 | 68.3 | | 82.5 | B-1.7 | 50.2 | 69.6 |
| L12S-V103T-L144T (Reference) | -1.4 | -0.5 | A-1.9 | 66.3 | 71 | 80 | B-1.9 | 58.2 | 68.5 |
| L144K-S148L | -0.2 | -0.8 | A-1.14 | 66 | | 81.2 | B-1.14 | 49.7 | 68.2 |
| L144A-S148N | -0.7 | -1.4 | A-1.15 | 63.3 | 72.9 | 80.7 | B-1.15 | 49.8 | 67.6 |
| L144K-T146E-S148K | -0.1 | -1.4 | A-1.16 | 61 | | 81.3 | B-1.16 | 50.1 | 67.6 |
| T146A | -1 | -1.4 | A-2.1 | n/d | 71.8 | 80.4 | B-2.1 | 56.7 | 67.6 |
| T146E | -0.4 | 0.1 | A-2.2 | 61.3 | | 81 | B-2.2 | 49.5 | 69.1 |
| T146K | -0.4 | -0.5 | A-2.3 | 61.5 | | 81 | B-2.3 | 53.1 | 68.5 |
| T146R | -0.7 | -0.1 | A-2.4 | 62.6 | | 80.7 | B-2.4 | 54.3 | 68.9 |
| T146Q | -0.5 | -0.5 | A-2.5 | 64 | | 80.9 | B-2.5 | 52 | 68.5 |
| T101A | -1.4 | -2 | A-2.6 | 61.6 | 71.1 | 80 | B-2.6 | 55.2 | 67 |
| T101R | -3.9 | -4.4 | A-2.7 | 56.3 | 66.6 | 77.5 | B-2.7 | 49.1 | 64.6 |
| T101S | -0.2 | -0.6 | A-2.8 | 61.8 | | 81.2 | B-2.8 | 49.3 | 68.4 |
| T101S-T146Q | 0.5 | -1.9 | A-2.9 | 58.8 | | 81.9 | B-2.9 | 0 | 67.1 |
| T101S-L144A-T146Q | -0.1 | 0.1 | A-3.1 | 61 | 72.7 | 81.3 | B-3.1 | 50.1 | 69.1 |

(continued)

| Mutation | Relative difference to wt | | | PRO2330-based | | | PRO1992-based | | |
|---|---|---|---|---|---|---|---|---|---|
| | Δ PRO2330 [°C] | Δ PRO1992 [°C] | Variant | $T_{on}$ [°C] | Tm 1 [°C] | Tm 2 [°C] | Variant | $T_{on}$ [°C] | Tm 1 [°C] |
| L12A-T101S-L144AT146Q | -0.5 | -0.3 | A-3.2 | 61.3 | 72.2 | 80.9 | B-3.2 | 47 | 68.7 |
| P15A | -0.3 | -1 | A-4.1 | 62.2 | | 81.1 | B-4.1 | 49.6 | 68 |
| P48A | -0.8 | 4.7 | A-4.2 | 59.6 | | 80.6 | B-4.2 | 50.1 | 73.7 |
| T101Q | -1.4 | -2.1 | A-2-2.1 | 65.5 | 69.3 | 79.3 | B-2-2.1 | 54.3 | 66.9 |
| T101 R-T146Q | -3.1 | -3.9 | A-2-2.2 | 63 | 66.6 | 77.6 | B-2-2.2 | 56 | 65.1 |
| T101 R-T146E | -1.7 | -2.3 | A-2-2.3 | 64 | 68.6 | 79 | B-2-2.3 | 50.1 | 66.7 |
| T101S-T146R | 0.1 | -0.6 | A-2-2.4 | 61.2 | 72.7 | 80.8 | B-2-2.4 | 58.6 | 68.4 |
| T101S-T146K | 0.3 | -0.5 | A-2-2.5 | 60.5 | 72.6 | 81 | B-2-2.5 | 58 | 68.5 |
| T101Q-T146Q | -1.3 | -2 | A-2-2.6 | 65.1 | 69.5 | 79.4 | B-2-2.6 | 53.2 | 67 |
| T101K-T146Q | -3.3 | -4.2 | A-2-2.7 | 62.2 | 66.1 | 77.4 | B-2-2.7 | 54.2 | 64.8 |
| T101N-T146Q | -1.3 | -2.2 | A-2-2.8 | 65.4 | 69.3 | 79.4 | B-2-2.8 | 52.1 | 66.8 |
| T101S-T146S | -0.1 | -0.7 | A-2-2.9 | 57.4 | 72.5 | 80.6 | B-2-2.9 | 57.2 | 68.3 |
| T101K-T146D | -1.4 | -1.8 | A-2-2.10 | 65.5 | 69.1 | 79.3 | B-2-2.10 | 50.4 | 67.2 |
| L12A-T101R-L144AT146Q | -4.6 | -5.8 | A-2-3.1 | 57.2 | 64.9 | 76.1 | B-2-3.1 | 55.5 | 63.2 |
| L12A-T101S-L144AT146R | -0.8 | -1.5 | A-2-3.2 | n/d | 70.4 | 79.9 | B-2-3.2 | 58.9 | 67.5 |
| L12A-T101S-L144K-T146Q | -0.5 | -1.2 | A-2-3.3 | n/d | 70.6 | 80.2 | B-2-3.3 | 56.7 | 67.8 |
| L12R-T101S-L144AT146Q | 0.3 | -0.3 | A-2-3.4 | n/d | 72.9 | 81 | B-2-3.4 | 59.5 | 68.7 |
| L12R-T101R-L144AT146Q | -4.2 | -5.3 | A-2-3.5 | 57.3 | 65 | 76.5 | B-2-3.5 | 55.5 | 63.7 |
| L12A-T101Q-L144AT146Q | -2.8 | -3.8 | A-2-3.6 | 64 | 67.3 | 77.9 | B-2-3.6 | 56.4 | 65.2 |
| wt | | | PD-L1 wt | 60.7 | | 80.7 | MSLN wt | 60.0 | 69.0 |

Example 5: Stability studies

For ten PRO1992-based variants and ten PRO2330-based variants, as well as for the reference molecules PRO1992-L12R-V103T-L144Q, PRO2330-L12R-V103T-L144Q and the wild types a long term stability study at a concentrated of 10 ± 0.5 mg/ml was performed for two (t2w 40°C) and four (t4w 40°C) weeks at 40 °C and for four weeks at 4 °C (t4w 4°C). All proteins were analyzed after each timepoint for changes in concentration by 280nm absorption and monomeric content by SE-HPLC.

5.1 Protein concentration

First, the concentration was determined for all stability study variants. A decrease in concentration of up to 10% (1mg/ml) was considered acceptable. Larger decreases, potentially caused by hydrophobic interactions leading to aggregation and subsequent precipitation, was not observed at 40 °C.

Results of the protein concentration study are summarized in Table 10.

shows the protein concentration after storage at 40 °C and 4 °C. 7 out of 11 variants showed only minimal concentration changes compared to the initial timepoint t0 at 4 °C and 40 °C after 4 weeks for both binding motifs. Most of the PRO2330 variants showed a small but acceptable decline in concentration after 4 weeks at 4 °C. Interestingly, the corresponding PRO1992 variants showed no solubility issues at 4 °C after 4 weeks. They rather showed an increase in concentration which can be due to buffer evaporation and therefore further concentrating the sample. An increase in protein concentration over time indicates buffer evaporation, that is sometime observed during storage of small volumes (< 200 μl).

Results of the protein concentration study are summarized in Table 10.

5.2 Monomeric content determination

For determining the monomeric content of the respective samples, SE-HPLC was used. The fraction of monomers and oligomers in the samples were evaluated by integration of SE-HPLC peak areas at different time points over the course of the study.

The results of the monomeric content analysis are summarized in Table 11. In general, most of the tested variants showed a loss in monomeric content after 4 weeks storage at 40 °C of less than 10%, which was considered acceptable. Most of the variants showed suitable monomer stability, especially when using a positively charged residue such as arginine or lysine on position T146 and a non-polar residue such as serine on position T101.

**5.3 Thermal stability measurement at a protein concentration of 10 mg/ml using nanoDSF**

Further nDSF measurements have been performed at a protein concentration of 10 mg/ml. The results of the analysis are summarized in Table 12. The determined melting points of most mutation variants are ranging closely around their respective wildtypes. No major differences for the thermal stability in comparison to the variants with a concentration of 1mg/ml were detected.

**Table 10: Concentration change of PRO2330 and PRO1992 variants after 4 weeks at 40 °C and 4 °C from initial timepoint t0.**

| Mutation | | Concentration t0 [mg/ml] | | $\Delta$concentration - t4w, 40 °C [mg/ml] | | $\Delta$concentration - t4w, 4 °C [mg/ml] | |
|---|---|---|---|---|---|---|---|
| | | PRO2330 | PRO1992 | PRO2330 | PRO1992 | PRO2330 | PRO1992 |
| 1.7 | L12R-V103T-L144Q (Reference) | 10.5 | 10.5 | 0.7 | 0.7 | -4.2 | 0.3 |
| 1.14 | L144K-S148L | 10.2 | 10.3 | 0.4 | 0.7 | -6.3 | 3.8 |
| 1.16 | L144K-T146E-S148K | 10.2 | 10.2 | 0.6 | 0.6 | -0.4 | 2.0 |
| 2.3 | T146K | 9.7 | 10.0 | 1.1 | 1.2 | -0.5 | 2.2 |
| 2.4 | T146R | 10.1 | 10.4 | 1.1 | 1.2 | 1.4 | 2.6 |
| 2.7 | T101R | 10.0 | 10.0 | 0.5 | 0.4 | -0.3 | 0.8 |
| 2.9 | T101S-T146Q | 10.5 | 10.2 | 0.8 | 0.7 | -5.9 | 2.8 |
| 2-2.3 | T101R-T146E | 10.2 | 10.2 | 0.7 | 0.4 | -1.6 | 0.2 |
| 2-2.4 | T101S-T146R | 10.2 | 9.8 | 0.9 | 0.6 | -0.1 | 0.3 |
| 2-2.5 | T101S-T146K | 10.4 | 10.5 | 0.4 | 0.3 | -0.6 | -0.9 |
| 3.2 | L12A-T101S-L144A-T146Q | 10.3 | 10.0 | 0.7 | 0.4 | -0.3 | 1.0 |
| | VVT | 10.3 | 10.1 | -1.0 | 1.8 | -0.6 | 1.4 |

**Table 11: Change in monomeric content over timepoints t0, t2w and t4w at 40 °C for stability study variants of PR02330 and PRO1992.**

| Mutation | Variant | t0 [%] | t2w[%] | t4w[%] | Δt4w [%] |
|---|---|---|---|---|---|
| PRO2330 | | | | | |
| wt | PRO2330 | 99.9 | 97.8 | 95.5 | -4.4 |
| L12R-V103T-L144Q (Reference) | A-1.7 | 98.5 | 96.8 | 94.6 | -3.9 |
| L144K-S148L | A-1.14 | 99.1 | 94.9 | 90.3 | -8.8 |
| L144K-T146E-S148K | A-1.16 | 97.2 | 88.8 | 83.6 | -13.6 |
| T146K | A-2.3 | 99.3 | 96.7 | 93.3 | -6.0 |
| T146R | A-2.4 | 99.4 | 95.9 | 92.0 | -7.4 |
| T101R | A-2.7 | 99.6 | 94.3 | 87.8 | -11.9 |
| T101S-T146Q | A-2.9 | 99.8 | 97.6 | 94.8 | -5.0 |
| T101R-T146E | A-2-2.3 | 99.8 | 97.1 | 91.9 | -7.9 |
| T101S-T146R | A-2-2.4 | 99.9 | 96.7 | 93.5 | -6.4 |
| T101S-T146K | A-2-2.5 | 99.9 | 96.7 | 93.3 | -6.6 |
| L12A-T101S-L144A-T146Q | A-3.2 | 99.8 | 97.6 | 94.8 | -5.0 |
| PRO1992 | | | | | |
| wt | PRO1992 | 100.0 | 99.3 | 98.7 | -1.3 |
| L12R-V103T-L144Q (Reference) | B-1.7 | 99.3 | 98.9 | 98.1 | -1.2 |
| L144K-S148L | B-1.14 | 100.0 | 98.0 | 96.9 | -3.1 |
| L144K-T146E-S148K | B-1.16 | 99.7 | 99.0 | 98.1 | -1.6 |
| T146K | B-2.3 | 99.9 | 98.9 | 98.2 | -1.7 |
| T146R | B-2.4 | 99.9 | 98.8 | 97.9 | -2.0 |
| T101R | B-2.7 | 99.8 | 95.1 | 92.5 | -7.3 |
| T101S-T146Q | B-2.9 | 99.9 | 98.1 | 98.0 | -1.9 |
| T101R-T146E | B-2-2.3 | 100.0 | 99.0 | 97.5 | -2.5 |
| T101S-T146R | B-2-2.4 | 99.7 | 98.8 | 97.9 | -1.8 |
| T101S-T146K | B-2-2.5 | 100.0 | 98.8 | 97.8 | -2.2 |
| L12A-T101S-L144A-T146Q | B-3.2 | 100.0 | 98.8 | 97.6 | -2.4 |

**Table 12: Overview of melting temperatures and thermal unfolding onsets for PRO2330 and PRO1992 variants.**

| Mutation | Δ PRO2330 [°C] | Δ PRO1992 [°C] | PRO2330 | $T_{on}$ [°C] | Tm 1 [°C] | Tm 2 [°C] | PRO1992 | Tm 1 [°C] |
|---|---|---|---|---|---|---|---|---|
| wt | | | | 74 | 81.1 | | | 69.0 |
| L12R-V103T-L144Q | 0.6 | 0.1 | A-1.7 | | 81.7 | | B-1.7 | 69.1 |
| L144K-S148L | -0.5 | -0.8 | A-1.14 | | 80.6 | | B-1.14 | 68.2 |
| L144K-T146E-S148K | -1.4 | 0.0 | A-1.16 | | 79.7 | | B-1.16 | 69.1 |
| T146K | -0.1 | -0.2 | A-2.3 | | 80.9 | | B-2.3 | 68.8 |
| T146R | -0.2 | -0.5 | A-2.4 | | 80.9 | | B-2.4 | 68.5 |
| T101R | -3.7 | -4.2 | A-2.7 | 66.2 | 77.4 | | B-2.7 | 64.8 |
| T101S-T146Q | 0.0 | -0.9 | A-2.9 | | 81.0 | | B-2.9 | 68.1 |
| T101 R-T146E | -1.9 | -2.5 | A-2-2.3 | 68.7 | 79.2 | | B-2-2.3 | 66.5 |

(continued)

| Mutation | Δ PRO2330 [°C] | Δ PRO1992 [°C] | PRO2330 | $T_{on}$ [°C] | Tm 1 [°C] | Tm 2 [°C] | PRO1992 | Tm 1 [°C] |
|---|---|---|---|---|---|---|---|---|
| T101S-T146R | -0.2 | -0.6 | A-2-2.4 | | 80.8 | | B-2-2.4 | 68.4 |
| T101S-T146K | -0.1 | -0.8 | A-2-2.5 | | 81.0 | | B-2-2.5 | 68.2 |
| L12A-T101S-L144A-T146Q | -0.5 | -1.3 | A-3.2 | | 80.5 | | B-3.2 | 67.7 |

**Example 6: Solubility testing by PEG precipitation assay**

For ten PRO1992-based variants and ten PRO2330-based variants, as well as for the reference molecules PRO1992-L12R-V103T-L144Q, PRO2330-L12R-V103T-L144Q and the wild types PRO1992 and PRO2330, a precipitation assay using polyethylene glycol (PEG) was performed to analyze the apparent solubility of these molecules.

There are other methods to assess protein solubility such concentrating (e.g. by ultrafiltration) or lyophilization. Those methods however need excessive amounts of protein or suffer from experimental issues such as aggregation or gel formation. PEG precipitation is useful for small protein quantities (<1mg) and measures solubility by precipitating proteins through exclusion volume effects. With increasing PEG concentration, the proteins start to precipitate. The apparent solubility is determined at the PEG8,000 (%w/v) concentration where protein concentration is reduced by half ($PEG_{midpoint}$). A gradient in 12 steps is created using 20 mM Histidine, pH 6 and a 37% (w/v) PEG8000 solution in 20 mM Histidine, pH 6. Subsequently, 8 $\mu$l of Protein at a concentration of 10 mg/ml was mixed with 72 $\mu$l of the PEG gradient solution using a 96-deep well plate. The plate is incubated overnight and the solutions filtered into a second 96 deep well plate at 2000xg for 2 min at RT using a Pall® AcroPrep™ Advance 96-well filter plate (1.2 $\mu$m). The protein concentration was measured as a duplicate at 280 nm with the subtraction of the reference wavelength at 310 nm for all proteins and all dilutions. Further, expanding the PEG/Histidine gradient to more measurable points can help create a more pronounced plateau in order to determine the PEG midpoint more precisely.

Data was fitted using a 4-parameter sigmoidal fit (4PL, sigmoidal, least squares fit) by Graphpad Prism 9 software and the $PEG_{midpoint}$ and the 95% confidence interval determined. A higher $PEG_{midpoint}$ value corresponds to a higher apparent solubility of the protein.

For most of the tested scFvs, the obtained precipitation curves had a similar shape, indicating a similar precipitation behavior relative to their wildtype, i.e. precipitation occurs at similar PEG8000 concentrations.

The results of the PEG midpoint determination for the tested **scFvs** is shown in Figure 3. The higher the midpoint is, the more soluble the protein is. PRO1992-based **scFvs** showed on average a higher PEGmidpoint than the PRO1992-based scFvs. Overall, the tested scFv variants generally show a similar solubility relative to their wildtypes.

**Claims**

1. An antibody fragment, which binds to a target antigen, wherein said antibody fragment does not comprise an antibody Fc part and wherein the antibody variable domaincomprises:

(i) a variable heavy chain (VH) comprising from N-terminus to C-terminus, the regions HFW1-HCDR1-HFW2-HCDR2-HFW3-HCDR3-HFW4, wherein each HFW designates a heavy chain framework region, and each HCDR designates a heavy chain complementarity-determining region,
wherein said variable heavy chain framework regions HFW1, HFW2, HFW3 and HFW4 are selected from a human VH framework, wherein said HFW1, HFW2, HFW3 and HFW4 have one or more substitutions selected from the group consisting of (AHo numbering):

- an alanine (A), serine (S), lysine (K), arginine (R), aspartate (D), glutamate (E), asparagine (N) or glutamine (Q) at amino acid position 101;
- an alanine (A), serine (S), lysine (K), arginine (R), aspartate (D), glutamate (E), asparagine (N) or glutamine (Q) at amino acid position 146; and
- a leucine (L), lysine (K), or asparagine (N) at amino acid position 148;
and

(ii) a variable light chain (VL), wherein the variable light chain comprises, from N-terminus to C-terminus, the regions LFW1-LCDR1-LFW2-LCDR2-LFW3-LCDR3-LFW4, wherein each LFW designates a light chain framework region, and each LCDR designates a light chain complementarity-determining region.

2. The antibody fragment of claim 1, wherein said HFW1, HFW2, HFW3 and HFW4 have one or more substitutions selected from the group consisting of (AHo numbering):

- an alanine (A), serine (S), lysine (K), arginine (R), asparagine (N) or glutamine (Q) at amino acid position 101;
- a lysine (K), aspartate (D), glutamate (E) arginine (R), or glutamine (Q) at amino acid position 146; and
- a lysine (K) at amino acid position 148.

3. The antibody fragment of any one of claims 1 or 2, wherein said HFW1, HFW2, HFW3 and HFW4 additionally have one or two substitutions selected from the group consisting of (AHo numbering):

- an alanine (A), lysine (K) or arginine (R) at amino acid position 12, particularly an alanine (A) or arginine (R) at amino acid position 12; and
- an alanine (A), lysine (K) or arginine (R) at amino acid position 144.

4. The antibody fragment of any one of claims 1 to 3, wherein said HFW1, HFW2, HFW3 and HFW4 have one of the following substitutions (AHo numbering):

a. an alanine (A), serine (S), lysine (K), arginine (R), asparagine (N) or glutamine (Q) at amino acid position 101;
b. an alanine (A), serine (S), lysine (K), arginine (R), aspartate (D), glutamate (E), asparagine (N) or glutamine (Q) at amino acid position 146;
c. an alanine (A), serine (S), lysine (K), arginine (R), asparagine (N) or glutamine (Q) at amino acid position 101; and
an alanine (A), serine (S), lysine (K), arginine (R), aspartate (D), glutamate (E), asparagine (N) or glutamine (Q) at amino acid position 146;
d. an alanine (A), lysine (K) or arginine (R) at amino acid position 144; and
a leucine (L), lysine (K) or asparagine (N) at amino acid position 148;
e. an alanine (A), lysine (K) or arginine (R) at amino acid position 144;

an alanine (A), serine (S), lysine (K), arginine (R), aspartate (D), glutamate (E), asparagine (N) or glutamine (Q) at amino acid position 146; and
a leucine (L), lysine (K) or asparagine (N) at amino acid position 148;

f. an alanine (A), serine (S), lysine (K), arginine (R), asparagine (N) or glutamine (Q) at amino acid position 101;

an alanine (A), lysine (K) or arginine (R) at amino acid position 144; and
an alanine (A), serine (S), lysine (K), arginine (R), aspartate (D), glutamate (E), asparagine (N) or glutamine (Q) at amino acid position 146.

g. an alanine (A) or arginine (R) at amino acid position 12;

an alanine (A), serine (S), lysine (K), arginine (R), asparagine (N) or glutamine (Q) at amino acid position 101;
an alanine (A), lysine (K) or arginine (R) at amino acid position 144; and
an alanine (A), serine (S), lysine (K), arginine (R), aspartate (D), glutamate (E), asparagine (N) or glutamine (Q) at amino acid position 146.

5. The antibody fragment of claim 4, wherein said HFW1, HFW2, HFW3 and HFW4 have one of the following substitutions (AHo numbering):

a. an alanine (A), lysine (K), arginine (R) or asparagine (N) at amino acid position 101;
b. a lysine (K), arginine (R), aspartate (D), glutamate (E) or glutamine (Q) at amino acid position 146;
c. a serine (S), lysine (K), arginine (R), asparagine (N) or glutamine (Q) at amino acid position 101; and
a lysine (K), aspartate (D), glutamate (E) or glutamine (Q) at amino acid position 146;
d. an alanine (A) or lysine (K) at amino acid position 144; and
a leucine (L) or asparagine (N) at amino acid position 148;

e. a lysine (K) at amino acid position 144;

a glutamate (E) at amino acid position 146; and
a lysine (K) at amino acid position 148;

f. a serine (S), arginine (R) or glutamine (Q) at amino acid position 101;

an alanine (A) or lysine (K) at amino acid position 144; and
an arginine (R), or glutamine (Q) at amino acid position 146

g. an alanine (A) at amino acid position 12;

a serine (S), arginine (R) or glutamine (Q) at amino acid position 101;
an alanine (A) or lysine (K) at amino acid position 144; and
an arginine (R), or glutamine (Q) at amino acid position 146.

6. The antibody fragment of any one of claims 1 to 5, wherein said variable heavy chain framework regions HFW1, HFW2, HFW3 and HFW4 are selected from the human VH framework subtypes VH1a, VH1b, VH3 and VH4, in particular are of the human VH framework subtype VH3.

7. The antibody fragment of any one of the preceding claims, wherein

a. said variable light chain framework regions LFW1, LFW2, LFW3 and LFW4 are selected from a human antibody Vκ framework or a human antibody Vλ framework, in particular a human antibody Vκ framework; or
b. said variable light chain framework regions LFW1, LFW2 and LFW3 are selected from a human antibody Vκ framework, and said variable light chain framework region LFW4 is selected from a Vλ framework, particularly from a Vλ framework sequence selected from the group consisting of SEQ ID NOs: 188, 189, 190, 191, 192, 193, 194, 195 and 196.

8. The antibody fragment of any one of the preceding claims, wherein said variable heavy chain framework regions HFW1, HFW2, HFW3 and HFW4 are selected from

a. the combination of framework regions HFW1, HFW2, HFW3 and HFW4 (*i. e.* the non-italicized residues in Tables 1 and 3) of any one of the SEQ ID NOs: 8 - 25, 28, 30 - 36, 86 - 103, 106 and 108 - 114, particularly of any one of the SEQ ID NOs: 8 - 25 and 86 - 103; and
b. the combination of framework regions HFW1, HFW2, HFW3 and HFW4 (*i. e.* the non-italicized residues in Tables 1 and 3) of any one of the SEQ ID NOs: 8 - 25, 28, 30 - 36, 86 - 103, 106 and 108 - 114, particularly of any one of the SEQ ID NOs: 8 - 25 and 86 - 103, having 1, 2 or 3 mutations within the framework regions at positions different from 12, 101, 144, 146 and 148 (AHo numbering);

and wherein said variable light chain framework regions LFW1, LFW2, LFW3 and LFW4 are selected from

a. the combination of framework regions LFW1, LFW2, LFW3 and LFW4 (*i. e.* the non-italicized residues in Tables 1, 3 and 5) of any one of the SEQ ID NOs: 37, 38, 39, 115, 116, 117, 180, 181, 182, 183, 184, 185, 186 and 187; and
b. the combination of framework regions LFW1, LFW2, LFW3 and LFW4 (*i. e.* the non-italicized residues in Tables 1, 3 and 5) of any one of the SEQ ID NOs: 37, 38, 39, 115, 116, 117, 180, 181, 182, 183, 184, 185, 186 and 187 having 1, 2 or 3 mutations within the framework regions at positions different from 101 (AHo numbering).

9. An antibody comprising one or more antibody fragments as defined in any one of claims 1 to 8.

10. The antibody of claims 9, wherein the format of said antibody is selected from Fc-bearing bivalent bispecific formats, more particularly wherein the format of said antibody is an Fc-bearing KiH-based format; Fc-bearing trivalent bispecific and tetravalent bispecific formats; and trivalent bispecific IgG formats and tetravalent bispecific IgG formats, wherein said formats comprise one or two Fv, scFv or disulfide stabilized scFv fragments; more particularly wherein the format of said antibody is selected from Fc-bearing KiH-based formats; DVD-Ig; CODV-IgG and Morrison (IgG $CH_3$-scFv fusion (Morrison-H) or IgG CL-scFv fusion (Morrison-L)), even more particularly from DVD-Ig and Morrison (IgG $CH_3$-scFv fusion (Morrison-H) or IgG CL-scFv fusion (Morrison-L)).

11. The antibody fragment of any one of claims 1 to 8, wherein said antibody fragment does not comprise an immunoglobulin Fc region, and wherein said antibody does further not comprise CH1 and/or CL regions, particularly wherein said antibody is in a scDb-scFv, a triabody, a tetrabody or a MATCH format, more particularly wherein said antibody is in a MATCH or scDb-scFv format, more particularly wherein said antibody is in a MATCH format, more particularly a MATCH3 or a MATCH4 format.

12. A nucleic acid or two nucleic acids encoding the antibody fragment of any one of claims 1 to 8 and 11, or the antibody of claims 9 or 10.

13. A vector or two vectors comprising the nucleic acid or the two nucleic acids of claim 12.

14. A host cell or host cells comprising the vector or the two vectors of claim 13.

15. A method for producing the antibody fragment of any one of claims 1 to 8 and 11, or the antibody of claims 9 or 10, comprising (i) providing the nucleic acid or the two nucleic acids of claim 12, or the vector or the two vectors of claim 13, expressing said nucleic acid sequence or nucleic acids, or said vector or vectors, and collecting said antibody variable domain or said antibody from the expression system, or (ii) providing a host cell or host cells of claim 14, culturing said host cell or said host cells; and collecting said antibody variable domain or said antibody from the cell culture.

16. A pharmaceutical composition comprising the antibody fragment of any one of claims 1 to 8 and 11, or the antibody of claims 9 or 10 and a pharmaceutically acceptable carrier.

Figure 1:

A

B

**Figure 2:**

pre-ADA best variants

Figure 3:

Europäisches
Patentamt

European
Patent Office

Office européen
des brevets

# EUROPEAN SEARCH REPORT

**Application Number**

EP 22 17 2186

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2017/121399 A1 (BUYSE MARIE-ANGE [BE] ET AL) 4 May 2017 (2017-05-04) * paragraphs [0023], [0032], [0087], [0195]; tables 1, 2; figure 1 * | 1-16 | INV. C07K16/00 C07K16/28 C07K16/30 |
| Y | WO 2016/150845 A1 (ABLYNX NV [BE]) 29 September 2016 (2016-09-29) * pages 9-11, claims * | 1-16 | |
| Y | US 2011/152505 A1 (URECH DAVID [CH] ET AL) 23 June 2011 (2011-06-23) * p. [0011] – [0012], [0016], [0051] and [0064] * | 1-16 | |
| Y | MATHIEU DONDELINGER ET AL: "Understanding the Significance and Implications of Antibody Numbering and Antigen-Binding Surface/Residue Definition", FRONTIERS IN IMMUNOLOGY, vol. 9, 16 October 2018 (2018-10-16), pages 1-15, XP055572450, DOI: 10.3389/fimmu.2018.02278 * pages 6-7, figures 7, S1, S2 * | 1-16 | |

**TECHNICAL FIELDS SEARCHED (IPC)**

C07K

-/--

The present search report has been drawn up for all claims

| Place of search | Date of completion of the search | Examiner |
|---|---|---|
| The Hague | 15 December 2022 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Europäisches Patentamt
European Patent Office
Office européen des brevets

**Application Number**

EP 22 17 2186

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TIMOTHY J. EGAN ET AL: "Novel multispecific heterodimeric antibody format allowing modular assembly of variable domain fragments", MABS, vol. 9, no. 1, 27 October 2016 (2016-10-27), pages 68-84, XP055467772, US ISSN: 1942-0862, DOI: 10.1080/19420862.2016.1248012 * abstract, figures, in particular figure 7 * | 10,11 | |
| Y | Bithi Chatterjee ET AL: "A low affinity bivalent mesothelin-binding MATCH4 multispecific T cell engager increases cytotoxic selectivity for high mesothelin expressing cells", Journal for immunotherapy of cancer, 1 November 2020 (2020-11-01), pages A723-A723, XP055754424, SITC2020 Poster #684 DOI: 10.1136/jitc-2020-SITC2020.0684 Retrieved from the Internet: URL:https://www.numab.com/wp-content/uploads/2020/11/Chatterjee-B_SITC_biMSLN_Poster_FINAL.pdf * poster, in particular figure 2 * | 10,11 | TECHNICAL FIELDS SEARCHED (IPC) |

The present search report has been drawn up for all claims

| Place of search | Date of completion of the search | Examiner |
|---|---|---|
| The Hague | 15 December 2022 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

**EP 22 17 2186**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-16(partially)**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
## SHEET B

**Application Number**

EP 22 17 2186

The Search Division considers that the present European patent application does not comply with the requirements of unity of invention and relates to several inventions or groups of inventions, namely:

1. claims: 1-16(partially)

   An antibody fragment, which binds to a target antigen, wherein said antibody fragment does not comprise an antibody Fc part and wherein the antibody variable domain comprises:
   (i) a variable heavy chain (VH) comprising from N-terminus to C-terminus, the regions HFW1-HCDR1-HFW2-HCDR2-HFW3-HCDR3-HFW4 of Seq ID No. 8 or of Seq ID No. 8 having 1, 2 or 3 mutations within the framework regions at positions different from 12, 101, 144, 146 and 148 (AHo numbering); and
   (ii) a variable light chain comprising from N-terminus to C-terminus, the combination of framework regions LFW1, LFW2, LFW3 and LFW4 according to claim 8;
   products and methods related thereto according to claims 9-16

   ---

2-52. claims: 1-16(partially)

   An antibody fragment, which binds to a target antigen, wherein said antibody fragment does not comprise an antibody Fc part and wherein the antibody variable domain comprises:
   (i) a variable heavy chain (VH) comprising from N-terminus to C-terminus, the regions HFW1-HCDR1-HFW2-HCDR2-HFW3-HCDR3-HFW4 of Seq ID No. 9-25, 28, 30-36, 86-103, 106 and 108-114, respectively, or of Seq ID No. 9-25, 28, 30-36, 86-103, 106 and 108-114, respectively, having 1, 2 or 3 mutations within the framework regions at positions different from 12, 101, 144, 146 and 148 (AHo numbering); and
   (ii) a variable light chain comprising from N-terminus to C-terminus, the combination of framework regions LFW1, LFW2, LFW3 and LFW4 according to claim 8;
   products and methods related thereto according to claims 9-16

   ---

## ANNEX TO THE EUROPEAN SEARCH REPORT
## ON EUROPEAN PATENT APPLICATION NO.

EP 22 17 2186

This annex lists the patent family members relating to the patent documents cited in the above-mentioned European search report.
The members are as contained in the European Patent Office EDP file on
The European Patent Office is in no way liable for these particulars which are merely given for the purpose of information.

15-12-2022

| Patent document cited in search report | Publication date | Patent family member(s) | Publication date |
|---|---|---|---|
| US 2017121399 A1 | 04-05-2017 | CA 3175002 A1 | 19-11-2015 |
| | | CN 106661100 A | 10-05-2017 |
| | | CN 109053885 A | 21-12-2018 |
| | | CN 113717280 A | 30-11-2021 |
| | | IL 259972 A | 31-07-2018 |
| | | IL 281755 A | 31-05-2021 |
| | | IL 295534 A | 01-10-2022 |
| | | JP 7098439 B2 | 11-07-2022 |
| | | JP 2017518072 A | 06-07-2017 |
| | | JP 2018162297 A | 18-10-2018 |
| | | JP 2021006563 A | 21-01-2021 |
| | | JP 2021130681 A | 09-09-2021 |
| | | KR 20170002645 A | 06-01-2017 |
| | | KR 20180091115 A | 14-08-2018 |
| | | NZ 726448 A | 24-02-2023 |
| | | PH 12016502282 A1 | 13-02-2017 |
| | | RU 2018120744 A | 15-11-2018 |
| | | RU 2021107470 A | 17-05-2021 |
| | | SG 10201805288Q A | 30-07-2018 |
| | | SG 10201810124P A | 28-12-2018 |
| | | SG 11201609162S A | 29-12-2016 |
| | | US 2017121399 A1 | 04-05-2017 |
| | | US 2020231662 A1 | 23-07-2020 |
| | | US 2021087261 A1 | 25-03-2021 |
| | | US 2021261653 A1 | 26-08-2021 |
| | | US 2021269514 A1 | 02-09-2021 |
| | | US 2021269515 A1 | 02-09-2021 |
| | | US 2022119512 A1 | 21-04-2022 |
| | | US 2022251183 A1 | 11-08-2022 |
| | | US 2022332806 A1 | 20-10-2022 |
| | | US 2022332807 A1 | 20-10-2022 |
| | | ZA 201607587 B | 25-05-2022 |
| | | ZA 202004757 B | 31-08-2022 |
| | | ZA 202004758 B | 31-08-2022 |
| | | ZA 202004759 B | 31-08-2022 |
| | | ZA 202100742 B | 28-09-2022 |
| WO 2016150845 A1 | 29-09-2016 | EP 3271391 A1 | 24-01-2018 |
| | | US 2018044407 A1 | 15-02-2018 |
| | | US 2022324951 A1 | 13-10-2022 |
| | | WO 2016150845 A1 | 29-09-2016 |
| US 2011152505 A1 | 23-06-2011 | AR 079700 A1 | 15-02-2012 |
| | | AU 2010335950 A1 | 19-07-2012 |
| | | BR 112012017051 A2 | 05-12-2017 |
| | | CA 2777527 A1 | 30-06-2011 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 22 17 2186

This annex lists the patent family members relating to the patent documents cited in the above-mentioned European search report.
The members are as contained in the European Patent Office EDP file on
The European Patent Office is in no way liable for these particulars which are merely given for the purpose of information.

15-12-2022

| Patent document cited in search report | Publication date | Patent family member(s) | Publication date |
|---|---|---|---|
| | | CL 2012001696 A1 | 08-03-2013 |
| | | CN 102686608 A | 19-09-2012 |
| | | CN 109293768 A | 01-02-2019 |
| | | EP 2516461 A1 | 31-10-2012 |
| | | EP 3498731 A1 | 19-06-2019 |
| | | JP 5914353 B2 | 11-05-2016 |
| | | JP 6815083 B2 | 20-01-2021 |
| | | JP 2013515460 A | 09-05-2013 |
| | | JP 2015131855 A | 23-07-2015 |
| | | JP 2016094487 A | 26-05-2016 |
| | | JP 2017121254 A | 13-07-2017 |
| | | JP 2019141071 A | 29-08-2019 |
| | | JP 2021153603 A | 07-10-2021 |
| | | KR 20120102061 A | 17-09-2012 |
| | | KR 20170036137 A | 31-03-2017 |
| | | KR 20170084357 A | 19-07-2017 |
| | | MX 352871 B | 13-12-2017 |
| | | RU 2012130945 A | 27-01-2014 |
| | | TW 201125975 A | 01-08-2011 |
| | | TW 201619383 A | 01-06-2016 |
| | | TW 201925228 A | 01-07-2019 |
| | | US 2011152505 A1 | 23-06-2011 |
| | | US 2015080555 A1 | 19-03-2015 |
| | | US 2018016350 A1 | 18-01-2018 |
| | | US 2021214461 A1 | 15-07-2021 |
| | | UY 33154 A | 30-06-2011 |
| | | UY 39290 A | 30-07-2021 |
| | | WO 2011075861 A1 | 30-06-2011 |
| | | ZA 201202299 B | 25-06-2014 |
| | | ZA 201402145 B | 30-05-2018 |
| | | ZA 201706443 B | 23-12-2020 |
| | | ZA 202006532 B | 28-04-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011075861 A
- WO 2019057787 A
- WO 200492219 A, Hinton
- WO 200442072 A, Presta
- WO 20160202457 A
- US 6075181 A
- US 6150584 A
- US 5766886 A
- WO 9957150 A
- US 5260203 A
- US 5455030 A

- US 4881175 A
- US 5132405 A
- US 5091513 A
- US 5476786 A
- US 5013653 A
- US 5258498 A
- US 5482858 A
- US 4458066 A
- WO 2019072868 A
- WO 2021239987 A

### Non-patent literature cited in the description

- **KÖHLER ; MILSTEIN.** mAbs. *Nature,* 1975, vol. 256, 495-497
- **ZHAO, L. ; LI, J.** *BMC Struct. Biol.,* 2010, vol. 10, S6
- **NATAGA, S. ; PASTAN, I.** *Adv Drug Deliv Rev,* 2009, 977-985
- **ONDA, M. et al.** *PNAS,* 2008, vol. 105 (32), 1 1311-11316
- **MAZOR, R. et al.** *Immunol Rev.,* 2016, vol. 270 (1), 152-64
- **KABAT et al.** Sequences of Proteins of Immunological Interest. Public Health Service, National Institutes of Health, 1991
- **AL-LAZIKANI et al.** *JMB,* 1997, vol. 273, 927-948
- **LEFRANC, M.-P.** *The Immunologist,* 1999, vol. 7, 132-136
- **LEFRANC, M.-P. et al.** *Dev. Comp. Immunol.,* 2003, vol. 27, 55-77
- **HONEGGER ; PLÜCKTHUN.** *J. Mol. Biol.,* 2001, vol. 309, 657-670
- **KNAPPIK et al.** *J. Mol. Biol.,* 2000, vol. 296, 57-86
- **EGAN et al.** *MAbs,* 2017, vol. 9 (1), 68-84
- **NIESEN et al.** *Nature Protocols,* 2007, vol. 2 (9), 2212-2221
- *MAbs,* February 2017, vol. 9 (2), 182-212
- **RIDGWAY et al.** *Protein Eng.,* 1996, vol. 9, 617-21
- **SPIESS et al.** *J Biol Chem.,* 2013, vol. 288 (37), 26583-93
- **M. DAERON.** *Annu. Rev. Immunol.,* 1997, vol. 5, 203-234

- **RAVETCH ; KINET.** *Annu. Rev. Immunol.,* 1991, vol. 9, 457-92
- **CAPET et al.** *Immunomethods,* 1994, vol. 4, 25-34
- **DE HAAS et al.** *J. Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249
- **GHETIE ; WARD.** *Immunol. Today,* 1997, vol. 18 (12), 592-8
- **GHETIE et al.** *Nature Biotechnology,* 1997, vol. 15 (7), 637-40
- **HINTON et al.** *J. Biol. Chem.,* 2004, vol. 8, 6213-6
- **SHIELDS et al.** *J. Biol. Chem.,* 2001, vol. 9 (2), 6591-6604
- **EGAN T. et al.** *MABS,* 2017, vol. 9, 68-84
- **HOOGENBOOM ; WINTER.** *J. Mol. Biol,* 1992, vol. 227, 381
- **MARKS et al.** *J. Mol. Biol,* 1991, vol. 222, 581
- **COLE et al.** Monoclonal Antibodies and Cancer Therapy. Alan R. Liss, 1985, 77
- **BOEMER et al.** *J. Immunol,* 1991, vol. 147 (I), 86-95
- **VAN DIJK ; VAN DE WINKEL.** *Curr. Opin. Pharmacol,* 2001, vol. 5, 368-74
- **LI et al.** *Proc. Natl. Acad. Sci. USA,* 2006, vol. 103, 3557-3562
- **MORRISON et al.** *Proc. Natl. Acad. Sci. USA,* 1984, vol. 81, 6851-6855
- **MORRISON ; OI.** *Adv. Immunol.,* 1988, vol. 44, 65-92
- **VERHOEYEN et al.** *Science,* 1988, vol. 239, 1534-1536

- **PADLAN.** *Molec. Immun.,* 1991, vol. 28, 489-498
- **PADLAN.** *Molec. Immun.,* 1994, vol. 31, 169-217
- **FISCHER, N. ; LEGER, O.** *Pathobiology,* 2007, vol. 74, 3-14
- **HORNIG, N. ; FÄRBER-SCHWARZ, A.** *Methods Mol. Biol.,* 2012, vol. 907, 713-727
- **LABRIJN et al.** *Proc. Natl. Acad. Sci. USA,* 2013, vol. 110, 5145-5150
- **DE KRUIF et al.** *Biotechnol. Bioeng.,* 2010, vol. 106, 741-750
- **YOKOYAMA et al.** Curr. Protoc. Immunol. 2006
- **CHAMES ; BATY.** *FEMS Microbiol. Letters,* 2000, vol. 189, 1-8
- **KARPOVSKY et al.** *J. Exp. Med.,* 1984, vol. 160, 1686
- **LIU, M A et al.** *Proc. Natl. Acad. Sci. USA,* 1985, vol. 82, 8648
- **PAULUS.** *Behring Ins. Mitt.,* 1985, (78), 118-132
- **BRENNAN et al.** *Science,* 1985, vol. 229, 81-83
- **GLENNIE et al.** *J. Immunol.,* 1987, vol. 139, 2367-2375
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **NARANG et al.** *Meth. Enzymol.,* 1979, vol. 68, 90
- **BROWN et al.** *Meth. Enzymol.,* 1979, vol. 68, 109
- **BEAUCAGE et al.** *Tetrahedron Lett.,* 1981, vol. 22, 1859
- PCR Technology: Principles and Applications for DNA Amplification. Freeman Press, 1992
- PCR Protocols: A Guide to Methods and Applications. Academic Press, 1990
- **MATTILA et al.** *Nucleic Acids Res.,* 1991, vol. 19, 967
- **ECKERT et al.** *PCR Methods and Applications,* 1991, vol. 1, 17
- **HARRINGTON et al.** *Nat Genet.,* 1997, vol. 15, 345
- **SMITH.** *Annu. Rev. Microbiol.,* 1995, vol. 49, 807
- **ROSENFELD et al.** *Cell,* 1992, vol. 68, 143
- **SCHARF et al.** *Results Probl. Cell Differ.,* 1994, vol. 20, 125
- **BITTNER et al.** *Meth. Enzymol.,* 1987, vol. 153, 516
- **WINNACKER.** FROM GENES TO CLONES. VCH Publishers, 1987
- **QUEEN et al.** *Immunol. Rev.,* 1986, vol. 89, 49-68
- **GREEN, M. R. ; SAMBROOK, J.** Molecular Cloning: A Laboratory Manual. Cold Spring Harbor Laboratory Press, 2012
- **ELLIOT ; O'HARE.** *Cell,* 1997, vol. 88, 223
- Remington: The Science and Practice of Pharmacy. Mack Publishing Co, 2000
- Sustained and Controlled Release Drug Delivery Systems. Marcel Dekker, Inc, 1978